(19) Europäisches Patentamt
European Patent Office
Office européen des brevets

(11) EP 4 056 163 A1

(12) EUROPEAN PATENT APPLICATION

(43) Date of publication:
**14.09.2022 Bulletin 2022/37**

(21) Application number: **21162479.6**

(22) Date of filing: **15.03.2021**

(51) International Patent Classification (IPC):
***A61K 6/62*** *(2020.01)* ***A61K 6/887*** *(2020.01)*
***C08F 2/50*** *(2006.01)* ***A61K 6/30*** *(2020.01)*

(52) Cooperative Patent Classification (CPC):
(C-Sets available)
**A61K 6/62; C08F 2/50** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2021 JP 2021040033**
**12.03.2021 JP 2021040037**
**12.03.2021 JP 2021040039**
**12.03.2021 JP 2021040040**

(71) Applicant: **Shofu Inc.**
**Kyoto-shi, Kyoto 605-0983 (JP)**

(72) Inventors:
- **YAMAMOTO, Kenzo**
  **KYOTO, 605-0983 (JP)**
- **HARA, Daisuke**
  **KYOTO, 605-0983 (JP)**
- **MIYATA, Shunsuke**
  **KYOTO, 605-0983 (JP)**

(74) Representative: **Eisenführ Speiser**
**Patentanwälte Rechtsanwälte PartGmbB**
**Postfach 31 02 60**
**80102 München (DE)**

(54) **DENTAL ADHESIVE COMPOSITION EXCELLENT IN STORAGE STABILITY**

(57) [Problem]
To provide a dental adhesive composition having excellent storage stability and adhesive strength.
[Solution]
To provide a dental adhesive composition, comprising (A) polymerizable monomer, (D) photopolymerization accelerator, and (F) volatile organic solvent, wherein, the dental adhesive composition comprises (A-1) polymerizable monomer having an acidic group as the (A) polymerizable monomer, and the dental adhesive composition comprises (D-1) aliphatic tertiary amine compound represented by formula (1) as the (D) photopolymerization accelerator.

[Formula (1)]

[Chemical formula 1]

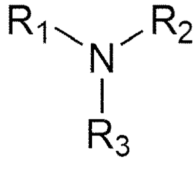

(wherein $R_1$ represents a substituent consisting of three or more carbons having an electron-attracting group at $\alpha$-carbon and/or $\beta$-carbon of an amine starting from N, $R_2$ represents a substituent consisting of three or more carbons which may have an electron-attracting group, $R_3$ represents a substituent consisting of one or more carbons which may have an electron-attracting group, and $\alpha$-carbon of N in the formula (1) is not an electron-attracting group.)

EP 4 056 163 A1

(52) Cooperative Patent Classification (CPC): (Cont.)

C-Sets
**A61K 6/30, C08L 33/08;**
**A61K 6/30, C08L 33/10;**
**A61K 6/887, C08L 33/08;**
**A61K 6/887, C08L 33/10**

## Description

BACKGROUND OF THE INVENTION

Field of the Invention

The present invention relates to a dental adhesive composition.

Description of the Related Art

A dental adhesive composition has been used in the dental field, and applied to a dental adhesive material, a dental composite resin, a dental core build-up material, a dental resin cement, a dental coating material, a dental sealant material, a dental manicure material, a dental splinting material and a dental glass ionomer cement.

In Japanese Unexamined Patent Publication No. 2006-225350 and Japanese Patent No. 4783151, an adhesive composition containing a basic compound containing an acidic group polymerizable monomer and an aliphatic tertiary amine is proposed.

In Japanese Unexamined Patent Publication No. 2006-76973, an adhesive composition containing an acidic group polymerizable monomer, an aromatic tertiary amine and a triazine compound is proposed.

SUMMARY OF THE INVENTION

Technical Problem

However, conventional dental adhesive compositions have room for improvement in terms of storage stability and adhesive strength.

An object of the present invention is to provide a dental adhesive composition having excellent storage stability and adhesive strength.

Solution to Problem

A dental adhesive composition of the present invention comprises (A) polymerizable monomer, (D) photopolymerization accelerator, and (F) volatile organic solvent, wherein, the dental adhesive composition comprises (A-1) polymerizable monomer having an acidic group as the (A) polymerizable monomer, and the dental adhesive composition comprises (D-1) aliphatic tertiary amine compound represented by formula (1) as the (D) photopolymerization accelerator.

[Formula (1)]

[Chemical formula 1]

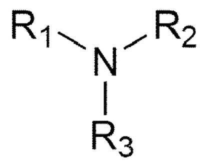

(wherein $R_1$ represents a substituent consisting of three or more carbons having an electron-attracting group at α-carbon and/or β-carbon of an amine starting from N, $R_2$ represents a substituent consisting of three or more carbons which may have an electron-attracting group, $R_3$ represents a substituent consisting of one or more carbons which may have an electron-attracting group, and α-carbon of N in the formula (1) is not an electron-attracting group.)

Advantageous Effects of Invention

The dental adhesive composition of the present invention is excellent in storage stability and adhesive strength.

DETAILED DESCRIPTION OF THE PREFERRED EMBODIMENTS

In the present invention, the electron-attracting group in $R_1$ may be a substituent selected from a functional group selected from the group consisting of a hydroxyl group, a carboxyl group, a vinyl group, an aryl group and a

halogen, and, an organic group which is bonded via an ether bond, an ester bond, a urethane bond or a urea bond and may have -OH group, -O-group, -C(O)-group, -S-group, -NH-C(O)-NH-group, -C(O)-O-group, -O-C(O)-group, -OC(O)-NH-group, -NH-C(O)-O-group, an aromatic hydrocarbon group, or a polymerizable functional group capable of radical polymerization.

In the present invention, the (D-1) aliphatic tertiary amine compound represented by formula (1) may be an aliphatic tertiary amine compound in which $R_1$ and $R_2$ have an aliphatic substituent consisting of three or more carbons having an electron-attracting group at α-carbon and/or β-carbon.

In the present invention, the (D-1) aliphatic tertiary amine compound represented by formula (1) may be an aliphatic tertiary amine compound in which $R_1$ and $R_2$ have an aryl group which may have a substituent, at α-carbon and/or β-carbon.

In the present invention, the dental adhesive composition may further comprise (G) water.

In the present invention, the dental adhesive composition may further comprise (B) photosensitizer.

In the present invention, the dental adhesive composition may further comprise (C) photoacid generator.

In the present invention, the dental adhesive composition may comprise an aryl iodonium salt as the (C) photoacid generator, and the aryl iodonium salt may be a salt of an anion having an organic group and one or more atoms of P, B, Al, S and Ga, and an aryl iodonium cation.

In the present invention, the dental adhesive composition may comprise an aryl iodonium salt as the (C) photoacid generator, and the aryl iodonium salt may be a salt of an anion having an organic group in which at least one H is substituted with F and one or more atoms of P, B, Al, S and Ga, and an aryl iodonium cation.

In the present invention, the dental adhesive composition may be one pack type dental adhesive composition comprising, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water, 0.1 to 20 parts by mass of the (A-1) polymerizable monomer having an acidic group, 1 to 99.9 parts by mass of the (F) volatile organic solvent, and 0.01 to 20 parts by mass of the (D-1) aliphatic tertiary amine compound represented by formula (1).

In the present invention, the dental adhesive composition may be two packs type dental adhesive composition consisting of a first pack and a second pack, wherein a mass ratio of the first pack and the second pack is 0.8:1 to 1.2:1, the first pack comprises the (A-1) polymerizable monomer having an acidic group, the (F) volatile organic solvent and the (D-1) aliphatic tertiary amine compound represented by formula (1), the first pack comprises, with respect to 100 parts by mass of total of the (A) polymerizable monomer and the (F) volatile organic solvent contained in the first pack or with respect to 100 parts by mass of total of the (A) polymerizable monomer, the (F) volatile organic solvent and (G) water in the case of containing the (G) water contained in the first pack, 0.2 to 20 parts by mass of the (A-1) polymerizable monomer having an acidic group, 1 to 99.9 parts by mass of the (F) volatile organic solvent, and 0.02 to 20 parts by mass of the (D-1) aliphatic tertiary amine compound represented by formula (1).

The present invention provides a dental adhesive kit comprising a dental photocurable composition and the dental adhesive composition according to any one of claims 1 to 11, wherein the dental photocurable composition comprises, with respect to 100 parts by mass of the (A) polymerizable monomer contained in the dental photocurable composition, 0.001 to 2 parts by mass of (B) photosensitizer, and 0.01 to 10 parts by mass of the (C) photoacid generator.

Hereinafter, each component in the dental adhesive composition of the present invention is described in detail. A dental adhesive composition of the present invention can be applied to a dental adhesive material, a dental composite resin, a dental core build-up material, a dental resin cement, a dental coating material, a dental sealant material, a dental manicure material, a dental splinting material and a dental glass ionomer cement.

In a dental practice, in order to restore aesthetically and functionally a lost portion of a tooth by caries, breakages and the like, a direct restoration by a dental composite resin and an indirect restoration by a prosthetic device consisting of ceramics or hard resin by using a dental resin cement have been performed as treatment. A dental adhesive material has been used for adhering a dental composite resin to various dental materials and natural tooth. In addition, a dental adhesive material has been used for adhering a dental splinting material for fixing a mobile tooth, a dental coating material for protect a vital tooth after forming, against a hyperesthesia, an external stimulation and secondary caries, a dental sealant material for preventing caries by filling complex grooves such as especially a deciduous tooth, a dental manicure material for temporary recovering aesthetic property by masking discoloration of a tooth, a dental core build-up material for forming an abutment tooth in the case of collapsing of a dental crown due to caries, and the like.

As a dental adhesive, a self-etching primer containing water and an acidic group-containing polymerizable monomer has been proposed in order to achieve both an enamel decalcification function and a dentin penetration promoting function. Such a composition has an advantage that the operation is simplified because the etching operation before applying the primer is not required. On the other hand, when the acidic group-containing polymerizable monomer and water coexist in the same package, the acidic group-containing polymerizable monomer is hydrolyzed when stored for a long period of time, therefore there has been a disadvantage that the adhesive strength to the adherend decreases.

Therefore, a composition in which an acidic group-containing polymerizable monomer, water and a basic

compound coexist has been proposed. In such a composition, the acidity is neutralized by forming a salt between the acidic group-containing polymerizable monomer and the basic compound, so that the storage stability is improved. However, since basic inorganic compounds and tertiary aliphatic amine compounds that are usually used form strong salts with acidic group-containing polymerizable monomers, there is a problem in that the adhesive strength is lowered.

In order to solve the above problem, the present inventors have been found that the dental adhesive composition of the present invention realizes both excellent storage stability and excellent adhesive strength when an aliphatic tertiary amine having a specific structure is used, and have completed the present invention.

Further, the present inventors have been found that when the dental adhesive composition of the present invention and the dental photocurable composition containing a photoacid generator are adhered, further excellent adhesive strength is exhibited.

The dental adhesive composition of the present invention can be applied as a dental adhesive material, a dental coating material and a dental manicure material, and further can be used as a kit in combination with a dental adhesive material, a dental composite resin, a dental core build-up material, a dental resin cement, a dental coating material, a dental sealant material, a dental manicure material, a dental splinting material, a dental hard resin, a dental cutting and machining material or a dental 3D printer material which are dental curable compositions.

[(A) Polymerizable monomer]

As the (A) polymerizable monomer of the present invention, any polymerizable monomers can be used without limitation as long as it is known. In the polymerizable monomer or the compound having a polymerizable group described in the present invention, the polymerizable group preferably exhibits radical polymerizability, and specifically, from the viewpoint of easy radical polymerization, the polymerizable group is preferably (meth) acrylic group and/or (meth) acrylamide group. In the present specification, "(meth) acrylic" means acrylic and/or methacrylic, "(meth) acryloyl" means acryloyl and/ or methacryloyl, "(meth) acrylate" means acrylate and/or methacrylate, and, "(meth) acrylamide" means acrylamide and/or methacrylamide. A polymerizable monomer having a substituent at the $\alpha$-position of an acrylic group and/or an acrylamide group can also be preferably used. Specific examples include one having one radical polymerizable group, one having two radical polymerizable groups, one having three or more radical polymerizable groups, one having an acidic group, one having an alkoxysilyl group, and one having a sulfur atom.

Specific examples of a polymerizable monomer having one radical polymerizable group and not containing acidic group include 2-hydroxyethyl (meth) acrylate, 3-hydroxypropyl (meth) acrylate, 4-hydroxybutyl (meth) acrylate, 2-hydroxypropyl (meth) acrylate, 2-hydroxybutyl (meth) acrylate, 6-hydroxyhexyl (meth) acrylate, 10-hydroxydecyl (meth) acrylate, propylene glycol mono (meth) acrylate, glycerol mono (meth) acrylate, erythritol mono (meth) acrylate, N-methylol (meth) acrylamide, N-hydroxyethyl (meth) acrylamide, N,N-(dihydroxyethyl) (meth) acrylamide, methyl (meth) acrylate, ethyl (meth) acrylate, propyl (meth) acrylate, isopropyl (meth) acrylate, butyl (meth) acrylate, isobutyl (meth) acrylate, benzyl (meth) acrylate, lauryl (meth) acrylate, 2,3-dibromopropyl (meth) acrylate, 3-(meth) acryloyloxypropyl trimethoxysilane, 11-(meth) acryloyloxyundecyl trimethoxysilane, (meth) acrylamide and the like.

Specific Examples of the polymerizable monomer having two radical polymerizable groups and not containing acidic group include 2,2-bis ((meth) acryloyloxy phenyl) propane, 2,2-bis [4-(3-(meth) acryloyloxy)-2-hydroxy propoxyphenyl] propane (generally called "Bis-GMA"), 2,2-bis (4-(meth) acryloyloxy phenyl) propane, 2,2-bis (4-(meth) acryloyloxy polyethoxyphenyl) propane, 2,2-bis (4-(meth) acryloyloxy diethoxyphenyl) propane, 2,2-bis (4-(meth) acryloyloxy tetraethoxy-phenyl) propane, 2,2-bis (4-(meth)) acryloyloxy pentaethoxyphenyl) propane, 2,2-bis (4-(meth) acryloyloxy dipropoxyphenyl) propane, 2-(4-(meth) acryloyloxy diethoxyphenyl)-2-(4-(meth) acryloyloxy diethoxyphenyl) propane, 2-(4-(meth) acryloyloxy diethoxyphenyl)-2-(4-(meth) acryloyloxy ditriethoxyphenyl) propane, 2-(4-(meth) acryloyloxy dipropoxyphenyl)-2-(4-(meth) acryloyloxy tri-ethoxyphenyl) propane, 2,2-bis (4-(meth) acryloyloxy propoxyphenyl) propane, 2,2-bis (4-(meth) acryloyloxy isopropoxyphenyl) propane, 1,4-bis (2-(meth) acryloyloxyethyl) pyromellitate, glycerol di (meth) acrylate, 1-(acryloyloxy)-3-(methacryloyloxy)-2-propanol, ethyleneglycol di (meth) acrylate, diethyleneglycol di (meth) acrylate, triethylene glycol di (meth) acrylate, propylene glycol di (meth) acrylate, butylene glycol di (meth) acrylate, neopentyl glycol di (meth) acrylate, polyethylene glycol di (meth) acrylate, 1,3-butanediol di (meth) acrylate, 1,5-pentanediol di (meth) acrylate, 1,6-hexanediol di (meth) acrylate, 1,10-decanediol di (meth) acrylate, 1,2-bis (3-methacryloyloxy-2-hydroxypropoxy) ethane, 2,2,4-trimethyl hexamethylene bis (2-carbamoyloxy ethyl) dimethacrylate (generally called "UDMA"), 1,2-bis (3-methacryloyloxy-2-hydroxy propoxy) ethane and the like.

Specific Examples of the polymerizable monomer having three or more radical polymerizable groups and not containing acidic group include trimethylolpropane tri (meth) acrylate, trimethylolethane tri (meth) acrylate, trimethylolmethane tri (meth) acrylate, pentaerythritol tri (meth) acrylate, pentaerythritol tetra (meth) acrylate, dipentaerythritol penta (meth) acrylate, N,N-(2,2,4-trimethyl hexamethylene) bis [2- (aminocarboxy) propane-1,3-diol] tetra methacrylate, 1,7-diacry-loyloxy-2,2,6,6-tetra acryloyloxymethyl-4-oxyheptane and the like.

For the (A-1) polymerizable monomer having an acidic group, any polymerizable monomer can be used without any limitation as long as it has one or more polymerizable group and at least one acidic group such as a phosphoric

acid group, a pyrophosphoric acid group, a thiophosphoric acid group, a phosphonic acid group, a sulfonic acid group and a carboxylic acid group and the like.

Specific examples of a phosphoric acid group-containing polymerizable monomer include 2-(meth) acryloyloxyethyl dihydrogen phosphate, 3-(meth) acryloyloxypropyl dihydrogen phosphate, 4-(meth) acryloyloxybutyl dihydrogen phosphate, 5-(meth) acryloyloxypentyl dihydrogen phosphate, 6-(meth) acryloyloxyhexyl dihydrogen phosphate, 7-(meth) acryloyloxyheptyl dihydrogen phosphate, 8-(meth) acryloyloxyoctyl dihydrogen phosphate, 9-(meth) acryloyloxynonyl dihydrogen phosphate, 10-(meth) acryloyloxydecyl dihydrogen phosphate, 11-(meth) acryloyloxyundecyl dihydrogen phosphate, 12-(meth) acryloyloxydodecyl dihydrogen phosphate, 16-(meth) acryloyloxyhexadecyl dihydrogen phosphate, 20-(meth) acryloyloxyicosyl dihydrogen phosphate, bis [2-(meth) acryloyl oxyethyl] hydrogensphosphate, bis [4-(meth) acryloyl oxybutyl] hydrogen phosphate, bis [6-(meth) acryloyl oxyhexyl] hydrogen phosphate, bis [8-(meth) acryloyl oxyoctyl] hydrogen phosphate, bis [9-(meth) acryloyl oxynonyl] hydrogen phosphate, bis [10-(meth) acryloyl oxydecyl] hydrogen phosphate, 1,3-di (meth) acryloyl oxypropyl dihydrogenphosphate, 2-(meth) acryloyl oxyethylphenyl hydrogen phosphate, 2-(meth) acryloyloxyethyl-2-bromoethyl hydrogen phosphate and bis [2-(meth) acryloyloxy-(1-hyrdoxymethyl) ethyl] hydrogen phosphate; acyl chloride, alkali metal salt and ammonium salt thereof; and (meth) acrylamide compound in which the ester bond of these compounds is substituted with an amide bond, and the like.

Specific examples of a pyrophosphoric acid group-containing polymerizable monomer include bis [2-(meth) acryloyl oxyethyl] pyrophosphate, bis [4-(meth) acryloyl oxybutyl] pyrophosphate, bis [6-(meth) acryloyl oxyhexyl] pyrophosphate, bis [8-(meth) acryloyl oxyoctyl] pyrophosphate, bis [10-(meth) acryloyl oxydecyl] pyrophosphate; acyl chloride, alkali metal salt and ammonium salt thereof; and (meth) acrylamide compound in which the ester bond of these compounds is substituted with an amide bond, and the like.

Specific examples of a thiophosphate group-containing polymerizable monomer include 2-(meth) acryloyloxyethyl dihydrogen thiophosphate, 3-(meth) acryloyloxypropyl dihydrogen thiophosphate, 4-(meth) acryloyloxybutyl dihydrogen thiophosphate, 5-(meth) acryloyloxypentyl dihydrogen thiophosphate, 6-(meth) acryloyloxyhexyl dihydrogen thiophosphate, 7-(meth) acryloyloxyheptyl dihydrogen thiophosphate, 8-(meth) acryloyloxyoctyl dihydrogen thiophosphate, 9-(meth) acryloyloxynonyl dihydrogen thiophosphate, 10-(meth) acryloyloxydecyl dihydrogen thiophosphate, 11-(meth) acryloyloxyundecyl dihydrogen thiophosphate, 12-(meth) acryloyloxydodecyl dihydrogen thiophosphate, 16-(meth) acryloyloxyhexadecyl dihydrogen thiophosphate, 20-(meth) acryloyloxyicosyl dihydrogen thiophosphate; acyl chloride, alkali metal salt and ammonium salt thereof; and (meth) acrylamide compound in which the ester bond of these compounds is substituted with an amide bond, and the like. The polymerizable monomer having a thiophosphate group is also classified as a polymerizable monomer having a sulfur atom.

Specific examples of a phosphonic acid group-containing polymerizable monomer include 2-(meth) acryloyloxy ethylphenyl phosphonate, 5-(meth) acryloyloxy pentyl-3-phosphonopropio-nate, 6-(meth) acryloyloxy hexyl-3-phosphonopropionate, 10-(meth) acryloyloxy decyl-3-phos-phonopropionate, 6-(meth) acryloyloxy hexyl-3-phosphonoacetate, 10-(meth) acryloyloxy decyl-3-phosphonoacetate; acyl chloride, alkali metal salt and ammonium salt thereof; and (meth)acrylamide compound in which the ester bond of these compounds is substituted with an amide bond, and the like.

Specific examples of a sulfonic acid group-containing polymerizable monomer include 2-(meth) acrylamide-2-methyl propanesulfonic acid and 2-sulfoethyl (meth) acrylate and the like.

The carboxylic acid group-containing polymerizable monomers are classified into a (meth) acrylic-based compound having one carboxyl group in the molecule and a (meth) acrylic-based compound having a plurality of carboxyl groups in the molecule. Examples of the (meth) acrylic-based compound having one carboxyl group in the molecule include (meth) acrylic acid, N-(meth) acryloyl glycine, N-(meth) acryloyl aspartic acid, O-(meth) acryloyl tyrosine, N-(meth) acryloyl tyrosine, N-(meth) acryloyl phenylalanine, N-(meth) acryloyl-p-aminobenzoic acid, N-(meth) acryloyl-o-aminobenzoic acid, p-vinylbenzoic acid, 2-(meth) acryloyloxybenzoic acid, 3-(meth) acryloyloxybenzoic acid, 4-(meth) acryloyloxybenzoic acid, N-(meth) acryloyl-5-aminosalicylic acid, N-(meth) acryloyl-4-aminosalicylic acid, 2-(meth) acryloyloxyethyl hydrogen succinate, 2-(meth) acryloyloxyethyl hydrogen phthalate, 2-(meth) acryloyloxyethyl hydrogenmalate; acyl chloride thereof; and (meth)acrylamide compound in which the ester bond of these compounds is substituted with an amide bond, and the like. Examples of the (meth) acrylic-based compound having a plurality of carboxyl groups in the molecule include 6-(meth) acryloyl oxyhexane-1,1-dicarboxylic acid, 9-(meth) acryloyl oxynonane-1,1-dicarboxylic acid, 10-(meth) acryloyl oxydecane-1,1-dicarboxylic acid, 11-(meth) acryloyloxy undecane-1,1-dicarboxylic acid, 12-(meth) acryloyl oxydodecane-1,1-dicarboxylic acid, 13-(meth) acryloyloxy tridecane-1,1-dicarboxylic acid, 4-(meth) acryloyloxyethyl trimeritate, 4-(meth) acryloyloxybutyl trimeritate, 4-(meth) acryloyloxyhexyl trimeritate, 4-(meth) acryloyloxydecyl trimeritate, 2-(meth) acryloyl oxyethyl-3'-(meth) acryloyloxy-2'-(3,4-dicarboxy benzoyloxy) propylsuccinate; acid anhydrides and acid halides thereof,; and (meth) acrylamide compound in which the ester bond of these compounds is substituted with an amide bond, and the like.

Specific examples of the polymerizable monomer having an alkoxysilyl group include a (meth) acrylic compound having one alkoxysilyl group in the molecule and a (meth) acrylic compound having a plurality of alkoxysilyl groups in the molecule. Specific examples include 2-(meth) acryloxyethyl trimethoxysilane, 3-(meth) acryloxypropyl trimethoxysilane, 3-(meth) acryloxypropyl triethoxysilane, 3-(meth) acryloxypropyl methyldimethoxysilane, 4-(meth) acryloxybutyl

trimethoxysilane, 5-(meth) acryloxypentyl trimethoxysilane, 6-(meth) acryloxyhexyl trimethoxysilane, 7-(meth) acryloxyheptyl trimethoxysilane, 8-(meth) acryloxyoctyl trimethoxysilane, 9-(meth) acry-loxynonyl trimethoxysilane, 10-(meth) acryloxydecyl trimethoxysilane, 11-(meth) acryloxyundecyl trimethoxysilane.

As the polymerizable monomer having a sulfur atom, any known compound can be used without any limitation as long as it is a polymerizable monomer having one or more sulfur atoms and a polymerizable group. Specifically, it refers to a compound having a partial structure such as -SH, -S-S-, >C=S, >C-S-C<, >P=S, or a compound prepared by tautomerism. Specific examples include 10-methacryloxy decyl-6,8-dithiooctanate, 6-methacryloxy hexyl-6,8-dithiooctanate, 6-methacryloxy hexyl-2-thiouracil-5-carboxylate, 2-(11-methacryloxy undecylthio)-5-mercapto-1,3,4-thiadiazole, 10-(meth) acryloxy decyl dihydrogenthiophosphate.

An oligomer or a prepolymer having at least one polymerizable group in its molecule may be used other than such a polymerizable monomer, without any limitation. There is no problem even if a substituent such as a fluoro group is contained in the same molecule. The polymerizable monomers described above can be used not only singly but also in combinations of a plurality thereof.

The dental adhesive composition of the present invention contains (A-1) polymerizable monomer having an acidic group in order to impart adhesive property with respect to a tooth substance and a prosthetic device. Among them, 10-methacryloyloxydecyl dihydrogenphosphate, 6-methacryloxyhexyl phosphonoacetate, 10-methacryloyloxydecyl dihydrogenthiophosphate, 4-(meth) acryloyloxyethyl trimeritate and 4-(meth) acryloyloxyethyl trimeritate anhydlide are preferable.

The compounding amount of the (A-1) polymerizable monomer having an acidic group in the case that the dental adhesive composition is one pack type dental adhesive composition is preferably 0.1 to 20 parts by mass, more preferably 1 to 20 parts by mass, with respect to 100 parts by mass of total of the (A) polymerizable monomer containing the (A-1) polymerizable monomer having an acidic group and the (F) volatile organic solvent or with respect to 100 parts by mass of total of the (A) polymerizable monomer, the (F) volatile organic solvent and (G) water in the case of containing the (G) water. When the compounding amount is less than 0.1 parts by mass, there is a case that adhesive property to the tooth substance and the prosthetic device is not imparted, and when the compounding amount exceeds 20 parts by mass, there is a case that the storage stability deteriorates.

The compounding amount of the (A-1) polymerizable monomer having an acidic group in the case that the dental adhesive composition is two packs type dental adhesive composition consisting of a first pack and a second pack is preferably 0.2 to 20 parts by mass, more preferably 1 to 20 parts by mass in the first pack, with respect to 100 parts by mass of total of the (A) polymerizable monomer and the (F) volatile organic solvent contained in the first pack or with respect to 100 parts by mass of total of the (A) polymerizable monomer, the (F) volatile organic solvent and (G) water in the case of containing the (G) water contained in the first pack. When the compounding amount is less than 0.2 parts by mass, there is a case that adhesive property to the tooth substance and the prosthetic device is not imparted, and when the compounding amount exceeds 20 parts by mass, there is a case that the storage stability deteriorates.

The dental adhesive composition of the present invention may contain a silane coupling agent having a polymerizable group as the (A) polymerizable monomer in order to impart adhesive property with respect to glass ceramics and a resin material consisting of a matrix of an inorganic component and a polymerizable monomer. Any known silane coupling agent can be used without any limitation, but 3-(meth) acryloxypropyl trimethoxysilane, 8-(meth) acryloxyoctyl trimethoxysilane, 11-(meth) acryloxyundecyl trimethoxysilane, 4,4-diethoxy-17-oxo-3,16-dioxa-18-aza-4-cilaicosan-20-yl (meth) acrylate and 4,4-diethoxy-17-oxo-3,16,21-trioxa-18-aza-4-silatricosan-23-yl (meth) acrylate are preferable. From the view point of imparting adhesive property, the compounding amount is preferably 0.01 to 10 parts by mass, more preferably 0.01 to 5 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water. Since the purpose of the silane coupling agent as a polymerizable monomer is to impart adhesive property with respect to glass ceramics or a resin material containing a filler consisting of glass ceramics, the silane coupling agent is compounded separately from the surface treatment agent of the filler.

The dental adhesive composition of the present invention may contain a polymerizable monomer having a sulfur atom as the (A) polymerizable monomer in order to impart adhesive property with in which respect to a noble metal. From the view point of imparting adhesive property, the compounding amount of the polymerizable monomer having a sulfur atom is preferably 0.01 to 10 parts by mass, more preferably 0.1 to 5 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water.

<Photopolymerization initiator>

The dental adhesive composition of the present invention contains (D-1) aliphatic tertiary amine compound

represented by formula (1). The dental adhesive composition of the present invention may contain (B) photosensitizer, (C) photoacid generator and (D) photopolymerization accelerator other than the (D-1) aliphatic tertiary amine compound represented by formula (1), as a photopolymerization initiator, in addition to the (D-1) aliphatic tertiary amine compound represented by formula (1). These may be compounded alone and are not particularly limited, and any known compounds commonly used may be used without any limitation in the case of containing these.

[(B) Photosensitizer]

Specific examples of the (B) photosensitizer which can be used in the present invention include α-diketones such as benzyl, camphorquinone, camphorquinone carboxylic acid, camphorquinone sulfonic acid, α-naphthyl, acetonaphthene, p,p'-dimethoxybenzyl, p,p'-dichlorobenzylacetyl, pentanedion, 1,2-phenanthrenequinone, 1,4-phenanthrenequinone, 3,4-phenanthrenequinone, 9,10-phenanthrenequinone and naphthoquinone; benzoin alkyl ethers such as benzoin, benzoin methyl ether and benzoin ethyl ether; thioxanthones such as thioxanthone, 2-chlorothioxanthone, 2-methylthioxanthone, 2-isopropylthioxanthone, 2-methoxythioxanthone, 2-hydroxythioxanthone, 2,4-diethylthioxanthone and 2,4-diisopropylthioxanthone; benzophenones such as benzophenone, p-chlorobenzophenone and p-methoxybenzophenone; acylphosphine oxides such as bis (2,6-dimethoxy benzoyl) phenylphosphine oxide, bis (2,6-dimethoxy benzoyl) (2,4,4-trimethyl pentyl) phosphine oxide, bis (2,6-dimethoxy benzoyl)-n-butylphosphine oxide, bis (2,6-dimethoxy benzoyl)-(2-methylprop-1-yl) phosphine oxide, bis (2,6-dimethoxy benzoyl)-(1-methylprop-1-yl) phosphine oxide, bis (2,6-dimethoxy benzoyl)-t-butyl phosphine oxide, bis (2,6-dimethoxy benzoyl) cyclohexyl phosphine oxide, bis (2,6-dimethoxy benzoyl) octyl phosphine oxide, bis (2-methoxy benzoyl) (2-methylprop-1-yl) phosphine oxide, bis (2-methoxy benzoyl) (1-methylprop-1-yl) phosphine oxide, bis (2,6-diethoxy benzoyl) (2-methylprop-1-yl) phosphine oxide, bis (2,6-diethoxy benzoyl) (1-methylprop-1-yl) phosphine oxide, bis (2,6-dibutoxy benzoyl) (2-methylprop-1-yl) phosphine oxide, bis (2,4-dimethoxy benzoyl) (2-methylprop-1-yl) phosphine oxide, bis (2,4,6-trimethyl benzoyl) phenyl phosphine oxide, 2,4,6-trimethyl benzoyl diphenyl phosphine oxide, bis (2,4,6-trimethyl benzoyl) (2,4-dipentoxy phenyl) phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl)-2-phenylethyl phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl)-2-phenylethyl phosphine oxide, 2,6-dimethoxy benzoyl benzyl butyl phosphine oxide, 2,6-dimethoxy benzoyl benzyl octyl phosphine oxide, bis (2,4,6-trimethyl benzoyl) isobutyl phosphine oxide and 2,6-dimethoxy benzoyl-2,4,6-trimethyl benzoyl-n-butyl phosphine oxide; acylgermanium compounds such as bisbenzoyl diethylgermanium, bisbenzoyl dimethylgermanium, bisbenzoyl dibutylgermanium, bis (4-methoxybenzoyl) dimethylgermanium and bis (4-methoxybenzoyl) diethylgermanium; α-aminoacetophenones such as 2-benzyl-dimethylamino-1-(4-morpholinophenyl)-butanone-1, and 2-benzyl-diethylamino-1-(4-morpholinophenyl)-propanone-1; ketals such as benzyl dimethyl ketal, benzyl diethyl ketal and benzyl (2-methoxyethyl ketal); and titanocenes such as bis (cyclopentadienyl)-bis [2,6-difluoro-3-(1-pyrrolyl) phenyl]-titanium, bis (cyclopentadienyl)-bis (pentanefluorophenyl)-titanium and bis (cyclopentadienyl)-bis (2,3,5,6-tetrafluoro-4-disiloxyphenyl)-titanium.

The photosensitizer (B) may be appropriately selected according to the wavelength, the intensity and the irradiation time of light used for polymerization, and the type and the compounding amount of other components to be combined. In addition, the photosensitizer may be used not only singly but also in combinations of two or more. Among them, α-diketone compounds having a maximum absorption wavelength in the visible light region are preferably used, and camphorquinone compounds such as camphorquinone, camphorquinone carboxylic acid and camphorquinone sulfonic acid are more preferable. Camphorquinone is particularly preferred because it is easily available.

In the case that the dental adhesive composition of the present invention contains (B) a photosensitizer, the compounding amount of the (B) photosensitizer is preferably 0.001 to 5 parts by mass, more preferably 0.1 to 1 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water. When the compounding amount of the photosensitizer is less than 0.001 parts by mass, there is a case that the polymerization activity with respect to the irradiation light is poor and the curing becomes insufficient. When the compounding amount is more than 5 parts by mass, there is a case that although sufficient curability is exhibited, the environmental light stability is shortened, and yellowness is increased.

[(C) Photoacid generator]

The dental adhesive composition of the present invention may contain (C) photoacid generator. As the (C) photoacid generator, known compound can be used without limitation. Specific examples include triazine compounds, iodonium salt compounds, sulfonium salt compounds, and sulfonic acid ester compounds. Among these, triazine compounds and iodonium salt-based compounds are preferable because of having high polymerizability in the case of using in combination with a sensitizer. Iodonium salt-based compounds are more preferable, lodonium-based salt compounds are susceptible to sensitization by photosensitizers that have absorption in the visible light region.

Specific examples of the triazine compound include 2,4,6-tris (trichloro methyl)-s-triazine, 2,4,6-tris (tribromo methyl)-s-triazine, 2-methyl-4,6-bis (trichloro methyl)-s-triazine, 2-methyl-4,6-bis (tribromo methyl)-s-triazine, 2-phenyl-4,6-bis (trichloro methyl)-s-triazine, 2-(p-methoxy phenyl)-4,6-bis (trichloro methyl)-s-triazine, 2-(p-methyl thiophenyl)-4,6-bis (trichloro methyl)-s-triazine, 2-(p-chloro phenyl)-4,6-bis (trichloro methyl)-s-triazine, 2-(2,4-dichloro phenyl)-4,6-bis (trichloro methyl)-s-triazine, 2-(p-bromo phenyl)-4,6-bis (trichloro methyl)-s-triazine, 2-(p-tolyl)-4,6-bis (trichloro methyl)-s-triazine, 2-n-propyl-4,6-bis (trichloro methyl)-s-triazine, 2-($\alpha$,$\alpha$,$\beta$-trichloro ethyl)-4,6-bis (trichloro methyl)-s-triazine, 2-styryl-4,6-bis (trichloro methyl)-s-triazine, 2-[2-(p-methoxy phenyl) ethenyl]-4,6-bis (trichloro methyl)-s-triazine, 2-[2-(o-methoxy phenyl) ethenyl]-4,6-bis (trichloro methyl)-s-triazine, 2-[2-(p-butoxy phenyl) ethenyl]-4,6-bis (trichloro methyl)-s-triazine, 2-[2-(3,4-dimethoxy phenyl) ethenyl]-4,6-bis (trichloro methyl)-s-triazine, 2-[2-(3,4,5-trimethoxy phenyl) ethenyl]-4,6-bis (trichloro methyl)-s-triazine, 2-(1-naphthyl)-4,6-bis(trichloro methyl)-s-triazine, 2-(4-biphenylyl)-4,6-bis(trichloro methyl)-s-triazine, 2-[2-{N,N-bis(2-hydroxy ethyl) amino} ethoxy]-4,6-bis (trichloro methyl)-s-triazine, 2-[2-{N-hydroxy ethyl-N-ethylamino} ethoxy]-4,6-bis (trichloro methyl)-s-triazine, 2-[2-{N-hydroxy ethyl-N-methylamino} ethoxy]-4,6-bis (trichloro methyl)-s-triazine, 2-[2-{N,N-diallyl amino} ethoxy]-4,6-bis (trichloro methyl)-s-triazine and the like. Among them, 2,4,6-tris (trichloro methyl)-s-triazine is preferable.

Any iodonium salt-based compound can be used as long as it is known. For the specific examples, the structural formula of the iodonium salt-based compound can be represented by the following formula (2).

[Formula (2)] $[(R1)_2I]^+ [A]^-$

(In the formula, $[(R1)_2I]^+$ is a cation part, $[A]^-$ is an anion part, R1 shown in the formula (2) represents an organic group bonded to I, and R1s may be the same or different. R1 represents, for example, an aryl group having 6 to 30 carbon atoms, a heterocyclic group having 4 to 30 carbon atoms, an alkyl group having 1 to 30 carbon atoms, an alkenyl group having 2 to 30 carbon atoms, or an alkynyl group having 2 to 30 carbon atoms, which may have at least one substituted group selected from the group consisting of groups such as alkyl, hydroxy, alkoxy, alkylcarbonyl, arylcarbonyl, alkoxycarbonyl, aryloxycarbonyl, arylthiocarbonyl, acyloxy, arylthio, alkylthio, aryl, heterocycle, aryloxy, alkylsulfinyl, arylsulfinyl, alkylsulfonyl, arylsulfonyl, alkyleneoxy, amino, cyano, nitro groups and halogens.)

In the above, examples of the aryl group having 6 to 30 carbon atoms include a monocyclic aryl group such as a phenyl group and a condensed polycyclic aryl group such as a naphthyl, anthrasenyl, phenanthrenyl, pyrenyl, chrysenyl, naphthacenyl, benzanthrasenyl, anthraquinolyl, fluorenyl, naphthoquinone and anthraquinone.

Examples of the heterocyclic group having 4 to 30 carbon atoms include cyclic groups containing 1 to 3 heteroatoms such as oxygen, nitrogen, and sulfur, which may be the same or different. Specific examples include a monocyclic heterocyclic group such as thienyl, furanyl, pyranyl, pyrrolyl, oxazolyl, thiazolyl, pyridyl, pyrimidyl and pyrazinyl, and a condensed polycyclic heterocyclic group such as indolyl, benzofuranyl, isobenzofuranyl, benzothienyl, isobenzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, dibenzothienyl, xanthonyl, thioxanthonyl and dibenzofuran.

Specific examples of alkyl groups having 1 to 30 carbon atoms include a linear alkyl group such as methyl, ethyl, propyl, butyl, hexadecyl and octadecyl, a branched alkyl group such as isopropyl, isobutyl, sec-butyl, tert-butyl, isopentyl, neopentyl, tert-pentyl, isohexyl and a cycloalkyl groups such as cyclopropyl, cyclobutyl, cyclopentyl and cyclohexyl.

In addition, specific examples of the alkenyl group having 2 to 30 carbon atoms include a linear chain or branched group such as vinyl, allyl, 1-propenyl, isopropenyl, 1-butenyl, 2-butenyl, 3-butenyl and 1-methyl-1-propenyl.

Further, specific examples of the alkynyl group having 2 to 30 carbon atoms include a linear chain or branched group such as ethynyl, 1-propynyl, 2-propynyl, 1-butynyl, 2-butynyl, 3-butynyl, 1-methyl-1-propynyl and 1-methyl-2-propynyl.

The above-described aryl group having 6 to 30 carbon atoms, heterocyclic group having 4 to 30 carbon atoms, alkyl group having 1 to 30 carbon atoms, alkenyl group having 2 to 30 carbon atoms and alkynyl group having 2 to 30 carbon atoms may have at least one substituted group. Specific examples of the substituted group include a linear alkyl group having 1 to 18 carbon atoms such as methyl, ethyl, propyl, butyl and octadecyl; a branched alkyl group having 1 to 18 carbon atoms such as isopropyl, isobutyl, sec-butyl and tert- butyl; a cycloalkyl group having 3 to 18 carbon atoms such as cyclopropyl, cyclobutyl, cyclopentyl and cyclohexyl; a hydroxy group; a linear chain or branched alkoxy group having 1 to 18 carbon atoms such as methoxy, ethoxy, propoxy, isopropoxy, butoxy, isobutoxy, sec-butoxy, tert-butoxy and dodecyloxy; a linear chain or branched alkylcarbonyl group having 2 to 18 carbon atoms such as acetyl, propionyl, butanoyl, 2-methylpropionyl, heptanoyl, 2-methylbutanoyl, 3-methylbutanoyl and octanoyl; an arylcarbonyl group having 7 to 11 carbon atoms such as benzoyl and naphthoyl; a linear chain or branched alkoxycarbonyl group having 2 to 19 carbon atoms such as methoxycarbonyl, ethoxycarbonyl, propoxycarbonyl, isopropoxycarbonyl, butoxycarbonyl, isobutoxycarbonyl, sec-butoxycarbonyl and tert-butoxycarbonyl; an aryloxycarbonyl group having 7 to 11 carbon atoms such as phenoxycarbonyl and naphthoxycarbonyl; an arylthiocarbonyl group having 7 to 11 carbon atoms such as phenylthiocarbonyl and naphthoxythiocarbonyl; a linear chain or branched acyloxy group having 2 to 19 carbon atoms such as

acetoxy, ethylcarbonyloxy, propylcarbonyloxy, isobutylcarbonyloxy, sec-butylcarbonyloxy, tert-butylcarbonyloxy and octadecylcarbonyloxy; an arylthio group having 6 to 20 carbon atoms such as phenylthio, biphenylthio, methylphenylthio, chlorophenylthio, bromophenylthio, fluorophenylthio, hydroxyphenylthio, methoxyphenylthio, naphthylthio, 4-[4-(phenylthio) benzoyl] phenylthio, 4-[4-(phenylthio) phenoxy] phenylthio, 4-[4-(phenylthio) phenyl] phenylthio, 4-(phenylthio) phenylthio, 4-benzoyl phenylthio, 4-benzoyl-chlorophenylthio, 4-benzoyl-methylthio phenylthio, 4-(methylthiobenzoyl) phenylthio and 4-(p-tert-butylbenzoyl) phenylthio; a linear chain or branched alkylthio group having 1 to 18 carbon atoms such as methylthio, ethylthio, propylthio, tert-butylthio, neopentylthio and dodecylthio; an aryl group having 6 to 10 carbon atoms such as phenyl, tolyl, dimethylphenyl and naphthyl; a heterocycle group having 4 to 20 carbon atoms such as thienyl, furanyl, pyranyl, xanthenyl, chromanyl, isochromanyl, xanthonyl, thioxanthonyl and dibenzofuranyl; an aryloxy group having 6 to 10 carbon atoms such as phenoxy and naphthyloxy; a linear chain or branched alkylsulfinyl group having 1 to 18 carbon atoms such as methylsulfinyl, ethylsulfinyl, propylsulfinyl, tert-pentylsulfinyl and octylsulfinyl; an arylsulfinyl group having 6 to 10 carbon atoms such as phenylsulfinyl, tolylsulfinyl and naphthylsulfinyl; a linear chain or branched alkylsulfonyl group having 1 to 18 carbon atoms such as methylsulfonyl, ethylsulfonyl, propylsulfonyl, isopropylsulfonyl, butylsulfonyl and octylsulfonyl; an arylsulfonyl group having 6 to 10 carbon atoms such as phenylsulfonyl, tolylsulfonyl (tosyl), naphthylsulfonyl; an alkyleneoxy groups; a cyano groups; a nitro groups; and halogens such as fluorine, chlorine, bromine and iodine.

Among the iodonium salt-based compounds, the aryl iodonium salt is preferable because of having high stability. Further, it is preferable that the aryl group has a substituent in order to improve the solubility to the dental adhesive composition. Specifically, a linear alkyl group such as methyl, propyl, octyl, decyl, undecyl, dodecyl and tridecyl, a branched alkyl group such as isopropyl, isobutyl, sec-butyl, tert-butyl, isopentyl, neopentyl, tert-pentyl and isohexyl, a functional group in which one or more of H of these is substituted with F, a perfluoroalkyl group and halogen is suitable as the substituent.

The structure of an anion portion of the iodonium salt-based compound is not particularly limited, and examples include those having atoms such as P, S, B, Al and Ga. From the viewpoint of safety, anions having As or Sb can be used, but they are not preferable in dental applications. Further, the anion preferably has an organic group such as an alkyl group and/or an alkoxy group and/or an aryl group, and further, most preferably has an organic group such as an alkyl group and/or an alkoxy group and/or an aryl group, in which at least one H is substituted with F. Since the iodonium salt-based compound having such an anion has high solubility in the dental adhesive composition, it is expected to preventing precipitation during low-temperature storage or long-term storage and to shorten the time for preparing due to dissolving in the composition in a short time.

Further, an iodonium salt-based compound of an anion having an organic group such as an alkyl group and/or an alkoxy group and/or an aryl group, in which at least one H is substituted with F can be expected to have higher solubility. When the photoacid generator is precipitated, there is a case that it may cause a decrease in light color stability and a decrease in flexural strength, and therefore it is not preferable. As the anion having an organic group such as an alkyl group and/or an alkoxy group and/or an aryl group, in which at least one H is substituted with F, an anion having any atom can be used. However, from the viewpoint of versatility and safety, those having one or more of P, S, B, Al and Ga are preferable.

Examples of the anion having no alkyl group and/or alkoxy group and/or aryl group include halogens such as chloride and bromide, perhalonic acids such as perchloric acid, aromatic sulfonic acids such as p-toluenesulfonate, camphorsulfonnic acids, nitrates, acetates, chloroacetates, carboxylates, phenolates, tetrafluoroborates, hexafluorophosphates, hexafluoroantimonates, hexafluoroarsenates and the like. Among these, p-toluenesulfonate, camphorsulfonic acid and carboxylate are preferably used.

Since the anionic part of $[A]^-$ of the iodonium salt-based compound of the formula (1) improves the solubility to the composition, it is preferable that the anion has an alkyl group and/or alkoxy group and/or aryl group, in which at least one H is substituted with F. Specifically, the number of carbon atoms of the alkyl group in the anion part of $[A]^-$ of the iodonium salt-based compound of the formula (2) is 1 to 8, and preferably 1 to 4. Specific examples include a linear alkyl group such as methyl, ethyl, propyl, butyl, pentyl and octyl, a branched alkyl group such as isopropyl, isobutyl, sec-butyl and tert-butyl, and a cycloalkyl group such as cyclopropyl, cyclobutyl, cyclopentyl and cyclohexyl. The ratio (F/H) of the number of hydrogen atoms to fluorine atoms in the alkyl group is 4 or more, and the ratio (F/H) of the number of hydrogen atoms to fluorine atoms in the alkyl group is preferably 9 or more. More preferably, all hydrogen atoms of the hydrocarbon are substituted with fluorine. An iodonium salt consisting of an anion having an alkyl group having a different ratio of a hydrogen atom and a fluorine atom may be compounded in the dental adhesive composition.

Further, specific examples of the alkyl group include a linear chain or branched perfluoroalkyl group such as $CF_3$, CF3CF2, $(CF_3)_2CF$, $CF_3CF_2CF_2$, $CF_3CF_2CF_2CF_2$, $(CF_3)_2CFCF_2$, $CF_3CF_2(CF_3)CF$ and $(CF_3)_3C$.

The number of carbon atoms of the alkyl group in the anion part of $[A]^-$ of the iodonium salt-based compound of the formula (2) is 1 to 8, and preferably 1 to 4. Specific examples include a linear alkoxy group such as methoxy, ethoxy, propoxy, butoxy, pentoxy and octoxy, and a branched alkoxy group such as isopropoxy, isobutoxy, sec-butoxy and tert-butoxy. The ratio (F/H) of the number of hydrogen atoms to fluorine atoms in the alkyl group is 4 or more, and

the ratio (F/H) of the number of hydrogen atoms to fluorine atoms in the alkyl group is preferably 9 or more. More preferably, all hydrogen atoms of the hydrocarbon are substituted with fluorine. An iodonium salt consisting of an anion having an alkoxy group having a different ratio of a hydrogen atom and a fluorine atom may be compounded in the dental adhesive composition.

Further, specific examples of the alkoxy group include a linear or branched perfluoroalkoxy group such as $CF_3O$, $CF_3CF_2O$, $CF_3CF_2CF_2O$, $(CF_3)_2CFO$, $CF_3CF_2CF_2CF_2O$, $(CF_3)_2CFCF_2O$, $CF_3CF_2(CF_3)CFO$, $CF_3CF_2CF_2CF_2CF_2O$ and $CF_3CF_2CF_2CF_2CF_2CF_2CF_2CF_2O$.

The phenyl group in the anion part of $[A]^-$ of the iodonium salt compound of the formula (2) may be a phenyl group, in which at least one H is substituted with fluorine atom, an alkyl group and/or an alkoxy group substituted with fluorine atom. The alkyl group and/or alkoxy group substituted with fluorine atom are preferably those described above. Specific examples of particularly preferable phenyl group include perfluorophenyl group such as pentafluorophenyl group ($C_6F_5$), trifluorophenyl group ($C_6H_2F_3$), tetrafluorophenyl group ($C_6HF_4$), trifluoromethylphenyl group ($CF_3C_6H_4$), bis (trifluoromethyl) phenyl group ($(CF_3)_2C_6H_3$), pentafluoroethyl phenyl group ($CF_3CF_2C_6H_4$), bis (pentafluoroethyl) phenyl group ($(CF_3)_2CrH_3$), trifluoromethyl fluorophenyl group ($CF_3C_6H_3F$), bistrifluoromethyl fluorophenyl group ($(CF_3)_2C_6H_2F$), pentafluoroethyl fluorophenyl group ($CF_3CF_2C_6H_3F$), bispentafluoroethyl fluorophenyl group ($(CF_3CF_2)_2CeH_2F$ and the like. An iodonium salt consisting of an anion having a phenyl group having a different ratio of a hydrogen atom and a fluorine atom may be compounded in the dental adhesive composition.

As specific examples of the anion portion of $[A]^-$ of the iodonium salt compound of the formula (3), examples of the anion having P include $[(CF_3CF_2)_3PF_3]^-$, $[(CF_3CF_2CF_2)_3PF_3]^-$, $[((CF_3)_2CF)_2PF_4]^-$, $[((CF_3)_2CF)_3PF_3]^-$, $[((CF_3)_2CF)_4PF_2]^-$, $[((CF_3)_2CFCF_2)_2PF4]^-$, $[((CF_3)_2CFCF_2)_3PF_3]^-$ and the like. Examples of the anion having S include $[(CF_3SO_2)_3C]^-$, $[(CF_3CF_2SO_2)_3C]^-$, $[(CF_3CF_2CF_2SO_2)_3C]^-$, $[(CF_3CF_2CF_2CF_2SO_2)_3C]^-$, $[CF_3CF_2CF_2CF_2SO_3]^-$, $[CF_3CF_2CF_2SO_3]^-$, $[(CF_3CF_2SO_2)_3C]^-$, $[(SO_2CF_3)_3N]^-$, $[(SO_2CF_2CF_3)_2N]^-$, $[((CF_3)C_6H_4)SO_3]^-$, $[SO_3(CF_2CF_2CF_2CF_2)SO_3]^{2-}$ and the like. Examples of the anion having B include $[B(C_6F_5)4]^-$, $[(C_6H_5)B(C_6F_5)_3]^-$, $[(C_6H_5)B((CF_3)_2C_6H_3)_3]^-$ and the like. Examples of an anion having Ga include $[((CF_3)_4Ga)]^-$, $[Ga (CrF_5)_4]^-$ and the like. Examples of anions having Al include $[((CF_3)_3CO)\ _4Al]^-$, $[((CF_3CF_2)_3CO)_4Al]^-$.

In the case that the dental adhesive composition of the present invention contains the (C) photoacid generator, the compounding amount of the (C) photoacid generator is preferably 0.01 to 10 parts by mass, more preferably 0.1 to 5 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water. When the compounding amount of the (C) photoacid generator is less than 0.01 parts by mass, there is a case that the polymerization promoting ability is poor and the curing becomes insufficient. When the compounding amount is more than 10 parts by mass, there is a case that storage stability decreases.

The photoacid generator that can be used in the dental adhesive composition of the present invention is not limited to the photoacid generator described in the specific example, and two or more types can be used in combination.

The dental adhesive composition of the present invention may comprise only an aryl iodonium salt which is a salt of an anion having an organic group and one or more atoms of P, B, Al, S, and Ga, and an aryl iodonium cation as the (C) photoacid generator. The dental adhesive composition of the present invention may comprise only an aryl iodonium salt which is a salt of an anion having an organic group in which at least one H may be substituted with F and one or more atoms of P, B, Al, S, and Ga, and an aryl iodonium cation as the (C) photoacid generator.

[(D) Photopolymerization Accelerator]

The dental adhesive composition of the present invention contains (D-1) aliphatic tertiary amine compound represented by formula (1) as the (D) photopolymerization accelerator. The dental adhesive composition of the present invention may contains (D) photopolymerization accelerator other than (D-1) aliphatic tertiary amine compound represented by formula (1). The (D) photopolymerization accelerator other than (D-1) aliphatic tertiary amine compound represented by formula (1) which is used for the dental adhesive composition of the present invention is not particularly limited as long as it has polymerization promoting ability, and any known photopolymerization accelerator commonly used in the dental field may be used without any limitation. As the photopolymerization accelerator, a primary to tertiary amine compound such as an aromatic amine compound and an aliphatic amine compound, an organic metal compound, a phosphine compound and the like can be used. Among these, an aromatic amine compound, a tertiary aliphatic amine compound and an organic metal compound are preferable because they are excellent in curability.

Aromatic amine compound refers to a compound in which one or more H of ammonia ($NH_3$) is replaced with an aromatic ring. Aromatic amine compound in which one H of $NH_3$ is substituted with an aromatic ring is classified into an aromatic primary amine compound, aromatic amine compound in which one H of $NH_3$ is substituted with an aromatic ring and one H of remaining two H is substituted with an aromatic ring or an alkyl group is classified into an aromatic secondary amine compound, and aromatic amine compound in which one H of $NH_3$ is substituted with an aromatic ring and remaining two H are substituted with an aromatic ring or an alkyl group is classified into an aromatic tertiary amine

compound.

Specific examples of the aromatic primary amine compound include aniline. Specific examples of the aromatic secondary amine compound include N-protected amino acid (ester) such as N-phenyl benzylamine, N-benzyl-p-anisidine, N-benzyl-o-phenetidine, N-phenylglycine ethyl and N-phenylglycine. Specific examples of the aromatic tertiary amine compound include N,N-dimethylaniline, N,N-diethylaniline, N,N-di-n-butylaniline, N,N-dibenzylaniline, p-N,N-dimethyl-toluidine, m-N,N-dimethyl-toluidine, p-N,N-diethyl-toluidine, p-bromo-N,N-dimethylaniline, m-chloro-N,N-dimethylaniline, p-dimethylamino benzaldehyde, p-dimethylamino acetophenone, p-dimethylamino benzoic acid, p-dimethylamino benzoic acid ethyl ester, p-dimethylamino benzoic acid isoamyl estel, p-dimethylamino benzoic acid 2-butoxyethyl, p-dimethylamino benzoic acid 2-ethylhexyl, p-dimethylamino benzoic acid amino ester, N,N-dimethyl anthranic acid methyl ester, N,N-dihydroxyethyl aniline, N,N-diisopropanol aniline, p-N,N-dihydroxyethyl-toluidine, p-N,N-diisopropanol-toluidine, p-dimethylamino phenyl alcohol, p-dimethylamino styrene, N,N-dimethyl-3,5-xylidine, 4-dimethylamino pyridine, N,N-dimethyl-$\alpha$-naphthylamine, N,N-dimethyl-$\beta$-naphthylamine and the like.

Specific examples of the above organic metal compound inlucde an orgic metal compound containing scandium (Sc), titanium (Ti), vanadium (V), manganese (Mn), iron (Fe), cobalt (Co), nickel (Ni), copper (Cu), tin (Sn), zinc (Zn) an/or zirconia (Zr), and an organic metal compound containing tin (Sn), vanadium (V) and/or copper (Cu) is preferable. Specific examples of the organic metal compound containing tin (Sn) include dibutyl-tin-diacetate, dibutyl-tin-dimaleate, dioctyl-tin-dimaleate, dioctyl-tin-dilaurate, dibutyl-tin-dilaurate, dioctyl-tin-diversate, dioctyl-tinS,S'-bis-isooctyl mercapto acetate, tetramethyl-1,3-diacetoxy distanoxane and the like. Specific examples of the organic metal compound containing vanadyl (V) include acetylacetone vanadium, divanadium tetraoxide, vanadyl acetylacetonate, vanadyl stearate oxide, vanadyl oxalate, vanadyl sulphate, oxobis (1-phenyl-1,3-butandionate) vanadium, bis (maltlate) oxovanadium, vanadium pentoxide and sodium metavanadate. Specific examples of the organic metal compound containing copper (Cu) include copper acetylacetone, copper naphthenate, copper octylate, copper stearate and copper acetate.

The phosphine compound refers to a compound which is trisubstituted on P atom with organic groups, and the aromatic phosphine compound refers to a compound which is substituted on P atom with a phenyl group which may have one or more substituents. Specific examples of the phosphine compound include trimethylphosphine, tributylphosphine, trihexylphosphine, tri-n-octylphosphine, tricyclohexylphosphine, tri (2-thienyl) phosphine, diphenylpropyl phosphine, di-tert-butyl (3-methyl-2-butenyl) phosphine, methyldiphenyl phosphine, triphenyl phosphine, 2-(diphenylphosphino) styrene, 3-(diphenylphosphino) styrene, 4-(diphenylphosphino) styrene, allyldiphenyl phosphine, 2-(diphenylphosphino) benzaldehyde, 3-(diphenylphosphino) benzaldehyde, 4-(diphenylphosphino) benzaldehyde, 2-(phenylphosphine) benzoic acid, 3-(phenylphosphino) benzoic acid, 4-(phenylphosphino) benzoic acid, tris (2-methoxyphenyl) phosphine, tris (3-methoxyphenyl) phosphine, tris (4-methoxyphenyl) phosphine, 2-(diphenylphosphino) biphenyl, tris (4-fluorophenyl) phosphine, tri (o-trill) phosphine, tri (m-trill) phosphine, tri (p-trill) phosphine, 2-(dimethylamino) phenyldiphenyl phosphine, 3-(dimethylamino) phenyldiphenyl phosphine, 4-(dimethylamino) phenyldiphenyl phosphine, 2,2'-bis (diphenylphosphino) biphenyl, bis [2-(diphenylphosphino) phenyl] ether and the like. Among them, triphenylphosphine, 4-(phenylphosphino) benzoic acid, tri (o-tolyl) phosphine, tri (m-tolyl) phosphine and tri (p-tolyl) phosphine are preferable.

Aliphatic amine compounds refer to compounds in which one or more H of ammonia ($NH_3$) are substituted with alkyl group. As for the alkyl group, $CH_3$- and -$CH_2$- are classified as a primary alkyl group, the one in which one H of -$CH_2$- is substituted with a substituent is classified as a secondary alkyl group, and the one in which two H of -$CH_2$- are substituted with substituents is classified as a tertiary alkyl group. Aliphatic amine in which one H of $NH_3$ is substituted with an alkyl group is classified into an aliphatic primary amine compound, aliphatic amine compound in which two H of $NH_3$ are substituted with an alkyl group is classified into an aliphatic secondary amine compound, and aliphatic amine compound in which three H of $NH_3$ are substituted with an alkyl group is classified into an aliphatic tertiary amine compound.

Specific examples of the aliphatic primary amine compound include amino acid or amino acid ester such as benzhydrylamine, triphenylmethylamine and glycine. Specific examples of the aliphatic secondary amine compound include dibenzylamine, N-benzyl-1-phenylethylamine, bis (1-phenylethyl) amine, bis (4-cyanobenzyl) amine, N-benzyl protected amino acid and N-benzyl protected amino acid ester. Specific examples of the aliphatic tertiary amine compound include tributylamine, tripropylamine, triethylamine, N,N-dimethyl hexylamine, N,N-dimethyl dodecylamine, N,N-dimethyl stearylamine, N-[3-(dimethylamino) propyl] acrylamide, N,N-dimethyl formamide dimethylacetal, N,N-dimethylacetamide dimethylacetal, N,N-dimethylformamide diethylacetal, N,N-dimethylformamide dipropylacetal, N,N-dimethylformamide di-tert-butylacetal, 1-(2-hydroxyethyl) ethyleneimine, N,N-dimethyl ethanolamine, N,N-dimethyl isopropanolamine, N,N-diisopropyl ethanolamine, N-methyl diethanolamine, N-ethyl diethanolamine, N-ethyl diethanolamine, N-butyl diethanolamine, N-lauryl diethanolamine, N-stearyl diethanolamine, triethanolamine, triisopropanolamine, tribenzylamine, dibenzylglycine ethylester, N'-(2-hydroxyethyl)-N,N,N'-trimethylethylene diamine, 2-(dimethylamino)-2-methyl-1-propanol, N,N-dimethyl-2,3-dihydroxy propylamine, N,N-diethylethanolamine, 1-methyl-3-pyrrolidinol, 1-(2-hydroxyethyl) pyrrolidine, 1-isopropyl-3-pyrrolidinol, 1-piperidin ethanol, 2-[2-(dimethylamino) ethoxy] ethanol, N,N-dimethylglycine, N,N-dimethylglycine methyl, N,N-diethylglycine methyl, N,N-dimethylglycine ethyl, N,N-diethylglycine sodium, 2-(dimethylamino) ethylacetate, N-methylimimino diacetic acid, N,N-dimethylamino ethylacrylate, N,N-diethylamino ethylmethacrylate, N,N-diisopropylamino ethylmethacrylate, N,N-dibutylamino ethylmethacrylate, N,N-dibenzylamino ethylmethacrylate, 3-dimethylamino propionitrile, tris (2-cyanoethyl) amine, N,N-dimethyl allylamine, N,N-diethyl allylamine and triallylamine.

The dental adhesive composition of the present invention contains (D-1) aliphatic tertiary amine compound represented by formula (1) as the (D) photopolymerization accelerator.

[Formula (1)]

[Chemical formula 2]

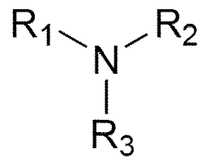

In formula, $R_1$ represents a substituent consisting of three or more carbons having an electron-attracting group at α-carbon and/or β-carbon of an amine starting from N, $R_2$ represents a substituent consisting of three or more carbons which may have an electron-attracting group, $R_3$ represents a substituent consisting of one or more carbons which may have an electron-attracting group, and α-carbon of N in the formula (1) is not an electron-attracting group.

The electron-attracting group in $R_1$ may be a substituent selected from a functional group selected from the group consisting of a hydroxyl group, a carboxyl group, a vinyl group, an aryl group and a halogen, and, an organic group which is bonded via an ether bond, an ester bond, a urethane bond or a urea bond and may have -OH group, -O-group, -C(O)-group, -S-group, -NH-C(O)-NH-group, -C(O)-O-group, -O-C(O)-group, -OC (O)-NH-group, -NH-C(O)-O-group, an aromatic hydrocarbon group, or a polymerizable functional group capable of radical polymerization.

Conventionally, an aliphatic tertiary amine such as dimethylaminoethyl methacrylate or triethanolamine have been compounded to the dental adhesive composition for the purpose of promoting photopolymerization, improving solubility and improving storage stability. However, when the above-mentioned conventional aliphatic tertiary amine and (A-1) polymerizable monomer having an acidic group are compounded in a dental adhesive composition, there is a case that it is confirmed that the adhesive property with respect to a tooth substance and a prosthetic device is reduced after long-term storage. As a result of study by the present inventors, it has been found that good adhesive property with respect to a tooth substance and a prosthetic device can be maintained even when stored for a long period of time by using the (D-1) aliphatic tertiary amine compound represented by formula (1) having an electron-attracting group and steric hindrance as compared with the conventionally used tertiary aliphatic amine compound. Further, the present inventors have been found that there is a case in which the (D-1) aliphatic tertiary amine compound represented by formula (1) exhibits the curing accelerating ability and therefore exhibits good adhesive property and mechanical property in the case of containing (B) photosensitizer and/or (C) photoacid generator in the dental adhesive composition or in the case of using a dental adhesive composition in combination with a dental curable composition containing (B) photosensitizer and/or (C) photoacid generator, and have completed the present invention.

In the (D-1) aliphatic tertiary amine compound represented by formula (1), $R_1$ represents a substituent consisting of three or more carbons having an electron-attracting group at α-carbon and/or β-carbon of an amine starting from N, $R_2$ represents a substituent consisting of three or more carbons which may have an electron-attracting group, $R_3$ represents a substituent consisting of one or more carbons which may have an electron-attracting group. Here, $R_2$ and $R_3$ may be a carbon alicyclic compound or a complex alicyclic compound in which three or more carbons are cyclically bonded. For the meaning of α-position and/or β-position starting from N, the carbon bonded to N is the α-position carbon and the carbon bonded to the α-position carbon is the β-position carbon. The number of the carbon in the substituent consisting of three or more carbons includes the carbon contained in the electron-attracting functional group. Further, N represented by the formula (1) does not bond with an electron-attracting group without passing through a hydrocarbon group.

The electron-attracting group refers a substituent that easily attracts an electron from the bonded atom side. Examples of the electron-attracting group include a hydroxyl group, a thiol group, a nitro group, a carbonyl group, a carboxyl group, a sulfonyl group, a cyano group, an aryl group, an amino group, a halogen, and an organic group bonded via an unsaturated bond such as a vinyl group and a propargyl group, an ether bond, an ester bond, a urethane bond or a urea bond.

Among the electron-attracting groups in the formula (1), the electron-attracting group of the α-carbon and/or β-carbon of $R_1$ may be a functional group selected from the group consisting of a hydroxyl group, a carboxyl group, a vinyl group, an aryl group and a halogen, and, an organic group which is bonded via an ether bond, an ester bond, a urethane bond or a urea bond. This organic group may have -OH group, -O-group, -C(O)-group, -S-group, -NH-C(O)-NH-group, - C(O)-O-group, -O-C(O)-group, -OC (O)-NH-group, -NH-C(O)-O-group, an aromatic hydrocarbon group, or a polymerizable functional group capable of radical polymerization. Preferable is a hydroxyl group, a carboxyl group, an aryl group and an organic group having an ether bond, an ester bond, a urethane bond or a urea bond. More preferable is an aryl group, a carboxyl group and an organic group having an ester bond or a urethane bond because steric hindrance is large or high electron attraction is expected. Furthermore preferable is a tertiary aliphatic amine compound in which the amino group is disubstituted or more with an aryl group which may have a substituent.

When $R_2$ and $R_3$ have an electron-attracting group, the electron-attracting group may be a functional group selected from the group consisting of a hydroxyl group, a carboxyl group, a vinyl group, an aryl group and a halogen, and, an organic group which is bonded via an ether bond, an ester bond, a urethane bond or a urea bond. This organic group may have -OH group, -O-group, -C(O)-group, -S-group, -NH-C(O)-NH-group, -C(O)-O-group, -O-C(O)-group, -OC (O)-NH-group, -NH-C(O)-O-group, an aromatic hydrocarbon group, or a polymerizable functional group capable of radical polymerization. Preferable is a hydroxyl group, a carboxyl group, an aryl group and an organic group having an ether bond, an ester bond, a urethane bond or a urea bond. More preferable is an aryl group, a carboxyl group and an organic group having an ester bond or a urethane bond because steric hindrance is large or high electron attraction is expected. Furthermore preferable is a tertiary aliphatic amine compound in which the amino group is disubstituted or more with an aryl group which may have a substituent.

In the formula (1), an aliphatic tertiary amine compound in which $R_1$ and $R_2$ have an aliphatic substituent consisting of three or more carbons having an electron-attracting group at α-carbon and/or β-carbon is preferable. In this case, further improvement in storage stability can be expected. Furthermore, an aliphatic tertiary amine compound in which the amino group is disubstituted or more with an aryl group which may have a substituent, that is, an aliphatic tertiary amine compound having an aryl group which may have a substituent at α-carbon and/or β-carbon is preferable. Specific examples include tribenzylamine, dibenzylglycine ester compound and dibenzylaminoalkyl (meth) acrylate. A compound having such a structure is expected to have an effect of improving the adhesive strength by improving the curability of the interface in the case that a dental adhesive composition contains a photoacid generator or in the case that a dental adhesive composition or a dental photocurable composition for use with and contacting with a dental adhesive composition contains a photoacid generator.

Specific examples of the (D-1) aliphatic tertiary amine compound represented by formula (1) include triisopropanolamine, 2-(dibutylamino)-1-phenyl-1-propanol, 1-[(3,3-diphenylpropyl) (methyl) amino]-2-methyl-2-propanol, 3,3',3''-nitrilotripropionic acid, N-benzyl-3,3'-iminodipropionic acid, 1-benzhydrill azetidine-3-carboxylic acid, 1-benzyl-3-pyrrolidone, 1-(2-phenylethyl)-4-piperidone, 1-benzylpiperidin, 1-phenyl-2-(1-pyrrolidinyl) propan-1-ol, 2-[hydroxy (diphenyl) methyl]-1-methylpyrrolidin, N,N,N',N'-tetrakis (2-hydroxypropyl) ethylenediamine, N,N,N',N'',N''-pentakis (2-hydroxypropyl) diethylenetriamine, 2-piperidino-1,1,2-triphenylethanol, 2-[benzyl (methyl) amino]-1-phenylethanol, 2-(dibenzylamino)-3-phenyl-1-propanol, 2,6-bis [2-(hydroxy diphenylmethyl)-1-pyrrolidinyl-methyl]-4-methylphenol, 2-benzyl-1,2,3,4-tetrahydro isoquinolin-8-carboxylic acid, 2-benzyl-1,2,3,4-tetrahydro isoquinolin-3-carboxylic acid, 2-(dibenzylamino) propionaldehyde, 3-(dibenzylamino)-1-propanol, 2-(dibenzylamino)-1-propanol, 2-(N,N-dibenzylamino)-3-methylbutanol, 1-[(dibenzylamino) methyl]-2-naphthanol, 2-(dibenzylamino)-4-methyl-1-pentanol, 4-dibenzylamino-cyclohexanone, N,N-dibenzyl-1,4-dioxaspiro [4.5] decane-8-amine, N,N-dipropyl-L-alanine, N,N-dibenzyl-2-aminoethanol, N,N-dibenzyl glycineethyl, tribenzylamine, triallylamine, 1,1'-(methylimino) dipropane-2-ol, 1-(benzyl (2-methylallyl) amino)-2- methylpropan-2-ol, 2-piperidino-1,1,2-triphenylethanol, N,N-dibenzylaminoethanol, N,N-dibenzylaminopropanol and 3-(N,N-dibenzylamino) propyltriethoxysilane. In addition, examples include a transesterification product of tertiary amine compound containing OH group such as N,N-dibenzyl aminoethanol, N,N-dibenzyl aminopropanol, N,N-dibutyl ethanolamine, N,N-diisopropyl aminoethanol, N-methyldiethanolamine, N-ethyldiethanolamine, N-butyldiethanolamine and N-tert-butyl diethanolamine and ester compound containing (meth) acrylic acid ester such as methyl methacrylate and butyl methacrylate, an urethane compound synthesized by reaction of tertiary amine compound containing OH group and compound having isocyanate such as 2-isocyanatoethyl (meth) acrylate and 1,1-(bisacryloyloxymethyl) ethylisocyanate, a transesterification product of an amine compound having a carboxyl group or ester bond such as 3,3',3''-nitrillo tripropionic acid, N-benzyl-3,3'-iminodipropionic acid, N-methylimino diacetic acid, N-(2-hydroxyethyl) iminodiacetic acid, N-(2-carboxyethyl) iminodiacetic acid, N, N-dipropyl-L-alanine and N,N-dibenzylglycine and a compound having an OH group such as an alcohol and 2-hydroxyethyl methacrylate, and an urea compound synthesized by a reaction of a secondary amine such as diisopropylamine and dibenzylamine and a compound having an isocyanate such as 2-isocyanatoethyl (meth) acrylate or 1,1-(bisacryloyl oxymethyl) ethyl isocyanate. Among these, a transesterification product of a tertiary amine compound having OH group such as dibenzylaminoethyl methacrylate and dibenzylaminopropyl methacrylate and an ester compound having a polymerizable group such as (meth) acrylic acid ester, an urethane compound synthesized by reaction with compound having isocyanate such as 2-isocyanatoethyl (meth) acrylate and 1,1-(bisacryloyl oxymethyl) ethylisocyanate, N,N-dibenzylglycine ester compound, triisopropanolamine and tribenzylamine are preferable.

When $R_2$ or $R_3$ of the formula (1) is an aliphatic tertiary amine compound having an alkoxysilyl group, it can also be used as a surface treatment agent for a filler. Specific examples include 3-(N,N-dibenzylamino) propyltriethoxysilane having both a benzylamino group and an alkoxysilyl group, and synthetic example include synthesizing by benzyl-protecting aminopropylethoxysilane. A dental adhesive composition containing a filler surface-treated with 3-(N,N-dibenzylamino) propyltriethoxysilane can be expected to exhibit a high curing depth as well as a dental adhesive composition containing 3-(N,N-dibenzylamino) propyltriethoxysilane. Even when the aliphatic tertiary amine compound is covalently immobilized on the filler in this way, the storage stability is lowered if the structure of the formula (1) is not satisfied.

The compounding amount of the (D-1) aliphatic tertiary amine compound represented by formula (1) in the case that the dental adhesive composition is one pack type dental adhesive composition is preferably 0.01 to 20 parts by mass, more preferably 0.1 to 10 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water, contained in the dental adhesive composition. When the compounding amount of the (D-1) aliphatic tertiary amine compound represented by formula (1) in the dental adhesive composition is less than 0.01 parts by mass, there is a case that the expected effect of improving the storage stability is not exhibited, and when the compounding amount of the (D-1) aliphatic tertiary amine compound represented by formula (1) in the dental adhesive composition exceeds 20 parts by mass, there is a case that the adhesive strength decreases.

The compounding amount of the (D-1) aliphatic tertiary amine compound represented by formula (1) in the case that the dental adhesive composition is two packs type dental adhesive composition consisting of a first pack and a second pack is preferably 0.02 to 20 parts by mass, more preferably 0.1 to 10 parts by mass in the first pack, with respect to 100 parts by mass of total of the (A) polymerizable monomer and the (F) volatile organic solvent contained in the first pack or with respect to 100 parts by mass of total of the (A) polymerizable monomer, the (F) volatile organic solvent and (G) water in the case of containing the (G) water contained in the first pack. When the compounding amount is less than 0.02 parts by mass, there is a case that adhesive property to the tooth substance and the prosthetic device is not imparted, and when the compounding amount exceeds 20 parts by mass, there is a case that the storage stability deteriorates.

The type of the (D) photopolymerization accelerator may be appropriately selected according to the type and the compounding amount of other components to be combined. In addition, the (D) photopolymerization accelerator may be used not only singly but also in combinations of two or more.

The dental adhesive composition of the present invention may contain only the (D-1) aliphatic tertiary amine compound represented by formula (1) as the (D) photopolymerization accelerator. The dental adhesive composition of the present invention may contain only the (D-1) aliphatic tertiary amine compound represented by formula (1) which is an aliphatic tertiary amine compound in which $R_1$ and $R_2$ have an aliphatic substituent consisting of three or more carbons having an electron-attracting group at α-carbon and/or β-carbon, as the (D) photopolymerization accelerator. The dental adhesive composition of the present invention may contain only the (D-1) aliphatic tertiary amine compound represented by formula (1) which is an aliphatic tertiary amine compound in which $R_1$ and $R_2$ have an aryl group which may have a substituent, at α-carbon and/or β-carbon, as the (D) photopolymerization accelerator.

There is no problem even if these (B) photosensitizers, (C) photoacid generators and (D) photopolymerization accelerators, which are polymerization initiators, are subjected to a secondary treatment such as finely pulverization, adsorption on a carrier and encapsulation in a microcapsule, if necessary. Furthermore, these photo polymerization initiators can be used not only singly but also in combinations of two or more, regardless of the polymerization manner or the polymerization method.

[(E) Filler]

The dental adhesive composition of the present invention may contain (E) filler, a known filler commonly used can be used without any limitation.

The type of the (E) filler is not limited as long as it is a known filler, and a filler suitable for the application can be compounded, and it is preferable that a filler such as an inorganic filler, an organic filler and an organic-inorganic composite filler is compounded. These fillers can be used not only singly but also in combinations of a plurality thereof regardless of the types of the fillers.

As the above described inorganic filler, the chemical composition is not particularly limited, but specific examples include silicon dioxide, alumina, silica-titania, silica-titania-barium oxide, silica-zirconia, silica-alumina, lanthanum glass, borosilicate glass, soda glass, barium glass, strontium glass, glass ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass and the like. Particularly, barium fluoroaluminosilicate glass, strontium fluoroaluminosilicate glass, fluoroaluminosilicate glass and the like, which are used in dental glass ionomer cement, resin reinforced glass ionomer cement and resin cement and the like, can also be suitably used. The fluoroaluminosilicate glass as used herein has a basic structure of silicon oxide and aluminum oxide and contains an alkali metal for introducing non-crosslinked oxygen. The fluoroaluminosilicate glass further has an alkaline earth metal including strontium and fluorine as modified/coordinated ions. The fluoroaluminosilicate glass may be also a composition in which a lanthanoid series element is incorporated into the skeleton in order to impart further radiopacity. This lanthanoid series element also participates in the composition as a modified/coordinated ion.

Specific examples of the organic filler include polymers such as polymethyl methacrylate, polyethyl methacrylate, methyl methacrylate-ethyl methacrylate copolymer, ethyl methacrylatebutyl methacrylate copolymer, methyl methacrylate-trimethylolpropane methacrylate copolymer, polyvinylchloride, polystyrene, chlorinated polyethylene, nylon, polysulfone, polyethersulfone and polycarbonate.

In addition, examples of the organic/inorganic composite filler include one obtained by covering the surface of a filler with a polymerizable monomer by polymerization, one obtained by mixing a filler and a polymerization monomer and polymerizing the monomer, and thereafter grinding the resultant to a proper particle size, or one obtained by dispersing a filler in a polymerizable monomer in advance for emulsion polymerization or suspension polymerization, but are not limited thereto at all.

The above described (E) filler can be treated with a surface treatment material represented by a silane coupling material in order to improve the affinity to the polymerizable monomer, the dispersibility in the polymerizable monomer, and the mechanical strength and water resistance of the cured product. The surface treatment material and the surface treatment method are not particularly limited, and known methods can be adopted without limitation. As a silane coupling material used for surface treatment of the filler, methyltrimethoxysilane, methyltriethoxysilane, methyltrichlorosilane, dimethyldichlorosilane, trimethylchlorosilane, vinyltrichlorosilane, vinyltriethoxysilane, vinyltris (2-methoxyethoxy) silane, 3-methacryloyloxypropyl trimethoxysilane, 3-chloropropyl trimethoxysilane, 3-glycidoxypropyl trimethoxysilane, 3-(meth) acryloxypropyl trimethoxysilane, 8-(meth) acryloxyoctyl trimethoxysilane, 11-(meth) acryloxiundecyl trimethoxysilane, hexamethyldisilazane and the like are preferable. In addition to the silane coupling material, surface treatment of the filler can be performed by a method using a titanate coupling material or an aluminate coupling material. The treatment amount of the surface treatment material in the filler is preferably 0.01 to 30 parts by mass, more preferably 0.5 to 20 parts by mass with respect to 100 parts by mass of the filler before treatment.

The shape of the filler is not particularly limited, and any shape of the filler such as an amorphous, a spherical shape, a needle shape, a plate shape, a crushed shape or a scale shape can be used. The average particle diameter of the filler is preferably 0.01 μm to 50 μm, more preferably 0.01 μm to 30 μm, still more preferably 0.05 μm to 20 μm, and more preferably 0.05 μm to 10 μm.

When (E) filler is compounded in the dental adhesive composition, the compounding amount is preferably 0.1 to 50 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water. When the compounding amount of the filler is less than 0.1 parts by mass, there is a case that the effect of improving the mechanical strength and of exhibiting the thixotropy by compounding the filler becomes poor. When the compounding amount is more than 50 parts by mass, there is a case that adhesive strength decrease.

The dental adhesive composition of the present invention contains (F) volatile organic solvent. The volatile organic solvent is used for the purpose of dissolving a polymerizable monomer component containing the (A-1) polymerizable monomer having an acidic group and water in the composition or reducing the viscosity of the dental adhesive composition. The volatile organic solvent usually has a boiling point of 150 °C or less under normal pressure and a solubility of 5% by weight or more with respect to water at 25 °C, more preferably 30% by weight or more, and, most preferably, an organic solvent which can dissolved in water at an arbitrary ratio is used. Among these, a water-soluble volatile organic solvent having a boiling point of 100 °C or less under normal pressure is preferable, and specific examples include ethanol, methanol, 1-propanol, isopropyl alcohol, acetone, methylethyl ketone, 1,2-dimethoxyethane, 1,2-diethoxyethane and tetrahydrofuran. Further, among the above-mentioned volatile organic solvents, ethanol, isopropyl alcohol, acetone and methylethyl ketone are more preferable.

The (F) volatile organic solvent may be used alone or in combination of two or more. The compounding amount of the (F) volatile organic solvent in the case that the dental adhesive composition is one pack type dental adhesive composition is preferably 1 to 99.9 parts by mass, more preferably 5 to 99.9 parts by mass, most preferably 10 to 80 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water, contained in the dental adhesive composition.

The compounding amount of the (F) volatile organic solvent in the case that the dental adhesive composition is two packs type dental adhesive composition consisting of a first pack and a second pack is preferably 1 to 99.9 parts by mass, more preferably 5 to 99.9 parts by mass, most preferably 10 to 80 parts by mass in the first pack, with respect to 100 parts by mass of total of the (A) polymerizable monomer and the (F) volatile organic solvent contained in the first pack or with respect to 100 parts by mass of total of the (A) polymerizable monomer, the (F) volatile organic solvent and (G) water in the case of containing the (G) water.

The dental adhesive composition of the present invention may contain (G) water in order to improve the wettability to the adherend, and specific examples thereof include deionized water and distilled water. Further, the compounding amount is preferably 5 to 80 parts by mass, more preferably 10 to 60 parts by mass with respect to 100 parts by mass of total of the (A) polymerizable monomer, the (F) volatile organic solvent and (G) water contained in the dental adhesive composition.

The dental adhesive composition of the present invention may contain a chemical polymerization initiator. Specific examples of an organic peroxide as chemical polymerization initiator include diacyl peroxides, peroxy esters, dialkyl peroxides, peroxy ketals, ketone peroxides, peroxy dicarbonates, and hydro peroxides. Specific examples of diacyl peroxides include acetyl peroxide, isobutyryl peroxide, benzoyl peroxide, decanoyyl peroxide, 3,5,5-trimethylhexanoyl peroxide, 2,4-dichlorobenzoyl peroxide, and lauroyl peroxide and the like. Specific examples of peroxyesters include α-cumylperoxy neodecanoate, t-butylperoxy neodecanoate, t-butylperoxy pivalate, 2,2,4-trimethylpentyl peroxy-2-ethyl hexanoate, t-amylperoxy-2-ethylhexanoate, t-butylperoxy-2-ethylhexanoate, di-t-butylperoxy isophthalate, dit-butylperoxy hexahydro terephthalate, t-butylperoxy-3,3,5-trimethyl hexanoate, t-butylperoxy acetate, t-butylperoxy benzoate and t-butylperoxy maleric acid. Specific examples of dialkyl peroxides include di-t-butyl peroxide, dicumyl peroxide, t-butylcumyl peroxide, 2,5-dimethyl-2,5-di (t-butylperoxy) hexane, 1,3-bis (t-butylperoxy isopropyl) benzene, 2,5-dimethyl-2,5-di (t-butylperoxy)-3-hexyne and the like. Specific examples of peroxyketals include 1,1-di (t-butylperoxy) cyclohexane, 2,2-di (t-butylperoxy) butane, and n-butyl-4,4-(t-butylperoxy) parerate, 1,1-di (t-amylperoxy) cyclohexane and the like. Specific examples of ketone peroxides include methyl ethyl ketone peroxide, methyl isobutyl ketone peroxide, methyl cyclohexanone peroxide, cyclohexanone peroxide and the like. Specific examples of peroxydicarbonates include di-3-methoxyperoxy dicarbonate, di-2-ethylhexylperoxy dicarbonate, bis (4-t-butylcyclohexyl) peroxy dicarbonate, diisopropylperoxy dicarbonate, di-n-propylperoxy dicarbonate, di-2-ethoxyethylperoxy dicarbonate, diallylperoxy dicarbonate and the like. Specific examples of hydroperoxides include 2,5-dimethyl hexane-2,5-dihydroperoxide, diisopropylbenzene hydroperoxide, cumene hydroperoxide, t-butyl hydroperoxide and 1,1,3,3-tetramethyl butylhydroperoxide.

As the organic peroxide, the above-mentioned organic peroxides may be used alone, or two or more kinds of organic peroxides may be used in combination. Among these organic peroxides, benzoyl peroxide and cumene hydroperoxide are preferable from the viewpoint of curability. The compounding amount of the organic peroxide as a chemical polymerization initiator is preferably set to 0.1 to 5 parts by mass, more preferably set to 0.3 to 3 parts by mass with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water from the viewpoint of improving the curability. When the compounding amount of the organic peroxide is more than 5 parts by mass, it may be difficult to ensure sufficient operation time. On the other hand, when the compounding amount of the organic peroxide is less than 0.1 parts by mass, there is a case in which mechanical strength may be insufficient.

In the dental adhesive composition of the present invention, in order to further improve the curability, a chemical polymerization accelerator may further be compounded. Examples of chemical polymerization accelerators include a transition metal compound of the group 4 in the periodic table, a thiourea derivative an aliphatic amine, an aromatic amine, a sulfinic acid and a salt thereof, a borate compound, a sulfur-containing reductive inorganic compound, a nitrogen-containing reductive inorganic compound, a barbituric acid derivative, a triazine compound, a halogen compound and the like. The compounding amount of the chemical polymerization accelerator is preferably 0.01 to 5 parts by mass, more preferably 0.1 to 3 parts by mass, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water.

The transition metal compound of the period 4 in the periodic table as a chemical polymerization accelerator refers to a metal compound of groups 3 to 12 of the period 4 in the periodic table, and specifically, each metal compound of scandium (Sc), titanium (Ti), vanadium (V), manganese (Mn), iron (Fe), cobalt (Co), nickel (Ni), copper (Cu), and zinc (Zn) can be used without any limitation. Although each of the above transition metal element may have a multiple valences, they can be added to the dental adhesive composition of the present invention as long as the valence is stable. Examples include Sc (trivalent), Ti (tetravalent), V (trivalent, tetravalent or pentavalent), Cr (divalent, trivalent or hexavalent), Mn (divalent to heptavalent), Fe (divalent or trivalent), Co (divalent or trivalent), Ni (divalent), Cu (monovalent or divalent), Zn (divalent). Specific examples of the transition metal compound include scandium iodide (trivalent) and the like as a scandium compound, titanium chloride (tetravalent), titanium tetraisopropoxide (tetravalent) and the like as titanium compounds, acetylacetone vanadium (trivalent), divanadium tetraoxide (tetravalent), vanadylacetyl acetonate (tetravalent), vanadium stearate oxide (tetravalent), vanadyl oxalate (tetravalent), vanazyl sulfate (tetravalent), oxobis (1-phenyl-1,3-butandionate) vanadium (tetravalent), bis (maltlate) oxovanadium (tetravalent), vanadium pentoxide (pentavalent), sodium metavanadate (pentavalent) and the like as a vanadium compound, manganese acetate (divalent), manganese naphthenate (divalent) and the like as manganese compounds, iron acetate (divalent), iron chloride (divalent), iron acetate (trivalent), iron chloride (trivalent) and the like as an iron compound, cobalt acetate (divalent), cobalt naphthenate (divalent) and the like as a cobalt compound, nickel chloride (divalent) and the like as a nickel compound, copper

chloride (monovalent), copper bromide (monovalent), copper chloride (divalent), copper acetate (divalent) and the like as a copper compound, and zinc chloride (divalent), zinc acetate (divalent) and the like as a zinc compound.

Among these, a trivalent or tetravalent vanadium compound and a divalent copper compound are preferable. Among them, because of having higher polymerization accelerating ability, a trivalent or tetravalent vanadium compound is more preferable, and a tetravalent vanadium compound is most preferable. A plurality of kinds of these transition metal compounds in the period 4 in the periodic table may be used in combination, if necessary. The compounding amount of transition metal compound is preferably 0.0001 to 1 parts by mass with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water. When the compounding amount is less than 0.0001 parts by mass, there is a case where the polymerization accelerating effect is insufficient, and when the compounding amount exceeds 1 part by mass, there is a case where it causes discoloration or gelation of the dental adhesive composition and the storage stability is lowered.

Any known thiourea derivatives can be used as the thiourea derivative as the chemical polymerization accelerator without any limitation. Specific examples of the thiourea derivatives include dimethylthiourea, diethylthiourea, tetramethylthiourea, (2-pyridyl) thiourea, N-methylthiourea, ethylenethiourea, N-allylthiourea, N-allyl-N'-(2-hydroxyethyl) thiourea, N-benzylthiourea, 1,3-dicyclohexyl thiourea, N,N'-diphenylthiourea, 1,3-di (p-tolyl) thiourea, 1-methyl-3-phenylthio-urea, N-acetylthiourea, N-benzoylthiourea, diphenylthiourea, dicyclohexylthiourea and the like. Among these, (2-pyridyl) thiourea, N-acetylthiourea and N-benzoylthiourea are preferable. A plurality of kinds of these thiourea derivatives can be used in combination, if necessary. The compounding amount of the thiourea derivative is preferably 0.1 to 5 parts by mass with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water. When the compounding amount is less than 0.1 parts by mass, there is a case where the ability as a polymerization accelerator is insufficient, and when the compounding amount exceeds 5 parts by mass, the storage stability may be lowered.

Examples of sulfinic acid and its salt include p-toluene sulfinic acid, sodium p-toluene sulfinate, potassium p-toluene sulfinate, lithium p-toluene sulfinate, calcium p-toluene sulfinate, benzenesulfinic acid, sodium benzene sulfinate, potassium benzene sulfinate, lithium benzenesulfinate, calcium benzenesulfinate, 2,4,6-trimethyl benzenesulfinic acid, sodium 2,4,6-trimethyl benzenesulfinate, potassium 2,4,6-trimethyl benzenesulfinate, lithium 2,4,6-trimethyl benzenesulfinate, calcium 2,4,6-trimethyl benzenesulfinate, 2,4,6-triethyl benzenesulfinic acid, sodium 2,4,6-triethyl benzenesulfinate, potassium 2,4,6-triethyl benzenesulfinate, lithium 2,4,6-triethyl benzenesulfinate, calcium 2,4,6-triethyl benzenesulfinate, 2,4,6-triisopropyl benzenesulfinic acid, sodium 2,4,6-triisopropyl benzenesulfinate, potassium 2,4,6-triisopropyl benzenesulfinate, lithium 2,4,6-triisopropyl benzenesulfinate, calcium 2,4,6-triisopropyl benzenesulfinate and the like. Among them, sodium benzenesulfinate, sodium p-toluenesulfinate, and sodium 2,4,6-triisopropyl benzenesulfinate are particularly preferable.

As the borate compound, specific examples of the borate compound having one aryl group in one molecule include trialkylphenylboron, trialkyl (p-chlorophenyl) boron, trialkyl (p-fluorophenyl) boron, trialkyl (3,5-bistrifluoro methyl) phenyl boron, trialkyl [3,5-bis (1,1,1,3,3,3-hex-afluoro-2-methoxy-2-propyl) phenyl] boron, trialkyl (p-nitrophenyl) boron, trialkyl (m-nitrophenyl) boron, trialkyl (p-butylphenyl) boron, trialkyl (m-butylphenyl) boron, trialkyl (p-butyloxyphenyl) boron, trialkyl (m-butyloxyphenyl) boron, trialkyl (p-octyloxyphenyl) boron and trialkyl (m-octyloxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having two aryl groups in one molecule include dialkyl diphenylboron, dialkyl di (p-chlorophenyl) boron, dialkyl di (p-fluorophenyl) boron, dialkyl di (3,5-bistrifluoro methyl) phenyl boron, dialkyl di [3,5-bis (1,1,1,3,3,3-hexafluoro-2-methoxy-2-propyl) phenyl] boron, dialkyl di (p-nitrophenyl) boron, dialkyl di (m-nitrophenyl) boron, dialkyl di (p-butylphenyl) boron, dialkyl di (m-butylphenyl) boron, dialkyl di (p-butyl oxyphenyl) boron, dialkyl di (m-butyl oxyphenyl) boron, dialkyl di (p-octyl oxyphenyl) boron and dialkyl di (m-octyl oxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having three aryl groups in one molecule include monoalkyl triphenylboron, monoalkyl tri (p-chlorophenyl) boron, monoalkyl tri (p-fluorophenyl) boron, monoalkyl tri (3,5-bistrifluoro methyl) phenyl boron, monoalkyl tri [3,5-bis (1,1,1,3,3,3-hexafluoro-2-methoxy-2-propyl) phenyl] boron, monoalkyl tri (p-nitrophenyl) boron, monoalkyl tri (m-nitrophenyl) boron, monoalkyl tri (p-butylphenyl) boron, monoalkyl tri (m-butylphenyl) boron, monoalkyl tri (p-butyl oxyphenyl) boron, monoalkyl tri (m-butyl oxyphenyl) boron, monoalkyl tri (p-octyl oxyphenyl) boron and monoalkyl tri (m-octyl oxyphenyl) boron (the alkyl group is at least one selected from the group consisting of n-butyl group, n-octyl group and n-dodecyl group etc.) and salts thereof (sodium salt, lithium salt, potassium salt, magnesium

salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having four aryl groups in one molecule include tetraphenylboron, tetra kis (p-chlorophenyl) boron, tetra kis (p-fluorophenyl) boron, tetra kis (3,5-bistrifluoro methyl) phenyl boron, tetra kis [3,5-bis (1,1,1,3,3,3-hexafluoro-2-methoxy-2-propyl) phenyl] boron, tetra kis (p-nitrophenyl) boron, tetra kis (m-nitrophenyl) boron, tetra kis (p-butylphenyl) boron, tetra kis (m-butylphenyl) boron, tetra kis (p-butyl oxyphenyl) boron, tetra kis (m-butyl oxyphenyl) boron, tetra kis (p-octyl oxyphenyl) boron, tetra kis (m-octyl oxyphenyl) boron, (p-fluorophenyl) triphenylboron, (3,5-bis trifluoromethyl) phenyltriphenylboron, (p-nitrophenyl) triphenylboron, (m-butyl oxyphenyl) triphenylboron, (p-butyl oxyphenyl) triphenylboron, (m-octyl oxyphenyl) triphenylboron and (p-octyl oxyphenyl) triphenylboron, and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like).

Among these aryl borate compounds, it is more preferable to use a borate compound having 3 or 4 aryl groups in one molecule from the viewpoint of storage stability. Further, these aryl borate compounds can be used alone or as a mixture of two or more.

Examples of sulfur-containing reductive inorganic compound include sulfites, bisulfites, pyrosulfites, thiosulfates, thionates and dithionite. Specific examples include sodium sulfite, potassium sulfite, calcium sulfite, ammonium sulfite, sodium bisulfite, potassium bisulfite, 3-mercaptopropyl trimethoxysilane, 2-mercaptobenzoxazole, decanethiol, thiobenzoic acid and the like.

Examples of nitrogen-containing reductive inorganic compound include nitrites, and specific examples include sodium nitrite, potassium nitrite, calcium nitrite, ammonium nitrite and the like.

Specific examples of barbituric acid derivative include salts (alkali metals or alkaline earth metals are preferred) of barbituric acid, 1,3-dimethyl barbituric acid, 1,3-diphenyl barbituric acid, 1,5-dimethyl barbituric acid, 5-butyl barbituric acid, 5-ethyl barbituric acid, 5-isopropyl barbituric acid, 5-cyclohexyl barbituric acid, 1,3,5-trimethyl barbituric acid, 1,3-dimethyl-5-ethyl barbituric acid, 1,3-dimethyl-n-butyl barbituric acid, 1,3-dimethyl-5-isobutyl barbituric acid, 1,3-dimethyl barbituric acid, 1,3-dimethyl-5-cyclopentyl barbituric acid, 1,3-dimethyl-5-cyclohexyl barbituric acid 1,3-dimethyl-5-phenyl barbituric acid, 1-cyclohexyl-1-ethyl barbituric acid, 1-benzyl-5-phenyl barbituric acid, 5-methyl barbituric acid, 5-propyl barbituric acid, 1,5-diethyl barbituric acid, 1-ethyl-5-methyl barbituric acid, 1-ethyl-5-isobutyl barbituric acid, 1,3-diethyl-5-butyl barbituric acid, 1-cyclohexyl-5-methyl barbituric acid, 1-cyclohexyl-5-ethyl barbituric acid, 1-cyclohexyl-5-octyl barbituric acid, 1-cyclohexyl-5-hexyl barbituric acid, 5-butyl-1-cyclohexyl barbituric acid, 1-benzyl-5-phenyl barbituric acid and thiobarbituric acids. Specifically, the salts of these barbituric acids include sodium 5-butyl barbiturate, sodium1,3,5-trimethyl barbiturate, sodium 1-cyclohexyl-5-ethyl barbiturate and the like.

Specific examples of the halogen compound include dilauryl dimethyl ammonium chloride, lauryl dimethyl benzyl ammonium chloride, benzyl trimethyl ammonium chloride, tetramethyl ammonium chloride, benzyl dimethyl acetyl ammonium chloride, dilauryl dimethyl ammonium bromide and the like.

The dental adhesive composition of the present invention may not contain a chemical polymerization initiator and a chemical polymerization accelerator. The dental adhesive composition of the present invention may not contain a polymerization initiator system other than the photopolymerization system.

<Other component>

Further, the dental adhesive composition of the present invention may contain a component other than above described components within a range not to impair the effect of the present invention. For example, an excipient typified by fumed silica, benzophenone-based and benzotriazole-based ultraviolet absorbers, polymerization inhibitors such as hydroquinone, hydroquinone monomethyl ether and 2,5-ditershally butyl-4-methylphenol, chain transfer materials such as $\alpha$-alkylstyrene compound, mercaptan compound such as n-butyl mercaptan and n-octyl mercaptan, and terpenoid compound such as limonene, myrsen, $\alpha$-terpinene, $\beta$-terpinene, $\gamma$-terpinene, terpinoren, $\beta$-pinene and $\alpha$-pinene, metal supplementary material such as aminocarboxylic acid chelating agent and phosphonic acid chelating agent, discoloration inhibitors, antibacterial materials, coloring pigments, and other additives conventionally known in the art may be added as necessary and as desired.

A preparing method of the dental adhesive composition of the present invention is not particularly limited. Examples of a general preparing method of a dental adhesive composition, for example, include a method which comprises simultaneously mixing (A) polymerizable monomer containing (A-1) polymerizable monomer having an acidic group, (D) photopolymerization accelerator containing (D-1) aliphatic tertiary amine compound represented by formula (1), (F) volatile organic solvent and (G) water, and a method which comprises mixing (A) polymerizable monomer containing (A-1) polymerizable monomer having an acidic group, (D) photopolymerization accelerator containing (D-1) aliphatic tertiary amine compound represented by formula (1) and (F) volatile and then adding (G) water and mixing. A mixer such as a mix rotor can be used for mixing. In the present invention, it can be prepare by the above-described

method without any problem.

The dental adhesive composition of the present invention can be used for a dental bonding material, a dental primer, a dental coating material and a dental glass ionomer cement, which are one pack type dental adhesive composition, two packs type dental adhesive composition or the like. Further, it can be used as a dental adhesive kit in which the dental adhesive composition of the present invention and a dental curable composition are combined. A dental curable compositions can be used for a dental adhesive material, a dental composite resin, a dental core build-up material, a dental resin cement, a dental coating material, a dental sealant material, a dental manicure material and a dental splinting material.

<One pack type dental adhesive composition>

When the present invention is used in one pack type dental adhesive composition, it is preferably used for a dental bonding material, a dental primer and a dental coating material. In the case of one pack type dental adhesive composition, since complicated steps such as mixing are not required before use, reduction of technical errors and reduction of burden on the operator can be expected. The one pack type dental adhesive composition may comprise (A) polymerizable monomer such as a polymerizable monomer having an alkoxysilyl group and a polymerizable monomer having a sulfur atom, (B) photosensitizer, (C) photoacid generator, (D) photopolymerization accelerator and the like in addition to (A-1) polymerizable monomer having an acidic group, (D-1) aliphatic tertiary amine compound represented by formula (1), (F) volatile organic solvent and (G) water. In particular, when (B) photosensitizer and (C) photoacid generator are contained, it is preferable that one pack type dental adhesive composition is cured by irradiating light after using the one pack type dental adhesive composition and therefore it can be expected to improve the adhesive property with respect to the composition to be applied next.

<Two packs type dental adhesive composition>

When the present invention is used in two packs type dental adhesive composition, it is preferably used for a dental bonding material, a dental primer, a dental coating material and a dental glass ionomer cement. The two packs type dental adhesive composition is used by mixing the two packs before use. The preferred mixing ratio is a mass ratio of 0.8 to 1.2: 1.0. Curing proceeds without light irradiation such as glass ionomer reaction or redox reaction. Since the two packs type dental adhesive composition cures without light irradiation, it is suitable for use in a deep cavity where light is difficult to reach, and further, it is expected that high adhesive strength is exhibited by performing light irradiation. The two packs type dental adhesive composition is packaged in a first pack and a second pack, and the first pack contains (A-1) polymerizable monomer having an acidic group, (D-1) aliphatic tertiary amine compound represented by formula (1), (F) volatile organic solvent and (G) water, and a chemical polymerization accelerator and an organic peroxide are separately contained in the first pack and the second pack, respectively. The first pack and the second pack may comprise (A) polymerizable monomer such as a polymerizable monomer having an alkoxysilyl group and a polymerizable monomer having a sulfur atom, (B) photosensitizer, (C) photoacid generator, (D) photopolymerization accelerator other than the (D-1) aliphatic tertiary amine compound represented by formula (1) and the like.

<Dental adhesive kit>

The dental adhesive composition of the present invention can be used as a dental adhesive kit in combination with a dental curable composition. As the dental adhesive composition, the above described one pack type dental adhesive composition or two packs type dental adhesive composition is used. Specific examples of a dental curable composition include a dental adhesive material, a dental composite resin, a dental core build-up material, a dental resin cement, a dental coating material, a dental sealant material, a dental manicure material, a dental splinting material and a glass ionomer cement. Among them, in particular, it is used as a two-step type dental bonding material combined with a dental adhesive material, or a dental adhesive kit in which it combined with a dental composite resin, a dental core build-up material, a dental resin cement or a glass ionomer cement. It is preferable that the dental curable composition contains (A) polymerizable monomer and a polymerization initiator as an essential component, and (E) filler. The polymerization initiator is classified into a photopolymerization initiator and a chemical polymerization initiator, and may contain either one or both of them. In particular, when the dental curable composition contains (B) photosensitizer and (C) photoacid generator as a photopolymerization initiator, high adhesive strength and improvement in mechanical properties can be expected in the case of combining with the dental adhesive composition containing (D-1) aliphatic tertiary amine compound represented by formula (1) of the present invention. For preferable compounding amount in such a dental curable composition, (B) photosensitizer is 0.001 to 2 parts by mass and (C) photoacid generator is 0.01 to 10 parts by mass with respect to 100 parts by mass of the (A) polymerizable monomer contained in the dental curable composition, and it is preferable that the composition is one pack type dental curable composition or two packs type

dental curable composition.

The dental adhesive composition of the present invention may comprise only (A) polymerizable monomer, (D) photopolymerization accelerator, and (F) volatile organic solvent. Further, as the components other than (A), (D) and (F), only one or more of the above-mentioned components may be contained.

[Examples]

Hereinafter, example of the present invention are specifically described. However, the present invention is not intended to be limited to these Examples.

The materials used in Examples and Comparative examples and their abbreviations are listed below.

[(A) Polymerizable monomer]

Bis-GMA: 2,2-bis [4-(3-methacryloyloxy-2-hydroxypropoxy) phenyl] propane
2.6E: 2,2-bis (4-(meth) acryloyloxy polyethoxyphenyl) propane in which the average addition mole number of ethoxy groups is 2.6
UDMA: N,N-(2,2,4-trimethyl hexamethylene) bis [2-(aminocarboxy) ethanol] methacrylate
TEGDMA: triethyleneglycol dimethacrylate
HEMA: 2-hydroxyethyl methacrylate

[(A-1) polymerizable monomer having an acidic group]

MDP: 10-methacryloyloxydecyl dihydrogen phosphate
MHPA: 6-methacryloxyhexyl phosphonoacetate
MET: 4-methacryloxyethyl trimellitic acid
META: 4-methacryloyloxy ethoxycarbonylphthalic anhydride

[Polymerizable monomer having alkoxysilyl group]

MPTMS: 3-methacryloxypropyl trimethoxysilane
[Polymerizable monomer having sulfur atom]
MDDT: 10-methacryloxydecyl-6,8-ditioctanate

[(B) Photosensitizer]

CQ: $\alpha$-camphorquinone
BAPO: phenyl bis (2,4,6-trimethylbenzoyl) phosphine oxide

[(C) Photoacid generator]

C1: bis (4-tert-butylphenyl) iodonium nonafluorobutane sulfonate

[Chemical formula 3]

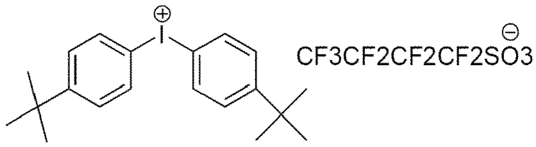

C2: bis (4-tert-butylphenyl) iodium camphor sulfonate

[Chemical formula 4]

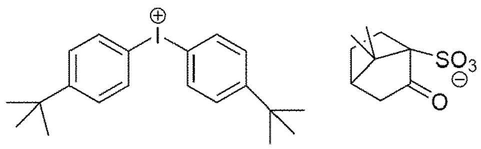

C3: bis (4-tert-butylphenyl) iodonium tris (pentafluoropropyl) trifluorophosphate

[Chemical formula 5]

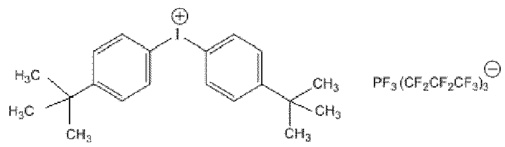

C4: bis (4-n-dodecylphenyl) iodonium tetrakis (pentafluorophenyl) borate

[Chemical formula 6]

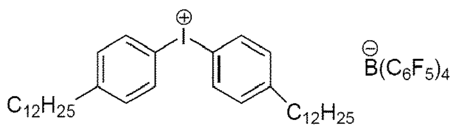

C5: bis [(4-tert-butyl) phenyl] iodonium tetra (nonafluoro-tert-butoxy) aluminate

[Chemical formula 7]

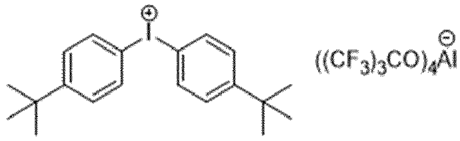

C6: bis [4-(tert-butyl) phenyl] iodonium tetra (pentafluorophenyl) gallate

[Chemical formula 8]

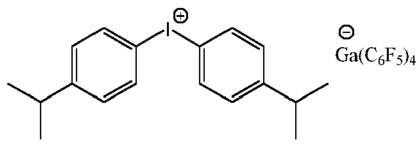

C7: diphenyliodonium trifluoromethane sulfonic acid

[Chemical formula 9]

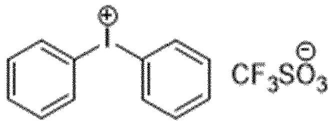

C8: bis (4-tert-butylphenyl) iodonium-p-toluenesulfonate

[Chemical formula 10]

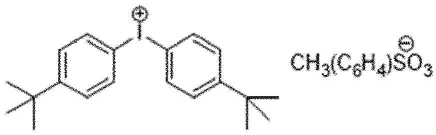

C9: bis (4-tert-butylphenyl) iodonium hexafluorophosphate

[Chemical formula 11]

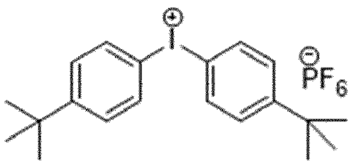

C10: bis (4-n- dodecylphenyl) iodonium hexafluorophosphate

[Chemical formula 12]

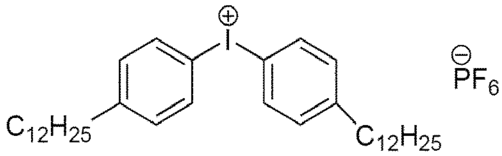

C11: 2,4,6-tris (trichloromethyl)-1,3,5-triazine

[Chemical formula 13]

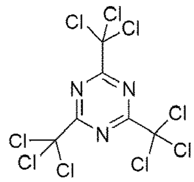

C12: diphenyliodonium-2-carboxylate monohydrate

[Chemical formula 14]

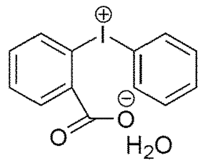

[(D) Photopolymerization accelerator]

[(D-1) aliphatic tertiary amine compound represented by formula (1)]

<Aliphatic tertiary amine having one substituent consisting of three or more carbons having an electron-attracting group at $\alpha$-carbon and/or $\beta$-carbon of an amine starting from N>

D1-1: N,N-diisopropylaminoethyl methacrylate

[Chemical formula 15]

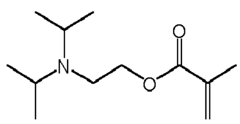

D1-2: 3-isopropyl-2-methyl-7-oxo-6,11-dioxo-3,8-diazatridecane-13-yl methacrylate

[Chemical formula 16]

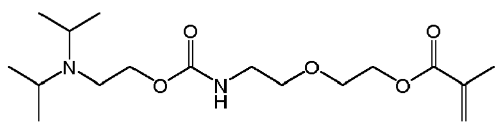

D1-3: 2-(((2-(dibutylamino) ethoxy) carbonyl) amino) ethyl methacrylate

[Chemical formula 17]

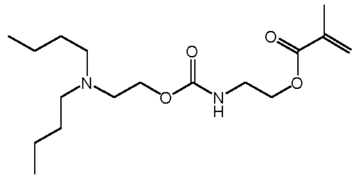

D1-4: 1-[(3,3-diphenylpropyl) (methyl) amino]-2-methyl-2-propanol

[Chemical formula 18]

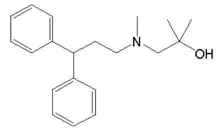

D1-5: (1R,2S)-1-phenyl-2-(1-pyrrolidinyl) propane-1-ol

[Chemical formula 19]

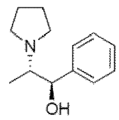

D1-6: (1S,2R)-2-(dibutylamino)-1-phenyl-1-propanol

[Chemical formula 20]

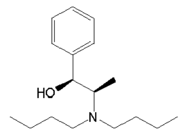

<Aliphatic tertiary amine having two or more substituents consisting of three or more carbons having an electron-attracting group at α-carbon and/or β-carbon of an amine starting from N>

D2-1: 1-benzhydryl azetidine-3-carboxylic acid

[Chemical formula 21]

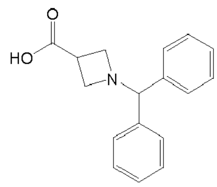

D2-2: triisopanol amine

[Chemical formula 22]

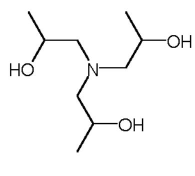

D2-3: N,N,N',N'-tetrakis (2-hydroxypropyl) ethylenediamine

[Chemical formula 23]

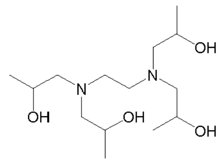

D2-4: diethylbenzylimino diacetate

[Chemical formula 24]

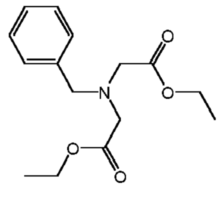

D2-5: 11-benzyl-7,15-dioxo-3,8,14,19-tetraoxo-6,11,16-triazahenicosan-1,21-diylbis (2-methacrylate)

[Chemical formula 25]

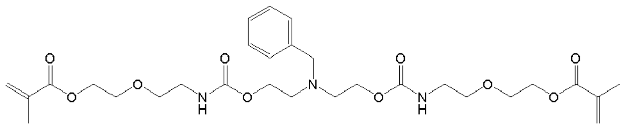

<Aliphatic tertiary amine having two or more aryls which may have a substituent as a substituent of N>

D3-1: 2-benzyl-6-oxo-1-phenyl-5,10-dioxo-2,7-diazadodecane-12-yl methacrylate

[Chemical formula 26]

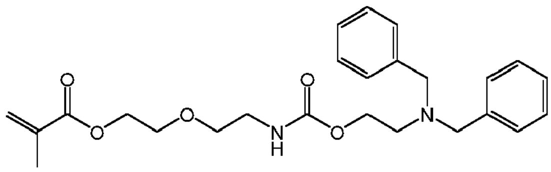

D3-2: N,N-dibenzylglycine ethyl ester

[Chemical formula 27]

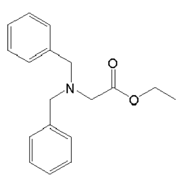

D3-3: dibenzylaminoethyl methacrylate

[Chemical formula 28]

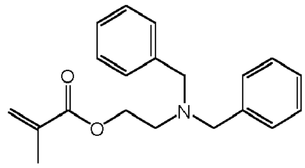

D3-4: (S)-(+)-2-(dibenzylamino)-3-phenyl-1-propanol

[Chemical formula 29]

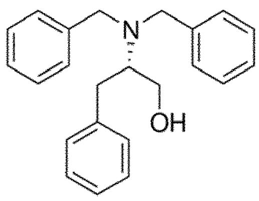

D3-5: dibenzylaminoethanol

[Chemical formula 30]

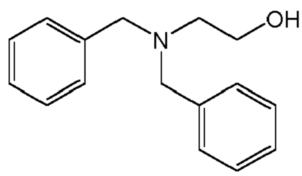

D3-6: tribenzylamine

[Chemical formula 31]

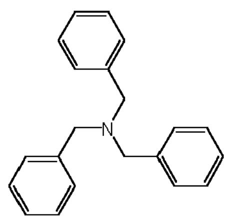

D3-7: 2-(((2-(dibenzylamino) ethoxy) carbonyl) amino)-2-methylpropane-1,3-diyl dimethacrylate

[Chemical formula 32]

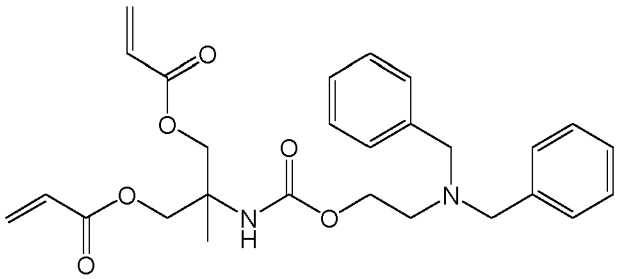

D3-8: 3-(N, N-dibenzylamino) propyltriethoxysilane

[Chemical formula 33]

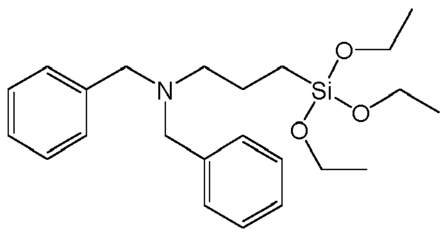

<Other aliphatic tertiary amine>

«Aliphatic tertiary amine compound having no primary hydroxyl group» DMAEMA: N,N-dimethylamino ethylmethacrylate
DEAEMA: N,N-diethylamino ethylmethacrylate

«Aliphatic tertiary amine compound having two primary hydroxyl groups»
MDEOA: methyl diethanolamine

«Aliphatic tertiary amine compound having three primary hydroxyl groups»
TEA: triethanolamine

<Aromatic tertiary amine compound>
DMBE: N,N-dimethylaminobenzoate ethyl

<Organic metal compound>
SnL: dioctyl-tin-dilaurate

[(F) Volatile organic solvent]

Ac: acetone
EtOH: ethanol

[(G) water]

DW: ion-exchanged water

[(E) Filler]

The preparing method of each filler used for preparing the dental adhesive composition and/or the dental photocurable composition is shown below.

(Filler E1)

A silane coupling treatment solution prepared by stirring 50.0 g of water, 35.0 g of ethanol, and 3.0 g of 3-methacryloyloxypropyl trimethoxysilane as a silane coupling material at room temperature for 2 hours was added to 100.0 g of fluoroaluminosilicate glass (average particle diameter: 0.9 μm) and stirred for 30 minutes. Thereafter, a heat treatment was performed at 100 °C for 15 hours to obtain a filler E1.

(Filler E2)

A silane coupling treatment solution prepared by stirring 50.0 g of water, 35.0 g of ethanol, and 5.0 g of 3-methacryloyloxypropyl trimethoxysilane as a silane coupling material at room temperature for 2 hours was added to 100.0 g of the zirconium silicate filler (average particle diameter: 0.8 μm, zirconia: 85 wt.%, silica:15 wt.%) and stirred for 30 minutes. Thereafter, a heat treatment was performed at 100 °C for 15 hours to obtain a filler E2.

(Filler E3)

A silane coupling treatment solution prepared by stirring 5 g of water, 100 g of ethanol, and 10.0 g of di3-(N,N-dibenzylamino) propyltriethoxysilane as a silane coupling material at room temperature for 2 hours was added to 100.0 g of Aerosil OX-50 (manufactured by Evonik Industries AG) and stirred for 24 minutes. Thereafter, a heat treatment was performed at 100 °C for 15 hours to obtain a filler E3.

(Filler E4)

Aerosil R7200 (manufactured by Evonik Industries AG)

(Filler E5)

Aerosil OX-50 (manufactured by Evonik Industries AG)

[Chemical polymerization initiator]

CHP: cumene hydroperoxide
tBHP: tert-butyl hydroperoxide
TPE: 1,1,3,3-tetramethylbutyl peroxy-2-ethylhexanoate

[Chemical polymerization accelerator]

PTU: (2-pyridyl) thiourea
DMPT: N,N-dimethyl-p-toluidine
DEPT: N,N-dihydroxyethyl-p-toluidine
GLA: copper gluconate
VOA: vanadyl acetylacetonate
TMBA: trimethyl barbituric acid

[UV absorber]

BT: 2-(2-hydroxy-5-methylphenyl) benzotriazole

[Polymerization inhibitor]

BHT: 2,6-di-t-butyl-4-methylphenol
MeHQ: p-methoxyphenol

[Fluorescent agent]

FA: 2.5-dihydroxyterephthalate diethyl

<Preparing method of one pack type dental adhesive composition>

Components shown in Table 1 to 2 were put into a wide mouthed plastic container and mixed by using a mix rotor VMRC-5 under light-shielding condition at 100 rpm for 48 hours to prepare a dental adhesive composition. The prepared dental adhesive composition was filled into a light-shielding bottle to prepare a dental adhesive composition of each composition P and each composition CP. In the Tables 1 to 2, the content (parts by mass) of each component is indicated by the numerical value in parentheses after the abbreviation of each component.

[Table 1]

| | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|
| | | Polymerizable monomer other than (A-1) | A-1) polymerizable monomer having an acidic group | (B) Photosensitizer | (C) Photoacid generator | (D) Photopolymerization accelerator | | | | | |
| Example PI | Composition P1 | - | MDP(5) | - | - | D3-1(1) | - | EtOH(95) | - | - | - |
| Example P2 | Composition P2 | - | MDP(0.1) | - | - | D3-2(0.1) | - | Ac(99.9) | - | - | - |
| Example P3 | Composition P3 | - | MDP(20) | - | - | D3-3(0.1) | - | Ac(80) | - | - | BHT (0.1) |
| Example P4 | Composition P4 | - | MDP(10), META(IO) | - | - | D3-4(20) | - | EtOH(80) | - | - | BHT (0.1) |
| Example P5 | Composition P5 | - | MDP(0.1) | - | - | D3-4(20) | - | Ac(99.9) | - | - | - |
| Example P6 | Composition P6 | Bis-GMA(35), TEGDMA(15) | MDP(0.1) | - | - | D3-1(0.1) | - | Ac(49.9) | - | - | BHT (0.1) |
| Example P7 | Composition P7 | Bis-GMA(35), TEGDMA(15) | MDP(2) | CQ(0.2) | C1(0.5) | D3-1(1) | - | Ac(48) | - | - | BHT (0.1) |
| Example P8 | Composition P8 | Bis-GMA(35), TEGDMA(15) | MDP(2) | CQ(0.5) | - | D3-1(1) | - | Ac(48) | - | DMPT(1.0) | BHT (0.1) |
| Example P9 | Composition P9 | Bis-GMA(35), TEGDMA(15) | MDP(2) | CQ(0.5) | C1(0.5) | D3-1(1) | - | Ac(48) | - | GLA(0.2) | BHT (0.1) |
| Example P10 | Composition P10 | Bis-GMA(35), TEGDMA(15) | MDP(2) | CQ(0.5) | C2(0.5) | D3-1(1) | - | Ac(48) | - | VOA(0.2) | MeHQ (0.1) |
| Example P11 | Composition P11 | Bis-GMA(35), TEGDMA(15) | MDP(2) | CQ(0.5) | C2(0.5) | D3-1(1),DMBE(0.3) | - | Ac(48) | - | VOA(0.2) | MeHQ (0.1) |
| Example P12 | Composition P12 | Bis-GMA(35), TEGDMA(12), MPTMS(2), MDDT(1) | MDP(2) | CQ(0.5) | C5(0.5) | D3-1(1),DMBE(0.3) | - | EtOH(28) | DW(20) | - | BHT (0.1) |

(continued)

| | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|
| | | Polymerizable monomer other than (A-1) | A-1) polymerizable monomer having an acidic group | (B) Photosensitizer | (C) Photoacid generator | (D) Photopolymerization accelerator | | | | | |
| Example P13 | Composition P13 | Bis-GMA(35), TEGDMA(15) | MDP(2) | CQ(0.5) | C3(0.5) | D3-1(1) | - | EtOH(28) | DW(20) | - | BHT (0.1) |
| Example P14 | Composition P14 | Bis-GMA(35), TEGDMA(13), MPTMS(2) | MDP(2) | CQ(0.5) | C4(0.5) | D3-1(1) | - | EtOH(28) | DW(20) | - | BHT (0.1) |
| Example P15 | Composition P15 | Bis-GMA(35), TEGDMA(12), MPTMS(2), MDDT(1) | MDP(2) | CQ(0.5) | C5(0.5) | D3-1(1) | - | EtOH(28) | DW(20) | - | BHT (0.1) |
| Example P16 | Composition P16 | MPTMS(2), MDDT(1) | MDP(3),META (9) | CQ(0.5) | C5(0.5) | D3-1(1) | - | EtOH(60) | DW(25) | VOA(0.05) | MeHQ (0.1) |
| Example P17 | Composition P17 | MPTMS(2), MDDT(1) | MDP(3),MET(9) | CQ(0.5) | C6(0.5) | D3-1(1) | - | EtOH(40) | DW(45) | GLA(0.2) | BHT (0.1) |
| Example P18 | Composition P18 | - | MDP(4) | - | - | D1-1(1) | - | EtOH(96) | - | - | - |
| Example P19 | Composition P19 | - | MDP(4) | - | - | D1-2(0.1) | - | Ac(96) | - | - | - |
| Example P20 | Composition P20 | - | MDP(4) | - | - | D1-3(20) | - | EtOH(96) | - | - | - |
| Example P21 | Composition P21 | MPTMS(2), MDDT(1) | MDP(4) | CQ(0.5) | C6(0.5) | D1-4(2) | - | EtOH(40) | DW(53) | - | BHT (0.1) |
| Example P22 | Composition P22 | Bis-GMA(35), TEGDMA(12), MPTMS(2), MDDT(1) | MDP(4) | CQ(0.5) | C7(0.5) | D1-5(2) | - | Ac(26) | DW(20) | - | BHT (0.1) |
| Example P23 | Composition P23 | - | MDP(4) | - | - | D2-1(1) | - | EtOH(96) | - | - | - |

(continued)

| | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|
| | | Polymerizable monomer other than (A-1) | A-1) polymerizable monomer having an acidic group | (B) Photosensitizer | (C) Photoacid generator | (D) Photopolymerization accelerator | | | | | |
| Example P24 | Composition P24 | - | MDP(4) | - | - | D2-2(0.1) | - | Ac(96) | - | - | - |
| Example P25 | Composition P25 | - | MDP(4) | - | - | D2-3(20) | - | EtOH(96) | - | - | - |
| Example P26 | Composition P26 | MPTMS(2), MDDT(1) | MDP(1) | CQ(0.5) | C5(0.5) | D2-4(2) | - | EtOH(40) | DW(56) | - | - |
| Example P27 | Composition P27 | Bis-GMA(35), TEGDMA(12), MPTMS(2), MDDT(1) | MDP(10) | CQ(0.5) | C4(0.5) | D2-5(2) | - | Ac(20) | DW(20) | - | BHT (0.1) |
| Example P28 | Composition P28 | - | MDP(4) | - | - | D3-1(1) | - | EtOH(96) | - | - | - |
| Example P29 | Composition P29 | - | MDP(4) | - | - | D3-2(0.1) | - | Ac(96) | - | - | - |
| Example P30 | Composition P30 | - | MDP(4) | - | - | D3-3(20) | - | EtOH(96) | - | - | - |

[Table 2]

| | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|
| | | Polymerizable monomer other than (A-1) | (A-1) polymerizable monomer having an acidic group | (B) Photosensitizer | (C) Photoacid generator | (D) Photopolymerization accelerator | | | | | |
| Example P31 | Composition P31 | MPTMS(2), MDDT(1) | MDP(4) | CQ(0.5) | C1(0.5) | D3-4(2) | - | EtOH (40) | DW (53) | - | BHT (0.1) |
| Example P32 | Composition P32 | Bis-GMA(35), TEGDMA(12), MPTMS(2), MDDT(1) | MDP(4) | CQ(0.5) | C2(0.5) | D3-5(2) | E5 (10) | Ac(26) | DW (20) | - | BHT (0.1) |
| Example P33 | Composition P33 | Bis-GMA(35), TEGDMA(12), MPTMS(2), MDDT(1) | MDP(2),META (9) | CQ(0.3) | C2(1) | D3-6(2) | - | EtOH (14) | DW (25) | - | BHT (0.1) |
| Example P34 | Composition P34 | Bis GMA(25), TEGDMA(8), MPTMS(2), MDDT(1) | MDP(2),MET (8) | CQ(0.3) | C6(3) | D3-6(2) | E4 (50) | EtOH (29) | DW (25) | - | BHT (0.1) |
| Example P35 | Composition P35 | Bis GMA(25), TEGDMA(8), MPTMS(2), MDDT(1) | MHPA(2),MET (8) | CQ(0.3) | C6(1) | D3-6(2) | - | EtOH (29) | DW (25) | - | BHT (0.1) |
| Example P36 | Composition P36 | Bis GMA(25), TEGDMA(8), MPTMS(2), MDDT(1) | MHPA(2), META(8) | CQ(0.3) | C6(5) | D3-6(2) | E4 (10) | EtOH (29) | DW (25) | - | BHT (0.1) |
| Example P37 | Composition P37 | Bis GMA(25), TEGDMA(8), MPTMS(2), MDDT(1) | MDP(2),MET (8) | CQ(0.3) | C6(1) | D3-6(2) | - | Ac(29) | DW (25) | VOA(0.01) | MeHQ (0.1) |

(continued)

| | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|
| | | Polymerizable monomer other than (A-1) | (A-1) polymerizable monomer having an acidic group | (B) Photosensitizer | (C) Photoacid generator | (D) Photopolymerization accelerator | | | | | |
| Example P38 | Composition P38 | Bis GMA(25), TEGDMA(8), MPTMS(2), MDDT(1) | MDP(2),MET (8) | CQ(0.3) | C6(1) | D3-6(2) | - | Ac(29) | DW (25) | GLA(0.1) | BHT (0.1) |
| Example P39 | Composition P39 | UDMA(35), TEGDMA(8), MPTMS(2), MDDT(1) | MHPA(2), META(8) | CQ(0.3) | C6(1) | D3-6(2 | - | EtOH (20) | DW (24) | - | BHT (0.1) |
| Example P40 | Composition P40 | UDMA(35), GDMA(12), MPTMS(2), MDDT(1) | MHPA(2), META(8) | CQ(0.3) | C6(1) | D3-6(2) | - | EtOH (20) | DW (20) | - | BHT (0.1) |
| Example P41 | Composition P41 | - | MDP(4) | - | - | D1-6(1) | - | Ac(96) | - | - | - |
| Example P42 | Composition P42 | Bis-GMA(35), TEGDMA(12), MPTMS(2), MDDT(1) | MDP(2) | CQ(0.5) | C8(0.5) | D3-6(2) | - | EtOH (28) | DW (20) | - | BHT (0.1) |
| Example P43 | Composition P43 | Bis-GMA(35), TEGDMA(12), MPTMS(2), MDDT(1) | MDP(2) | CQ(0.5) | C9(0.5) | D3-6(2 | - | EtOH (28) | DW (20) | - | BHT (0.1) |
| Example P44 | Composition P44 | Bis-GMA(35), TEODMA(12), MPTMS(2), MDDT(1) | MDP(2) | CQ(0.5) | C10(0.5) | D3-6(2 | - | EtOH (28) | DW (20) | - | BHT (0.1) |

(continued)

| | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|
| | | Polymerizable monomer other than (A-1) | (A-1) polymerizable monomer having an acidic group | (B) Photosensitizer | (C) Photoacid generator | (D) Photopolymerization accelerator | | | | | |
| Example P45 | Composition P45 | Bis-GMA(35), TEGDMA(12), MPTMS(2), MDDT(1) | MDP(2) | CQ(0.5) | C11(0.5) | D3-6(2) | - | EtOH (28) | DW (20) | - | BHT (0.1) |
| Example P46 | Composition P46 | Bis-GMA(35), TEGDMA(12), MPTMS(2), MDDT(1) | MDP(2) | CQ(0.5) | C12(0.5) | D3-6(2) | - | EtOH (28) | DW (20) | - | BHT (0.1) |
| Example P47 | Composition P47 | Bis-GMA(35), TEGDMA(12), MPTMS(2), MDDT(1) | MDP(2) | BAPO(0.5) | C5(0.5) | D3-1(1) | - | EtOH (28) | DW (20) | - | BHT (0.1) |
| Comparative Examnle CP1 | Composition CP1 | UDMA(35), GDMA(12), MPTMS(2), MDDT(1) | MHPA(2), META(8) | CQ(0.3) | C6(1) | - | E3 (10) | EtOH (20) | DW (20) | - | BHT (0.1) |
| Comparative Examnle CP2 | Composition CP2 | - | MDP(5) | - | - | - | - | Ac(95) | - | - | - |
| Comparative Example CP3 | Composition CP3 | - | - | - | - | D3-2(2) | - | Ac(100) | - | - | - |
| Comparative Example CP4 | Composition CP4 | - | MDP(5) | - | - | DMBE(0.5) | - | Ac(95) | - | - | - |
| Comparative Examnle CP5 | Composition CP5 | - | MDP(5) | - | - | DMAEMA(2) | - | Ac(95) | - | - | - |
| Comparative Examnle CP6 | Composition CP6 | - | MDP(5) | - | - | TEA(1) | - | Ac(95) | - | - | - |

(continued)

| | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|
| | | Polymerizable monomer other than (A-1) | (A-1) polymerizable monomer having an acidic group | (B) Photosensitizer | (C) Photoacid generator | (D) Photopolymerization accelerator | | | | | |
| Comparative Examnle CP7 | Composition CP7 | - | MDP(5) | - | - | MDEOA(0.5) | - | Ac(95) | - | - | - |
| Comparative Example CP8 | Composition CP8 | Bis-GMA(35), TEGDMA(15) | MDP(2) | CQ(0.5) | - | DMBE(0.5) | - | Ac(48) | - | - | - |
| Comparative Examnle CP9 | Composition CP9 | Bis-GMA(35), TEGDMA(15) | MDP(2) | CQ(0.5) | - | DMAEMA(2) | - | Ac(48) | - | - | - |
| Comparative Examnle CP10 | Composition CP10 | Bis-GMA(35), TEGDMA(15) | MDP(2) | CQ(0.5) | C1(0.5) | DMAEMA(2) | - | EtOH (48) | - | - | BHT (0.1) |
| Comparative Examnle CP11 | Composition CP11 | Bis-GMA(35), TEGDMA(15) | MDP(2) | CQ(0.5) | C2(1) | DMAEMA(2) | - | EtOH (48) | - | - | BHT (0.1) |
| Comparative Example CP12 | Composition CP12 | Bis-GMA(35), TEGDMA(15) | MDP(2) | CQ(0.5) | C3(2) | TEA(1) | - | EtOH (48) | - | - | BHT (0.1) |
| Comparative Example CP13 | Composition CP13 | Bis-GMA(35), TEGDMA(15) | MDP(2) | CQ(0.5) | C4(1) | MDEOA(0.5) | - | EtOH (48) | - | - | BHT (0.1) |

<Preparing method of two packs type dental adhesive composition>

Components of first pack shown in Tables 3 to 5 were put into a wide mouthed plastic container and mixed by using a mix rotor VMRC-5 under light-shielding condition at 100 rpm for 48 hours. Similarly, components of second pack were put into a wide mouthed plastic container and mixed by using a mix rotor VMRC-5 under light-shielding condition at 100 rpm for 48 hours. The first pack and the second pack were filled in different light-shielding bottles to prepare a dental adhesive composition of each composition Q and each composition CQ. Each composition Q and each composition CQ were used after mixing in a weight ratio of 0.8 to 1.2: 1.0 before use. In the Tables 3 to 5, the content (parts by mass) of each component is indicated by the numerical value in parentheses after the abbreviation of each component.

[Table 3]

| | | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | (A-1) polymerizable monomer having an acidic group | (B) Photosensitizer | (C) Photoacid generator | (D) Photopolymerization accelerator | | | | | |
| Example Q1 | Composition Q1 | First oack | - | MDP(5) | - | - | D3-6(1) | | EtOH (45) | | | |
| | | Second pack | MPTMS(2), MDDT(1) | | - | - | - | | EtOH (20) | DW(27) | | |
| Example Q2 | Composition Q2 | First oack | - | MDP(0.1) | - | - | D3-2(0.1) | | Ac(49.9) | | | |
| | | Second pack | MPTMS(2), MDDT(1) | | - | - | - | | EtOH (20) | DW(27) | | |
| Example Q3 | Composition Q3 | First oack | - | MDP(20) | - | - | D3-3(0.1) | | Ac(30) | | | |
| | | Second pack | MPTMS(2), MDDT(1) | | - | - | - | | EtOH (20) | DW(27) | | |
| Example Q4 | Composition Q4 | First oack | - | MDP(10), META(10) | - | - | D3-4(20) | | EtOH (30) | | | |
| | | Second pack | MPTMS(2), MDDT(1) | | - | - | - | | EtOH (20) | DW(27) | | |
| Example Q5 | Composition Q5 | First oack | - | MDP(0.1) | - | - | D3-6(20) | | Ac(49.9) | | | |
| | | Second pack | MPTMS(2), MDDT(1) | | - | - | - | | EtOH (20) | DW(27) | | |
| Example Q6 | Composition Q6 | First oack | Bis-GMA(15), TEGDMA(15) | MDP(0.1) | - | - | D3-1(0.1) | | Ac(19.9) | | | BHT (0.1) |
| | | Second pack | MPTMS(2), MDDT(1) | | - | - | - | | EtOH (20) | DW(27) | | |
| Example Q7 | Composition Q7 | First oack | Bis-GMA(15), TEGDMA(15) | MDP(2) | CQ(0.2) | - | D3-1(1) | | Ac(18) | | | BHT (0.1) |
| | | Second pack | MPTMS(2), MDDT(1) | | - | C1(0.5) | - | | EtOH (20) | DW(27) | | BHT (0.1) |

(continued)

| | | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | (A-1) polymerizable monomer having an acidic group | (B) Photosensitizer | (C) Photoacid generator | (D) Photopolymerization accelerator | | | | | |
| Example Q8 | Composition Q8 | First oack | Bis-GMA(15), TEGDMA(15) | MDP(2) | CQ(0.5) | - | D3-1(1) | | Ac(18) | | DMPT(1.0) | BHT (0.1) |
| | | Second pack | MPTMS(2), MDDT(1) | | - | C1(0.5) | - | | EtOH (20) | DW(27) | $_t$BHP(1.0) | BHT (0.1) |
| Example Q9 | Composition Q9 | First pack | Bis-GMA(15), TEGDMA(15) | MDP(2) | CQ(0.5) | C1(0.5) | D3-1(1) | | Ac(18) | | GLA(0.2) | BHT (0.1) |
| | | Second pack | MPTMS(2) MDDT(1) | | - | - | - | | EtOH (20) | DW(27) | $_t$BHP(1.0) | BHT (0.1) |
| Example Q10 | Composition Q10 | First oack | Bis-GMA(15), TEGDMA(15) | MDP(2) | CQ(0.5) | C2(0.5) | D3-1(1) | | Ac(18) | | VOA(0.2) | MeHQ (0.1) |
| | | Second pack | MPTMS(2), MDDT(1) | | - | - | - | | EtOH (20) | DW(27) | $_t$BHP(1.0) | BHT (0.1) |
| Example Q11 | Composition Q11 | First oack | Bis-GMA(15), TEGDMA(15) | MDP(2) | - | - | D3-1(1), DMRE(0.3) | | Ac(18) | | VOPA(0.2 | MeHQ (0.1) |
| | | Second pack | - | | cq(O 5) | C2(0.5) | - | | EtOH (30) | DW(20) | $_t$BHP(1.0) | |
| Example Q12 | Composition Q12 | First oack | Bis-GMA(16), TEGDMA(12), MPTMS(2) MDDT(1) | MDP(1) | CQ(0.5) | C5(05) | D3-1(1), . DMBE(0.3) | | EtOH (18) | | VOA(0.2) | MeHQ (0.1) |
| | | Second pack | | | - | - | - | | Ac(50) | | tBHP(1.0) | |
| Example Q13 | Composition Q13 | First oack | Bis-GMA(15), TEGDMA(15) | MDP(2) | CQ(0.5) | C3(0.5) | D3-1(1) | | EtOH(8) | DW(10) | TPE(1.0) | BHT(0. 1) |
| | | Second pack | | | - | - | - | | Ac(50) | | VOA(0.2) | |

(continued)

| | | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | (A-1) polymerizable monomer having an acidic group | (B) Photosensitizer | (C) Photoacid generator | (D) Photopolymerization accelerator | | | | | |
| Example Q14 | Composition Q14 | First oack | Bis-GMA(15), TEGDMA(13), MPTMS(2) | MDP(2) | CQ(0.5) | C4(0.5) | D3-1(5) | | EtOH(8) | DW(10) | VOA(0.01) | MeHQ (0.1) |
| | | Second pack | | | - | - | - | | Ac(50) | | TPE(1.0) | |
| Example Q15 | Composition Q15 | First pack | Bis-GMA(15), TEGDMA(12), MPTMS(2), MDDT(1) | MDP(2) | CQ(0.5) | C5(0.5) | D3-1(10) | | EtOH(8) | DW(10) | TPE(1.0) | BHT (0.1) |
| | | Second pack | | | - | - | - | | Ac(50) | | VOA(0.05), TMBA(1) | |
| Example Q16 | Composition Q16 | First oack | Bis-GMA(15), TEGDMA(12), MPTMS(2), MDDT(1) | MDP(3),META (9) | CQ(0.5) | - | D3-1(20) | | EtOH (10) | DW(25) | VOA(0.05) | MeHQ (0.1) |
| | | Second pack | | | - | C5(5) | - | | Ac(50) | | $_{t}$BHP(2) | |

[Table 4]

| | | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | (A-1) polymerizable monomer having an acidic group | (B) Photosensitizer | (C) Photoacid generator | (D) Photopolymerization accelerator | | | | | |
| Example Q17 | Composition Q17 | First pack | MPTMS(2), MDDT(1) | MDP(3),MET (9) | CQ(0.5) | C6(0.5) | D3-1(1), | | EtOH (20) | DW(15) | GLA(0.2) | BHT (0.1) |
| | | Second pack | - | | - | - | - | | Ac(50) | | tBHP(2) | |
| Example Q18 | Composition Q18 | First oack | - | MDP(4) | - | - | D1-1(1), | | EtOH (46) | | | BHT (0.1) |
| | | Second pack | - | | - | - | - | E1(20) | Ac(25) | DW(25) | | |
| Example Q19 | Composition Q19 | First oack | - | MDP(4) | - | - | D1-2(0.1) | | Ac(46) | | | BHT (0.1) |
| | | Second pack | MPTMS(2), MDDT(1) | | CQ(0.5) | C7(0.5) | - | | EtOH (20) | DW(27) | | |
| Example Q20 | Composition Q20 | First pack | | MDP(4) | - | - | D1-3(20) | | EtOH (46) | | | BHT (0.1) |
| | | Second oack | MPTMS(2), MDDT(1) | | CQ(0.5) | C7(0.5) | - | | EtOH (20) | DW(27) | | BHT (0.1) |
| Example Q21 Example Q21 | Q21 Composition Q21 | First oack | MPTMS(2), MDDT(1) | MDP(4) | CQ(0.5) | C6(0.5) | D1-4(2) | | EtOH (20) | DW(23) | | BHT (0.1) |
| | | Second oack | - | | - | - | - | | Ac(50) | | | |
| Example Q22 | Composition Q22 | First pack | Bis-GMA(15), TEGDMA(12), MPTMS(2), MDDT(1) | MDP(4) | - | - | D1-5(2) | | EtOH (10) | DW(6) | | MeHQ (0.1) |
| | | Second pack | - | | CQ(0.5) | C7(0.5) | - | | EtOH (50) | | | |

(continued)

| | | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | (A-1) polymerizable monomer having an acidic group | (B) Photosensitizer | (C) Photoacid generator | (D) Photopolymerization accelerator | | | | | |
| Example Q23 | Composition Q23 | First oack | Bis-GMA(15), TEGDMA(12), MPTMS(2), MDDT(1) | MDP(4) | - | - | D2-1(1) | | EtOH (46) | | | MeHQ (0.1) |
| | | Second pack | | | - | - | - | E1(20) | EtOH (20) | DW(30) | | |
| Example Q24 | Composition Q24 | First pack | - | MDP(4) | - | - | D2-2(0.1) | | Ac(46) | | | MeHQ (0.1) |
| | | Second pack | - | | CQ(0.2) | C1(0.5) | - | | EtOH (50) | | | |
| Example Q25 | Composition Q25 | First oack | - | MDP(4) | - | - | D2-9(20) | | EtOH (46) | | | BHT (0.1) |
| | | Second pack | - | | CQ(0.2) | C1(0.5) | - | | EtOH (50) | | | |
| Example Q26 | Composition Q26 | First oack | MPTMS(2), MDDT(1) | MDP(4) | CQ(0.5) | C5(0.5) | D2-4(2) | | EtOH (20) | DW(23) | | BHT (0.1) |
| | | Second oack | | | - | - | - | E1(20) | Ac(50) | | | |
| Example Q27 | Composition Q27 | First pack | Bis-GMA(15), TEGDMA(12), MPTMS(2), MDDT(1) | MDP(4) | CQ(0.5) | C4(0.5) | D2-5(2) | | EtOH(8) | DW(8) | | MeHQ (0.1) |
| | | Second oack | | | - | - | - | | Ac(50) | | | |

(continued)

| | | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | (A-1) polymerizable monomer having an acidic group | (B) Photosensitizer | (C) Photoacid generator | (D) Photopolymerization accelerator | | | | | |
| Example Q28 | Composition Q28 | First oack | | MDP(4) | - | - | D3-1(1) | | EtOH (46) | | | BHT (0.1) |
| | | Second pack | Bis-GMA(15), TEGDMA(12), MPTMS(2), MDDT(1) | | CQ(0.5) | C5(0.5) | - | E1(20) | EtOH (10) | DW(10) | | MeHQ (0.1) |
| Example Q29 | Composition Q29 | First pack | - | MDP(4) | - | - | D3-2(0.1) | | Ac(6) | DW(40) | | BHT (0.1) |
| | | Second pack | Bis-GMA(15), TEGDMA(12), MPTMS(2), MDDT(1) | | CQ(0.5) | C5(0.5) | - | | EtOH (10) | DW(10) | | BHT (0.1) |
| Example Q30 | Composition Q30 | First pack | - | MDP(4) | - | - | D3-3(20) | | EtOH (46) | | | BHT (0.1) |
| | | Second pack | Bis-GMA(15), TEGDMA(12), MPTMS(2), MDDT(1) | | CQ(0.5) | C5(0.5) | - | | EtOH (10) | DW(10) | | BHT (0.1) |
| Example Q31 | Composition Q31 | First oack | MPTMS(2), MDDT(1) | MDP(4) | CQ(0.5) | C1(0.5) | D3-4(2) | | EtOH (20) | DW(23) | | BHT (0.1) |
| | | Second pack | | | - | - | - | E1(10) | EtOH (20) | DW(30) | | |

(continued)

| | | | (A) Polymerizable monomer | | Photopolymerization initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | (A-1) polymerizable monomer having an acidic group | (B) Photosensitizer | (C) Photoacid generator | (D) Photopolymerization accelerator | | | | | |
| Example Q32 | Composition Q32 | First pack | Bis-GMA(15), TEGDMA(12), MPTMS(2), MDDT(1) | MDP(4) | CQ(0.5) | C2(0.5) | D3-5(2) | E1(10) | Ac(8) | DW(8) | | MeHQ (OIJ |
| | | Second pack | | | - | - | - | | EtOH (20) | DW(30) | | |

[Table 5]

| | | | (A) Polymerizable monomer | | Photopolymerzation initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | A-1 polymerizable monomer having an acidic group | (B) Photosensitizer | (C) Photoacid generator | (D) Photopolymerization accelerator | | | | | |
| Example Q33 | Composition Q33 | First pack | Bis-GMA(5), TEGDMA(12), MPTMS(2), MDDT(1) | MDP(2),META (9) | CQ(0.3) | C2(1) | D3-6(2) | | EtOH (1O) | DW(9) | | MeHQ (0.1) |
| | | Second pack | | | - | - | - | E1(10) | EtOH (20) | DW(30) | | |
| Example Q34 | Composition Q34 | First pack | Bis-GMA(10), TEGDMA(8) | MDP(3).MET (10) | - | - | D3-6(2) | E2(50) | EtOH (1O) | DW(9) | | MeHA (0.1) |
| | | Second pack | MPTMS(2), MDDT(1) | | CQ(0.3) | C6(1) | - | | EtOH (20) | DW(27) | | BHT (0.1) |
| Example Q35 | Composition Q35 | First pack | Bis-GMA(10), TEGDMA(8), MPTMS(2), MDDT(1) | MHPA(2),MET (8) | - | - | D3-7(2) | | EtOH (1O) | DW(9) | | MeHQ (0.1) |
| | | Second pack | MPTMS (2).MDDT(1) | | CQ(0.3) | C6(1) | - | | EtOH (20) | DW(27) | | BHT (0.1) |
| Example Q36 | Composition Q36 | First pack | Bis-GMA(10), TEGDMA(8), MPTMS(2), MDDT(1) | MHPA(2), META(8) | CQ(0.3) | C6(1) | D3-8(2) | E2(10) | EtOH (1O) | DW(9) | | MeHQ (0.1) |
| | | Second pack | MPTMS(5), MDDT(0.1) | | - | - | - | | EtOH (20) | DW (24.9) | | BHT (0.1) |

(continued)

| | | | (A) Polymerizable monomer | | Photopolymerzation initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | A-1 polymerizable monomer having an acidic group | (B) Photosensitizer | (C) Photoacid generator | (D) Photopolymerization accelerator | | | | | |
| Example Q37 | Composition Q37 | First pack | Bis-GMA(10), TEGDMA(8), MPTMS(2), MDDT(1) | MDP(2),MET (8) | - | - | D3-6(2) | | EtOH (1O) | DW(9) | VOA(0.05) | MeHQ (0.1) |
| | | Second pack | MPTMS(3), MDDT(0.1) | | CQ(0.3) | C6(1) | - | | EtOH (20) | DW (26.9) | tBHP(2) | BHT (0.1) |
| Example Q38 | Composition Q38 | First pack | Bis-GMA(10), TEGDMA(8), MPTMS(2), MDDT(1) | MDP(2),MET (8) | CQ(0.3) | C6(1) | D3-6(2) | | EtOH (1O) | DW(9) | GLA(0.1) | BHT (0.1) |
| | | Second pack | | | - | - | D3-5(2) | | EtOH (20) | DW(30) | tBHP(2) | |
| Example Q39 | Composition Q39 | First pack | UDMA(15), TEGDMA(8), MPTMS(2), MDDT(1) | MHPA(2), META(8) | CQ(0.3) | C6(1) | D3-6(2) | | EtOH(4) | DW(10) | GLA(0.1) | BHT (0.1) |
| | | Second pack | | | - | - | - | | EtOH (20) | DW(30) | tBHP(2) | |
| Example Q40 | Composition Q40 | First pack | UDMA(11), GDMA(12), MPTMS(2), MDDT(1) | MHPA(2), META(8) | CQ(0.3) | C6(1) | D3-6(2) | | EtOH(4) | DW(10) | GLA(0.1) | BHT (0.1) |
| | | Second pack | | | - | - | - | | EtOH (20) | DW(30) | tBHP(2) | |

(continued)

| | | | (A) Polymerizable monomer | | Photopolymerzation initiator | | | (E) Filler | (F) Volatile organic solvent | (G) Water | Chemical polymerization initiator or Chemical polymerization accelerator | Others |
|---|---|---|---|---|---|---|---|---|---|---|---|---|
| | | | Polymerizable monomer other than (A-1) | A-1 polymerizable monomer having an acidic group | (B) Photosensitizer | (C) Photoacid generator | (D) Photopolymerization accelerator | | | | | |
| Example Q41 | Composition Q41 | First pack | Bis-GMA(11), TEGDMA(11) | MDP(10) | CQ(0.2) | - | D3-1(1) | | Ac(18) | | | BHT (0.1) |
| | | Second pack | MPTMS(2), MDDT(1) | | - | C1(0.5) | - | | EtOH (20) | DW(27) | | BHT (0.1) |
| Example Q42 | Composition Q42 | First pack | Bis-GMA(11), TEGDMA(6) | MDP(0.01) | CQ(0.2) | - | D3-1(1) | | Ac(18) | | | BHT (0.1) |
| | | Second pack | MPTMS(2), MDDT(1) | | - | C1(0.5) | - | | EtOH (20) | DW(27) | | BHT (0.1) |
| Comparative Example CQ1 | Composition CQ1 | First pack | | MDP(5) | - | - | - | | EtOH (45) | | | |
| | | Second pack | MPTMS(2), MDDT(1) | | - | - | - | | EtOH (20) | DW(27) | | |
| Comparative Example CQ2 | Composition CQ2 | First pack | | | - | - | D3-6(1) | | EtOH (50) | | | BHT(0. |
| | | Second pack | MPTMS(2), MDDT(1) | | - | - | - | | EtOH (20) | DW(27) | | 1) BHT (0.1) |
| Comparative Example CQ3 | Composition CQ3 | First pack | Bis-GMA(15), TEGDMA(15) | MDP(2) | CQ(0.2) | - | - | | Ac(18) | | | |
| | | Second pack | MPTMS(2), MDDT(1) | | - | - | DMBE(1) | | EtOH (20) | DW(27) | | |
| Comparative Example CQ4 | Composition CQ4 | First pack | Bis-GMA(10), TEGDMA(8), MPTMS(2), MDDT(1) | MHPA(2),MET (8) | - | - | - | | EtOH (1O) | DW(9) | VOA(0.05) | |
| | | Second pack | MPTMS(2), MDDT(1) | | CQ(0.3) | C6(1) | - | | EtOH (20) | DW(27) | tBHP(2) | |

<Preparing method of dental curable composition>

All components shown in Tables 6 and 7 other than filler (E) were mixed by using a mix rotor VMRC-5 under the condition of 100 rpm for 48 hours to prepare a binder resin. Then, the binder resin and filler (E) were put into a rotation and revolution mixer (ARV-300), mixed at 1400 rpm for 10 minutes, and then defoamed under vacuum. The one pack type dental curable composition shown in Table 6 was directly collected, and the two packs type dental curable composition shown in Table 7 was filled in a double syringe container manufactured by Mixpack Co., Ltd., to prepare a dental curable composition of the compositions R1 to R7 and the compositions S1 to S7. In the Tables 6 to 7, the content (parts by mass) of each component is indicated by the numerical value in parentheses after the abbreviation of each component.

[Table 6]

| | (A) Polymerizable monomer | (E) Filler | | | | Others | |
|---|---|---|---|---|---|---|---|
| | | | (B) Photosensitizer | (C) Photoacid generator | (D) Photopolymerization accelerator | Polymerization inhibitor | Others |
| Composition R1 | Bis-GMA(60), TEGDMA(40) | E1(250) | CQ(0.3) | C5(3) | - | BHT(0.005) | FA(0.01) |
| Composition R2 | 2.6E(80), TEGDMA (20) | E2(250) | CQ(0.2) | C3(2) | - | MeHQ(0.005) | - |
| Composition R3 | Bis-GMA(60), TEGDMA(40) | E1(250) | CQ(0.3) | C4(0.2) | MDEOA(0.5) | MeHQ(0.005) | FA(0.01) |
| Composition R4 | 2.6E(80), TEGDMA (20) | E2(250) | CQ(0.2) | C2(0.5) | DEAEMA(1.5) | MeHQ(0.005) | - |
| Composition R5 | UDMA(70), TEGDMA (30) | E1(250) | CQ(0.1) | C3(2) | D3-6(2) | MeHQ(0.005) | FA(0.01) |
| Composition R6 | Bis-GMA(60), TEGDMA(40) | E1(250) | CQ(0.2) | - | - | MeHQ(0.005) | FA(0.01) |
| Composition R7 | Bis-GMA(60), TEGDMA(40) | E2(250) | CQ(0.5) | - | DMBE(0.5) | MeHQ(0.005) | - |

[Table 7]

| | | (A) Polymerizable monomer | (E) Filler | Photopolymerization initiator | | | Chemical polymerization initiator or Chemical polymerization accelerator | Others | |
|---|---|---|---|---|---|---|---|---|---|
| | | | | (B) Photosensitizer | (C) Photoacid generator | (D) Photopolymerization accelerator | | Polymerization inhibitor | Others |
| Composition S1 | Paste 1 | Bis-GMA(60), TEGDMA(40) | E1 (200) | CQ(0.6) | - | - | PTU(1.0) | MeHQ(0.005) | FA (0.01) |
| | Paste 2 | UDMA(70), TEGDMA(30) | E1 (200) | - | C1(4) | - | CHP(1.5) | BHT(0.1) | - |
| Composition S2 | Paste 1 | 2.6E(80), TEGDMA(20) | E2 (200) | CQ(0.4) | - | - | PTU(1.0) | MeHQ(0.005) | - |
| | Paste 2 | Bis-GMA(60), TEGDMA(40) | E2 (200) | - | C2(2) | MDEOA(0.5) | CHP(1.5) | BHT(0.1) | - |
| Composition S3 | Paste 1 | UDMA(70), TEGDMA(30) | E1 (200) | CQ(0.2) | C3(1) | - | PTU(1) VOA(0.01) | MeHQ(0.005) | FA (0.01) |
| | Paste 2 | 2.6E(80), TEGDMA(20) | E1 (200) | - | - | D3-6(2) | CHP(1.5) | BHT(0.1) | - |
| Composition S4 | Paste 1 | UDMA(70), TEGDMA(30) | E2 (200) | - - | - - | DEAEMA(1.5) | PTU(1) COA(0.1) | MeHQ(0.005) | - |
| | Paste 2 | Bis-GMA(65), HEMA(25), MDP (10) | E2 (200) | CQ(1) | C4(2) | - | CHP(1.5) | BHT(0.1) | - |
| Composition S5 | Paste 1 | Bis-GMA(60), TEGDMA(20), MHPA(20) | E1 (200) | CQ(0.2) | C5(0.5) | - | PTU(1) COA(0.1) | MeHQ(0.005) | FA (0.01) |
| | Paste 2 | Bis-GMA(60), TEGDMA(40) | E1 (200) | - | - | - | CHP(1.5) | BHT(0.1) | - |
| Composition S6 | Paste 1 | 2.6E(80), TEGDMA(20) | E2 (200) | CQ(1.4) | - | - | PTU(2) VOA(0.01) | MeHQ(0.005) | - |
| | Paste 2 | Bis-GMA(60), TEGDMA(40) | E2 (200) | - | - | - | CHP(3) | BHT(0.1) | - |

(continued)

| | | (A) Polymerizable monomer | (E) Filler | Photopolymerization initiator | | | Chemical polymerization initiator or Chemical polymerization accelerator | Others | |
|---|---|---|---|---|---|---|---|---|---|
| | | | | (B) Photosensitizer | (C) Photoacid generator | (D) Photopolymerization accelerator | | Polymerization inhibitor | Others |
| Composition S7 | Paste 1 | 2.6E(80), TEGDMA(20) | E2 (200) | CQ(0.2) | - | - | PTU(2) VOA(0.01) | MeHQ(0.005) | - |
| | Paste 2 | Bis-GMA(60), TEGDMA(40) | E2 (200) | - | - | - | CHP(3) | BHT(0.1) | - |

The test method using each composition is as follows. For the two packs type dental composition of the compositions S1 to S7, a paste prepared by mixing the first paste and the second paste with a mixing chip manufactured by Mixpack Co., Ltd. was used.

<Storage stability of dental adhesive composition (acceleration test)>

In a dark room where the room temperature is 23 ± 2 °C, 5 mL of each composition was collected with a plastic dropper and filled in a black polypropylene bottle. Thereafter, the nozzle and cap were attached in order, and it was confirmed that the composition is not leak even if it is turned upside down. The bottle filled with the composition was stored in an incubator at 50 °C for 3 months, and it was confirmed that no significant increase in viscosity or gelation occurred.

[Adhesive strength to enamel]

<Adhesion test 1>

A test specimen of an epoxy resin embedded bovine central incisor was polished with water-resistant abrasive paper #600 to carve out an enamel plane. Thereafter, a tape (thickness 20 μm) with a hole having a diameter of 4 mm was affixed to the adherend surface to prescribe an adhesion area. The composition was applied into the tape with the hole and immediately air dried. For the two packs type dental adhesive composition, the same weight were weighed before application, sufficiently mixed, and thereafter applied and then immediately air-dried. Light irradiation was performed for 5 seconds with the dental polymerization LED light irradiator (PEN Bright, SHOFU INC.). A plastic mold with a hole having 2 mm of the height and 4 mm of diameter was overlaid the tape with the hole, and the dental composite resin (BEAUTIFIL Flow Plus A3: SHOFU INC.) or the dental curable composition shown in Table 3 was filled. Thereafter, the light was irradiated for 10 seconds by the dental polymerization LED light irradiator (PEN Bright, SHOFU INC.). After immersing in water at 37 °C for 24 hours, immersing both in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and an Instron universal tester (manufactured by Instron) was used to measure the shear adhesive strength at a crosshead speed of 1 mm/min. Evaluation criteria were as follows.

Particularly good: adhesive strength was 20 MPa or more.
Good: adhesive strength was 12 MPa or more and less than 20 MPa.
Applicable: adhesive strength was 5 MPa or more and less than 12 MPa.
Insufficient: adhesive strength was less than 5 MPa.

In the test in which the dental curable composition of the compositions R1 to R7 were combined with, immersing both in cold water phase at 4 °C for 60 seconds and in high temperature phase at 60 °C for 60 seconds was repeated 20000 times by using a thermal shock tester (manufactured by THOMAS KAGAKU Co.,Ltd). The specimen was taken out and an Instron universal tester (manufactured by Instron) was used to measure the adhesive strength at a crosshead speed of 1 mm/min. Evaluation criteria were as follows.

Extremely good: adhesive strength was 28 MPa or more.
Particularly good: adhesive strength was 20 MPa or more and less than 28 MPa.
Good: adhesive strength was 12 MPa or more and less than 20 MPa.
Applicable: adhesive strength was 5 MPa or more and less than 12 MPa.
Insufficient: adhesive strength was less than 5 MPa.

<Adhesion test 2>

A test specimen of an epoxy resin embedded bovine central incisor was polished with water-resistant abrasive paper #600 to carve out an enamel plane. Thereafter, a tape (thickness 20 μm) with a hole having a diameter of 4 mm was affixed to the adherend surface to prescribe an adhesion area. The composition was applied into the tape with the hole and immediately air dried. For the two packs type dental adhesive composition, the same weight were weighed before application, sufficiently mixed, and thereafter applied and then immediately air-dried. The adherend surface of the stainless rod (φ4.5 mm) was sandblasted (0.2 MPa, 1 second) with alumina (50 μm), then was washed with water and dried, and applied with a metal adhesive primer (METAL LINK, SHOFU INC.). The adherend surface of the stainless rod was applied with appropriate amount of resin cement (Resicem, SHOFU INC.) or a kneaded material of the dental curable composition shown in Table 4, and a tooth substance and the stainless rod were bonded so as to fit in the frame

of the double-sided tape with a hole. A load of 200 N was applied from the vertical direction of the stainless rod, and excess cement was wiped off with a cloth. Thereafter, Light irradiation was performed for 10 seconds with the dental polymerization LED light irradiator (PEN Bright, SHOFU INC.). After removing the load, the prepared adhesive test piece was immersed in water at 37 °C for 24 hours, immersing both in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and an Instron universal tester (manufactured by Instron) was used to measure the shear adhesive strength at a crosshead speed of 1 mm/min. Evaluation criteria were as follows.

Particularly good: adhesive strength was 20 MPa or more.
Good: adhesive strength was 12 MPa or more and less than 20 MPa.
Applicable: adhesive strength was 5 MPa or more and less than 12 MPa.
Insufficient: adhesive strength was less than 5 MPa.

In the test in which the dental curable composition of the compositions S1 to S7 were combined with, immersing both in cold water phase at 4 °C for 60 seconds and in high temperature phase at 60 °C for 60 seconds was repeated 20000 times by using a thermal shock tester (manufactured by THOMAS KAGAKU Co.,Ltd). The specimen was taken out and an Instron universal tester (manufactured by Instron) was used to measure the adhesive strength at a crosshead speed of 1 mm/min. Evaluation criteria were as follows.

Extremely good: adhesive strength was 28 MPa or more.
Particularly good: adhesive strength was 20 MPa or more and less than 28 MPa.
Good: adhesive strength was 12 MPa or more and less than 20 MPa.
Applicable: adhesive strength was 5 MPa or more and less than 12 MPa.
Insufficient: adhesive strength was less than 5 MPa.

When the adhesive strength is low, the risk of detachment of the filler used in filling and repairing and the prosthetic device, marginal leakage and secondary caries and the like increases, therefore the adhesive strength is preferably high.

[Adhesive strength to dentin]

<Adhesion test 1>

A test specimen of an epoxy resin embedded bovine central incisor was polished with water-resistant abrasive paper #600 to carve out a dentin plane. Thereafter, a tape (thickness 20 μm) with a hole having a diameter of 4 mm was affixed to the adherend surface to prescribe an adhesion area. The composition was applied into the tape with the hole and immediately air dried. For the two packs type dental adhesive composition, the same weight were weighed before application, sufficiently mixed, and thereafter applied and then immediately air-dried. Light irradiation was performed for 5 seconds with the dental polymerization LED light irradiator (PEN Bright, SHOFU INC.). A plastic mold with a hole having 2 mm of the height and 4 mm of diameter was overlaid the tape with the hole, and the dental composite resin (BEAUTIFIL Flow Plus A3: SHOFU INC.) or the dental curable composition shown in Table 3 was filled. Thereafter, the light was irradiated for 10 seconds by the dental polymerization LED light irradiator (PEN Bright, SHOFU INC.). After immersing in water at 37 °C for 24 hours, immersing both in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and an Instron universal tester (manufactured by Instron) was used to measure the shear adhesive strength at a crosshead speed of 1 mm/min. Evaluation criteria were as follows.

Particularly good: adhesive strength was 12 MPa or more.
Good: adhesive strength was 8 MPa or more and less than 12 MPa.
Applicable: adhesive strength was 3 MPa or more and less than 8 MPa.
Insufficient: adhesive strength was less than 3 MPa.

In the test in which the dental curable composition of the compositions R1 to R7 were combined with, immersing both in cold water phase at 4 °C for 60 seconds and in high temperature phase at 60 °C for 60 seconds was repeated 20000 times by using a thermal shock tester (manufactured by THOMAS KAGAKU Co.,Ltd). The specimen was taken out and an Instron universal tester (manufactured by Instron) was used to measure the adhesive strength at a crosshead speed of 1 mm/min. Evaluation criteria were as follows.

Extremely good: adhesive strength was 19 MPa or more.
Particularly good: adhesive strength was 12 MPa or more and less than 19 MPa.
Good: adhesive strength was 8 MPa or more and less than 12 MPa.
Applicable: adhesive strength was 3 MPa or more and less than 8 MPa.
Insufficient: adhesive strength was less than 3 MPa.

<Adhesion test 2>

A test specimen of an epoxy resin embedded bovine central incisor was polished with water-resistant abrasive paper #600 to carve out a dentin plane. Thereafter, a tape (thickness 20 μm) with a hole having a diameter of 4 mm was affixed to the adherend surface to prescribe an adhesion area. The composition was applied into the tape with the hole and immediately air dried. For the two packs type dental adhesive composition, the same weight were weighed before application, sufficiently mixed, and thereafter applied and then immediately air-dried. The adherend surface of the stainless rod (φ4.5 mm) was sandblasted (0.2 MPa, 1 second) with alumina (50 μm), then was washed with water and dried, and applied with a metal adhesive primer (METAL LINK, SHOFU INC.). The adherend surface of the stainless rod was applied with appropriate amount of resin cement (Resicem, SHOFU INC.) or a kneaded material of the dental curable composition shown in Table 4, and a tooth substance and the stainless rod were bonded so as to fit in the frame of the double-sided tape with a hole. A load of 200 N was applied from the vertical direction of the stainless rod, and excess cement was wiped off with a cloth. Thereafter, Light irradiation was performed for 10 seconds with the dental polymerization LED light irradiator (PEN Bright, SHOFU INC.). After removing the load, the prepared adhesive test piece was immersed in water at 37 °C for 24 hours, immersing both in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and an Instron universal tester (manufactured by Instron) was used to measure the shear adhesive strength at a crosshead speed of 1 mm/min. Evaluation criteria were as follows.

Particularly good: adhesive strength was 12 MPa or more.
Good: adhesive strength was 8 MPa or more and less than 12 MPa.
Applicable: adhesive strength was 3 MPa or more and less than 8 MPa.
Insufficient: adhesive strength was less than 3 MPa.

In the test in which the dental curable composition of the compositions S1 to S7 were combined with, immersing both in cold water phase at 4 °C for 60 seconds and in high temperature phase at 60 °C for 60 seconds was repeated 20000 times by using a thermal shock tester (manufactured by THOMAS KAGAKU Co.,Ltd). The specimen was taken out and an Instron universal tester (manufactured by Instron) was used to measure the adhesive strength at a crosshead speed of 1 mm/min. Evaluation criteria were as follows.

Extremely good: adhesive strength was 19 MPa or more.
Particularly good: adhesive strength was 12 MPa or more and less than 19 MPa.
Good: adhesive strength was 8 MPa or more and less than 12 MPa.
Applicable: adhesive strength was 3 MPa or more and less than 8 MPa.
Insufficient: adhesive strength was less than 3 MPa.

[Adhesive strength to zirconia]

<Adhesion test 1>

A test specimen having a thickness of 3 mm and a length and a width of 20 mm was prepared by processing zirconia (SHOFU DISK Lucent Supra, manufactured by SHOFU INC.) by a predetermined method. Thereafter, the adherend surface was sandblast-treated and a tape (thickness 20 μm) with a hole having a diameter of 4 mm was affixed to the adherend surface to prescribe an adhesion area. The composition was applied into the tape with the hole and immediately air dried. For the two packs type dental adhesive composition, the same weight were weighed before application, sufficiently mixed, and thereafter applied and then immediately air-dried. Light irradiation was performed for 5 seconds with the dental polymerization LED light irradiator (PEN Bright, SHOFU INC.). A plastic mold with a hole having 2 mm of the height and 4 mm of diameter was overlaid the tape with the hole, and the dental composite resin (BEAUTIFIL Flow Plus A3: SHOFU INC.) or the dental curable composition shown in Table 3 was filled. Thereafter, the light was irradiated for 10 seconds by the dental polymerization LED light irradiator (PEN Bright, SHOFU INC.). After immersing in water at 37 °C for 24 hours, immersing both in cold water phase at 4 °C for 60 seconds and in high temperature phase at 60 °C for 60 seconds was repeated 5000 times by using a thermal shock tester (manufactured

by THOMAS KAGAKU Co.,Ltd). The specimen was taken out and an Instron universal tester (manufactured by Instron) was used to measure the shear adhesive strength at a crosshead speed of 1 mm/min. Evaluation criteria were as follows.

Particularly good: adhesive strength was 20 MPa or more.
Good: adhesive strength was 12 MPa or more and less than 20 MPa.
Applicable: adhesive strength was 5 MPa or more and less than 12 MPa.
Insufficient: adhesive strength was less than 5 MPa.

In the test in which the dental curable composition of the compositions R1 to R7 were combined with, immersing both in cold water phase at 4 °C for 60 seconds and in high temperature phase at 60 °C for 60 seconds was repeated 20000 times by using a thermal shock tester (manufactured by THOMAS KAGAKU Co.,Ltd). The specimen was taken out and an Instron universal tester (manufactured by Instron) was used to measure the adhesive strength at a crosshead speed of 1 mm/min. Evaluation criteria were as follows.

Extremely good: adhesive strength was 28 MPa or more.
Particularly good: adhesive strength was 20 MPa or more and less than 28 MPa.
Good: adhesive strength was 12 MPa or more and less than 20 MPa.
Applicable: adhesive strength was 5 MPa or more and less than 12 MPa.
Insufficient: adhesive strength was less than 5 MPa.

<Adhesion test 2>

A test specimen having a thickness of 3 mm and a length and a width of 20 mm was prepared by processing zirconia (SHOFU DISK Lucent Supra, manufactured by SHOFU INC.) by a predetermined method. Thereafter, the adherend surface was sandblast-treated and a tape (thickness 20 μm) with a hole having a diameter of 4 mm was affixed to the adherend surface to prescribe an adhesion area. The composition was applied into the tape with the hole and immediately air dried. For the two packs type dental adhesive composition, the same weight were weighed before application, sufficiently mixed, and thereafter applied and then immediately air-dried. The adherend surface of the stainless rod (φ4.5 mm) was sandblasted (0.2 MPa, 1 second) with alumina (50 μm), then was washed with water and dried, and applied with a metal adhesive primer (METAL LINK, SHOFU INC.). The adherend surface of the stainless rod was applied with appropriate amount of resin cement (Resicem, SHOFU INC.) or a kneaded material of the dental curable composition shown in Table 4, and a tooth substance and the stainless rod were bonded so as to fit in the frame of the double-sided tape with a hole. A load of 200 N was applied from the vertical direction of the stainless rod, and excess cement was wiped off with a cloth. Thereafter, Light irradiation was performed for 10 seconds with the dental polymerization LED light irradiator (PEN Bright, SHOFU INC.). After removing the load, the prepared adhesive test piece was immersed in water at 37 °C for 24 hours, immersing both in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and an Instron universal tester (manufactured by Instron) was used to measure the shear adhesive strength at a crosshead speed of 1 mm/min. Evaluation criteria were as follows.

Particularly good: adhesive strength was 20 MPa or more.
Good: adhesive strength was 12 MPa or more and less than 20 MPa.
Applicable: adhesive strength was 5 MPa or more and less than 12 MPa.
Insufficient: adhesive strength was less than 5 MPa.

In the test in which the dental curable composition of the compositions S1 to S7 were combined with, immersing both in cold water phase at 4 °C for 60 seconds and in high temperature phase at 60 °C for 60 seconds was repeated 20000 times by using a thermal shock tester (manufactured by THOMAS KAGAKU Co.,Ltd). The specimen was taken out and an Instron universal tester (manufactured by Instron) was used to measure the adhesive strength at a crosshead speed of 1 mm/min. Evaluation criteria were as follows.

Extremely good: adhesive strength was 28 MPa or more.
Particularly good: adhesive strength was 20 MPa or more and less than 28 MPa.
Good: adhesive strength was 12 MPa or more and less than 20 MPa.
Applicable: adhesive strength was 5 MPa or more and less than 12 MPa.
Insufficient: adhesive strength was less than 5 MPa.

[Evaluation of storage stability of acceleration test product]

In the evaluation of the storage stability of the acceleration test product, the adhesive strength of the same composition to the same adherend was measured before and after the acceleration test, and the maintenance rate of the adhesive strength was calculated according to formula 3. Evaluation criteria were as follows.

Particularly good storage stability: maintenance rate was 90% or more.
Good storage stability: maintenance rate was 70% or more and less than 90%.
Applicable storage stability: maintenance rate was 50% or more and less than 70%.
Poor storage stability: maintenance rate was less than 50%.

[Formula 3]

(Adhesive strength after acceleration test [MPa]/Adhesive strength before acceleration test [MPa]) x 100 [%]

Tables 8 and 9 show the results of each composition P and each composition CP.

[Table 8]

| | Adhesion test 1 | | | | | | | | | Adhesion test 2(with light irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example P1 | 27.4 | 24.1 | 88.0 | 14.6 | 12.1 | 82.9 | 29.4 | 23.1 | 78.3 | 22.2 | 18.7 | 84.2 | 17.8 | 15.5 | 86.7 | 29.8 | 21.9 | 73.4 |
| Example P2 | 19.4 | 18.5 | 95.8 | 8.6 | 9.0 | 105.4 | 16.8 | 16.4 | 98.1 | 18.2 | 17.7 | 97.1 | 11.0 | 11.1 | 100.5 | 17.4 | 18.1 | 104.1 |
| Example P3 | 21.1 | 17.4 | 82.5 | 18.0 | 14.9 | 82.5 | 26.0 | 18.8 | 72.1 | 23.2 | 17.9 | 77.0 | 15.0 | 12.1 | 81.0 | 29.1 | 24.4 | 84.0 |
| Example P4 | 27.5 | 24.3 | 88.2 | 17.6 | 14.5 | 82.6 | 26.4 | 19.6 | 74.2 | 26.6 | 23.2 | 87.0 | 17.8 | 12.9 | 72.3 | 22.6 | 17.7 | 78.3 |
| Example P5 | 14.1 | 10.2 | 72.6 | 11.5 | 8.8 | 76.6 | 14.4 | 10.9 | 75.7 | 15.2 | 12.3 | 80.9 | 9.8 | 8.6 | 88.5 | 15.1 | 12.2 | 81.1 |
| Example P6 | 14.5 | 10.3 | 70.6 | 9.4 | 7.0 | 74.4 | 15.1 | 11.5 | 76.1 | 14.6 | 10.8 | 74.0 | 10.4 | 9.3 | 89.8 | 19.1 | 13.5 | 70.9 |
| Example P7 | 24.9 | 26.8 | 107.5 | 12.9 | 13.0 | 100.5 | 26.3 | 25.6 | 97.3 | 21.6 | 22.6 | 104.3 | 12.5 | 13.6 | 109.2 | 21.1 | 21.4 | 101.7 |
| Example P8 | 24.3 | 25.3 | 103.9 | 15.9 | 15.5 | 97.5 | 27.3 | 29.7 | 108.8 | 25.6 | 27.4 | 106.9 | 12.3 | 11.4 | 92.4 | 26.6 | 24.0 | 90.5 |
| Example P9 | 28.8 | 28.0 | 97.0 | 16.9 | 15.5 | 91.7 | 28.5 | 31.2 | 109.5 | 25.4 | 26.3 | 103.7 | 13.2 | 13.2 | 99.7 | 29.0 | 26.7 | 92.2 |
| Example P10 | 21.1 | 21.5 | 102.3 | 13.1 | 13.2 | 101.1 | 25.1 | 26.7 | 106.0 | 27.8 | 26.4 | 95.0 | 13.0 | 13.4 | 102.7 | 22.3 | 21.7 | 97.4 |
| Example P11 | 26.8 | 28.0 | 104.6 | 17.7 | 18.2 | 102.8 | 28.4 | 25.9 | 91.2 | 28.9 | 27.7 | 95.9 | 16.2 | 15.3 | 94.8 | 20.2 | 18.5 | 91.3 |

(continued)

| | Adhesion test 1 | | | | | | | | | Adhesion test 2(with light irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example P12 | 23.7 | 23.4 | 98.4 | 17.0 | 18.1 | 106.1 | 20.6 | 21.0 | 101.8 | 21.1 | 23.0 | 108.8 | 17.7 | 18.6 | 105.0 | 28.2 | 27.6 | 97.6 |
| Example P13 | 27.0 | 25.5 | 94.7 | 17.5 | 15.8 | 90.6 | 20.9 | 19.8 | 95.0 | 22.4 | 23.7 | 105.7 | 15.4 | 16.1 | 105.0 | 22.1 | 22.6 | 102.3 |
| Example P14 | 23.3 | 24.8 | 106.6 | 12.2 | 12.4 | 102.2 | 26.0 | 27.8 | 107.0 | 22.8 | 21.3 | 93.4 | 18.8 | 17.1 | 90.7 | 24.8 | 26.6 | 107.5 |
| Example P15 | 25.8 | 24.9 | 96.7 | 13.8 | 13.1 | 95.1 | 24.7 | 24.2 | 98.0 | 29.2 | 26.6 | 91.0 | 15.0 | 14.7 | 97.8 | 24.7 | 22.5 | 90.8 |
| Example P16 | 25.6 | 26.4 | 103.3 | 18.0 | 19.1 | 106.0 | 21.8 | 22.1 | 101.5 | 23.8 | 25.6 | 107.4 | 13.2 | 12.1 | 91.8 | 23.9 | 25.1 | 105.3 |
| Example P17 | 23.6 | 22.9 | 97.0 | 14.3 | 13.7 | 95.8 | 20.5 | 19.5 | 94.8 | 29.3 | 31.5 | 107.7 | 16.5 | 17.0 | 103.4 | 21.1 | 20.5 | 97.6 |
| Example P18 | 24.7 | 17.4 | 70.1 | 16.2 | 13.8 | 85.3 | 20.0 | 17.1 | 85.3 | 25.1 | 18.5 | 73.6 | 18.4 | 12.9 | 70.1 | 21.5 | 17.7 | 82.4 |
| Example P19 | 29.1 | 22.2 | 76.2 | 12.2 | 10.4 | 85.6 | 28.1 | 24.4 | 86.7 | 29.7 | 23.1 | 77.9 | 14.4 | 11.4 | 78.6 | 27.7 | 20.6 | 74.2 |
| Example P20 | 21.1 | 15.8 | 74.9 | 16.0 | 12.6 | 79.0 | 20.4 | 14.5 | 71.3 | 23.5 | 16.8 | 71.4 | 15.1 | 12.8 | 85.0 | 29.8 | 22.1 | 74.2 |
| Example P21 | 21.1 | 16.8 | 79.6 | 15.2 | 13.4 | 88.1 | 26.0 | 18.8 | 72.1 | 24.3 | 19.4 | 80.0 | 14.7 | 13.1 | 88.8 | 26.7 | 23.8 | 89.0 |
| Example P22 | 29.7 | 23.5 | 79.2 | 14.8 | 12.5 | 83.9 | 28.4 | 21.9 | 77.1 | 27.3 | 20.0 | 73.0 | 13.6 | 9.9 | 72.5 | 28.1 | 24.1 | 85.8 |

(continued)

| | Adhesion test 1 | | | | | | | | | Adhesion test 2(with light irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example P23 | 22.9 | 23.0 | 100.4 | 12.0 | 10.9 | 90.4 | 29.2 | 31.3 | 107.2 | 29.7 | 29.5 | 99.3 | 15.7 | 16.8 | 107.1 | 27.1 | 25.1 | 92.5 |
| Example P24 | 27.1 | 23.1 | 85.5 | 15.9 | 13.6 | 85.6 | 28.2 | 22.9 | 81.2 | 24.6 | 18.8 | 76.7 | 15.0 | 11.1 | 74.1 | 24.9 | 18.9 | 76.0 |
| Example P25 | 24.6 | 18.2 | 73.8 | 16.4 | 12.1 | 73.7 | 29.4 | 26.1 | 88.6 | 23.0 | 20.6 | 89.5 | 16.5 | 13.1 | 79.1 | 29.1 | 20.9 | 71.9 |
| Example P26 | 29.7 | 32.6 | 109.8 | 17.4 | 18.9 | 108.6 | 27.8 | 27.2 | 97.7 | 28.0 | 27.8 | 99.3 | 17.4 | 16.9 | 97.0 | 22.0 | 22.7 | 103.3 |
| Example P27 | 28.9 | 26.0 | 90.1 | 12.3 | 13.0 | 105.5 | 22.8 | 21.1 | 92.6 | 27.5 | 24.9 | 90.7 | 18.3 | 17.6 | 96.3 | 27.6 | 25.6 | 92.8 |
| Example P28 | 20.8 | 19.1 | 91.8 | 14.6 | 13.7 | 93.5 | 23.0 | 24.1 | 104.6 | 21.9 | 21.9 | 99.9 | 19.0 | 20.1 | 106.0 | 29.4 | 30.8 | 104.6 |
| Example P29 | 23.2 | 16.4 | 70.9 | 13.3 | 9.6 | 72.5 | 29.5 | 23.6 | 79.9 | 27.0 | 19.5 | 72.1 | 14.4 | 12.5 | 86.7 | 22.9 | 16.2 | 70.9 |
| Example P30 | 24.4 | 17.3 | 70.9 | 16.6 | 14.0 | 84.2 | 25.9 | 19.5 | 75.2 | 24.4 | 17.4 | 71.1 | 12.9 | 10.8 | 83.2 | 20.1 | 16.4 | 81.6 |

[Table 9]

| | Adhesion test 1 | | | | | | | | | Adhesion test 2 (with lig ht irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate |
| | (MPa) | (MPa) | (%) | (MPa) | MPa | (%) | (MPa) | (MPa) | (%) | (MPa) | (MPa) | (%) | (MPa) | (MPa) | (%) | (MPa) | (MPa) | (%) |
| Example F31 | 24.4 | 24.7 | 101.3 | 16.1 | 15.0 | 92.6 | 21.4 | 20.4 | 95.3 | 21.4 | 21.7 | 101.5 | 14.8 | 14.7 | 99.0 | 23.9 | 21.6 | 90.2 |
| Example F32 | 29.3 | 30.1 | 102.8 | 14.3 | 15.3 | 106.8 | 26.9 | 26.5 | 98.5 | 22.0 | 20.4 | 92.6 | 14.8 | 14.3 | 96.6 | 28.8 | 27.0 | 93.8 |
| Example F33 | 29.5 | 27.5 | 93.2 | 16.6 | 15.3 | 92.3 | 22.5 | 23.7 | 105.2 | 20.6 | 22.4 | 108.9 | 14.0 | 12.7 | 90.3 | 28.3 | 26.5 | 93.5 |
| Example P34 | 26.4 | 25.7 | 97.6 | 14.2 | 13.1 | 92.1 | 21.5 | 23.5 | 109.2 | 21.3 | 20.4 | 96.0 | 15.3 | 15.4 | 100.6 | 20.6 | 21.1 | 102.5 |
| Example P35 | 26.8 | 25.8 | 96.1 | 16.0 | 17.3 | 108.2 | 24.2 | 25.3 | 104.5 | 28.0 | 28.6 | 102.2 | 15.1 | 13.8 | 91.4 | 29.7 | 28.7 | 96.6 |
| Example F36 | 23.2 | 21.3 | 91.9 | 12.4 | 11.3 | 90.9 | 27.7 | 30.4 | 109.6 | 21.0 | 22.2 | 106.1 | 18.4 | 18.9 | 102.6 | 27.8 | 26.2 | 94.2 |
| Example P37 | 21.2 | 20.9 | 98.9 | 12.4 | 12.9 | 104.1 | 21.3 | 22.3 | 104.6 | 25.0 | 25.7 | 102.9 | 16.5 | 15.5 | 94.2 | 22.4 | 24.4 | 109.1 |
| Example P38 | 27.8 | 28.0 | 100.6 | 14.6 | 13.3 | 91.1 | 24.1 | 23.6 | 97.8 | 29.7 | 29.7 | 100.1 | 17.8 | 16.7 | 94.0 | 22.4 | 20.7 | 92.3 |
| Example P39 | 28.8 | 30.5 | 106.0 | 17.1 | 17.0 | 99.7 | 29.5 | 28.7 | 97.3 | 22.6 | 22.6 | 100.4 | 16.8 | 18.3 | 109.0 | 24.2 | 23.3 | 96.4 |
| Example P40 | 26.5 | 26.1 | 98.3 | 14.1 | 14.6 | 103.1 | 22.5 | 23.5 | 104.3 | 21.9 | 23.2 | 105.8 | 13.3 | 13.7 | 102.5 | 22.6 | 20.6 | 90.9 |
| Example P41 | 26.8 | 23.9 | 88.9 | 18.6 | 15.5 | 83.3 | 28.6 | 22.8 | 79.5 | 24.8 | 20.0 | 80.4 | 12.9 | 9.1 | 70.8 | 29.8 | 25.0 | 83.9 |

(continued)

| | Adhesion test 1 | | | | | | | | | Adhesion test 2 (with lig ht irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | MPa | (%) | (MPa) | (MPa) | (%) | (MPa) | (MPa) | (%) | (MPa) | (MPa) | (%) | (MPa) | (MPa) | (%) |
| Example P42 | 25.8 | 24.9 | 96.7 | 13.8 | 13.1 | 95.1 | 24.7 | 24.2 | 98.0 | 29.2 | 26.6 | 91.0 | 15.0 | 14.7 | 97.8 | 24.7 | 22.5 | 90.8 |
| Example P43 | 27.4 | 27.1 | 98.9 | 13.9 | 12.9 | 92.3 | 25.6 | 23.5 | 91.8 | 22.1 | 24.2 | 109.5 | 16.7 | 15.3 | 91.4 | 20.9 | 21.4 | 102.5 |
| Example P44 | 25.3 | 27.4 | 108.4 | 12.5 | 11.4 | 90.8 | 22.7 | 22.0 | 96.9 | 27.5 | 27.3 | 99.2 | 17.8 | 17.1 | 96.5 | 20.6 | 19.9 | 96.4 |
| Example P45 | 21.3 | 19.3 | 90.5 | 18.9 | 18.8 | 99.4 | 26.3 | 27.7 | 105.3 | 27.1 | 27.3 | 100.7 | 13.2 | 13.1 | 99.4 | 22.3 | 20.1 | 90.2 |
| Example P46 | 25.9 | 25.4 | 98.3 | 12.9 | 12.9 | 99.8 | 23.8 | 24.5 | 103.1 | 28.7 | 28.6 | 99.6 | 16.7 | 15.1 | 90.7 | 27.1 | 27.1 | 99.8 |
| Example P47 | 26.2 | 24.9 | 95.0 | 14.9 | 15.7 | 105.6 | 28.5 | 27.2 | 95.7 | 27.0 | 28.9 | 106.8 | 13.3 | 12.1 | 91.0 | 28.4 | 28.0 | 98.5 |
| Comparative Example CP 1 | 26.9 | 3.6 | 13.5 | 18.3 | 2.6 | 14.3 | 25.2 | 12.2 | 48.3 | 25.4 | 7.0 | 27.6 | 18.3 | 6.8 | 37.3 | 29.9 | 13.3 | 44.5 |
| Comparative Example CP2 | 1.3 | 0.5 | 39.8 | 1.5 | 0.4 | 24.3 | 1.6 | 0.4 | 28.4 | 1.5 | 0.4 | 24.4 | 2.6 | 1.0 | 36.1 | 2.2 | 0.8 | 34.6 |
| Comparative Example CP3 | 6.4 | 4.4 | 69.2 | 7.5 | 4.4 | 58.4 | 11.4 | 7.6 | 66.7 | 8.3 | 4.7 | 56.3 | 6.3 | 3.7 | 58.7 | 7.3 | 4.2 | 56.9 |

(continued)

| | Adhesion test 1 | | | | | | | | | Adhesion test 2 (with lig ht irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate | Initial adhe-sive strength | Adhe-sive strength after storage | Mainte-nance rate |
| | (MPa) | (MPa) | (%) | (MPa) | MPa | (%) | (MPa) | (MPa) | (%) | (MPa) | (MPa) | (%) | (MPa) | (MPa) | (%) | (MPa) | (MPa) | (%) |
| Compar-ative Ex-ample CP4 | 18.7 | 2.8 | 15.0 | 9.7 | 3.4 | 34.5 | 19.4 | 9.2 | 47.3 | 14.8 | 6.2 | 42.0 | 9.9 | 4.4 | 45.0 | 17.5 | 8.5 | 48.7 |
| Compar-ative Ex-ample CP5 | 16.5 | 1.9 | 11.2 | 9.5 | 4.7 | 49.3 | 14.6 | 7.0 | 47.6 | 13.8 | 6.5 | 47.5 | 8.6 | 4.2 | 49.1 | 12.6 | 4.9 | 38.5 |
| Compar-ative Ex-ample CP6 | 13.8 | 6.0 | 43.9 | 10.1 | 2.1 | 21.2 | 14.6 | 3.0 | 20.4 | 12.2 | 2.5 | 20.6 | 11.3 | 5.2 | 45.9 | 12.4 | 4.4 | 35.5 |
| Compar-ative Ex-ample CP7 | 19.2 | 6.2 | 32.2 | 11.8 | 2.8 | 23.7 | 19.3 | 3.6 | 18.8 | 15.1 | 4.5 | 29.6 | 10.6 | 2.0 | 18.5 | 15.4 | 4.8 | 31.1 |
| Compar-ative Ex-ample CP8 | 26.5 | 11.3 | 42.6 | 14.5 | 6.4 | 44.4 | 28.6 | 7.2 | 25.0 | 21.5 | 7.6 | 35.5 | 18.8 | 8.3 | 44.4 | 24.3 | 8.4 | 34.6 |
| Compar-ative Ex-ample CP9 | 16.7 | 8.0 | 47.7 | 10.4 | 2.3 | 21.6 | 15.9 | 7.6 | 47.5 | 15.6 | 5.4 | 34.9 | 10.2 | 2.3 | 22.9 | 15.9 | 6.8 | 42.8 |

(continued)

| | Adhesion test 1 | | | | | | | | | Adhesion test 2 (with lig ht irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | MPa | (%) | (MPa) | (MPa) | (%) | (MPa) | (MPa) | (%) | (MPa) | (MPa) | (%) | (MPa) | (MPa) | (%) |
| Comparative Example CP10 | 13.4 | 3.5 | 25.9 | 8.7 | 3.3 | 37.6 | 14.2 | 2.8 | 19.5 | 15.7 | 2.4 | 15.1 | 8.1 | 1.8 | 22.4 | 16.9 | 2.8 | 16.2 |
| Comparative Example CP11 | 16.1 | 7.3 | 45.2 | 9.9 | 1.9 | 19.1 | 15.6 | 6.9 | 44.2 | 15.4 | 4.6 | 30.0 | 11.9 | 4.6 | 38.6 | 15.5 | 2.9 | 18.7 |
| Comparative Example CP 12 | 18.2 | 5.9 | 32.2 | 11.1 | 1.5 | 13.6 | 14.1 | 1.9 | 13.5 | 17.5 | 6.0 | 34.0 | 10.8 | 4.5 | 41.7 | 18.3 | 4.0 | 21.9 |
| Comparative Example CP13 | 14.5 | 6.1 | 42.1 | 9.1 | 1.7 | 18.5 | 17.1 | 3.2 | 18.9 | 17.8 | 8.3 | 46.4 | 11.8 | 5.4 | 45.5 | 18.3 | 2.2 | 12.2 |

The compositions P1 to P47 showed good adhesive property and maintenance rate. Among them, it was confirmed that Examples P1, P7 to P17, P23, P24, P26, P27, P31 to 40, and P42 to 47 had both high adhesive strength and extremely high maintenance rate.

On the other hand, since the compositions CP1 and CP2 did not contain an amine compound, the adhesive strength after storage was significantly reduced. Since the composition CP3 did not contain the (A-1) polymerizable monomer having an acidic group, it was confirmed that the adhesive strength was hardly exhibited. Although the compositions CP4 to CP13 contained an amine compound, since the structure of the amine compound differed from the structure represented by the formula (1), the adhesive strength after storage is remarkably reduced. Based on the above, it is presumed that when the (A-1) polymerizable monomer having an acidic group and the (D-1) aliphatic tertiary amine compound coexist and the compounded amount thereof are appropriate, adhesive property is high and the property is maintained.

Tables 10 to 12 show the results of each composition Q and each composition CQ.

[Table 10]

| | Adhesion te st 1 | | | | | | | | | Adhesion test 2(with lig ht irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example Q1 | 22.0 | 18.8 | 85.4 | 18.1 | 16.1 | 88.9 | 21.6 | 19.2 | 89.1 | 28.0 | 24.0 | 85.7 | 18.7 | 16.7 | 89.3 | 22.0 | 17.7 | 80.5 |
| Example Q2 | 15.2 | 12.1 | 79.4 | 11.9 | 9.5 | 80.1 | 18.5 | 13.3 | 71.6 | 14.3 | 12.3 | 85.8 | 10.3 | 8.5 | 82.6 | 15.3 | 13.4 | 87.7 |
| Example Q3 | 27.4 | 23.0 | 83.7 | 12.7 | 10.8 | 85.0 | 25.2 | 20.7 | 82.2 | 28.4 | 25.4 | 89.5 | 12.4 | 10.5 | 84.8 | 27.7 | 24.2 | 87.4 |
| Example Q4 | 24.3 | 18.8 | 77.3 | 12.3 | 8.7 | 70.7 | 27.2 | 22.4 | 82.2 | 25.0 | 19.7 | 78.7 | 14.7 | 12.2 | 82.8 | 23.4 | 17.0 | 72.6 |
| Example Q5 | 12.6 | 9.9 | 78.5 | 11.5 | 9.9 | 85.7 | 12.2 | 10.5 | 86.3 | 16.8 | 14.9 | 88.3 | 10.0 | 7.5 | 74.6 | 19.5 | 14.8 | 76.0 |
| Example Q6 | 17.7 | 13.2 | 74.5 | 8.1 | 7.0 | 86.4 | 19.8 | 15.5 | 78.5 | 16.8 | 12.4 | 73.7 | 9.2 | 6.7 | 73.1 | 12.3 | 9.6 | 78.4 |
| Example Q7 | 23.3 | 21.4 | 91.9 | 16.3 | 15.2 | 93.7 | 27.4 | 27.4 | 99.7 | 22.3 | 24.0 | 107.7 | 14.2 | 14.6 | 102.3 | 25.8 | 27.4 | 106.0 |
| Example Q8 | 23.3 | 23.2 | 99.6 | 15.5 | 16.3 | 104.9 | 25.5 | 25.6 | 100.2 | 25.1 | 27.6 | 109.7 | 19.0 | 20.4 | 107.7 | 27.7 | 30.0 | 108.2 |
| Example Q9 | 26.7 | 24.4 | 91.4 | 13.8 | 14.0 | 101.5 | 21.3 | 21.9 | 102.4 | 24.0 | 25.8 | 107.6 | 18.4 | 18.6 | 101.3 | 22.8 | 21.2 | 93.0 |
| Example Q10 | 20.5 | 21.0 | 102.2 | 19.0 | 19.1 | 100.9 | 22.5 | 21.3 | 94.6 | 28.8 | 27.9 | 96.8 | 14.9 | 15.8 | 106.6 | 27.8 | 26.4 | 95.0 |

(continued)

| | Adhesion te st 1 | | | | | | | | | Adhesion test 2(with lig ht irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example Q11 | 28.2 | 29.8 | 105.4 | 16.1 | 17.5 | 108.9 | 22.3 | 21.2 | 95.1 | 28.7 | 31.5 | 109.9 | 15.8 | 14.5 | 91.3 | 28.9 | 26.8 | 92.8 |
| Example Q12 | 29.8 | 32.4 | 108.7 | 13.2 | 12.6 | 95.4 | 29.8 | 31.9 | 106.8 | 20.6 | 21.2 | 103.0 | 15.4 | 16.7 | 108.4 | 28.1 | 29.9 | 106.5 |
| Example Q13 | 20.2 | 20.0 | 99.0 | 16.9 | 18.0 | 106.6 | 27.7 | 29.3 | 105.9 | 26.1 | 26.7 | 102.3 | 16.6 | 16.2 | 97.8 | 23.7 | 24.2 | 102.0 |
| Example Q14 | 21.9 | 22.6 | 103.2 | 18.3 | 18.0 | 98.7 | 22.8 | 22.2 | 97.2 | 20.9 | 20.0 | 95.7 | 14.6 | 14.3 | 98.1 | 30.0 | 27.6 | 91.9 |
| Example Q15 | 25.6 | 23.7 | 92.4 | 18.4 | 17.1 | 93.4 | 20.4 | 21.0 | 102.9 | 28.3 | 29.7 | 105.0 | 18.3 | 17.1 | 93.7 | 26.7 | 27.6 | 103.2 |
| Example Q16 | 26.5 | 20.4 | 77.2 | 14.7 | 11.9 | 80.4 | 28.3 | 22.8 | 80.5 | 29.6 | 22.3 | 75.3 | 15.7 | 12.6 | 80.1 | 25.1 | 18.1 | 72.2 |

[Table 11]

| | Adhesion test 1 | | | | | | | | | Adhesion test 2(with light irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example Q17 | 23.6 | 25.2 | 107.0 | 13.0 | 13.9 | 107.4 | 25.7 | 24.1 | 93.8 | 28.4 | 30.6 | 107.6 | 18.3 | 16.8 | 91.8 | 29.1 | 31.8 | 109.1 |
| Example Q18 | 21.3 | 16.4 | 76.9 | 17.3 | 15.6 | 89.9 | 24.6 | 21.7 | 87.9 | 26.1 | 21.4 | 81.9 | 12.6 | 10.3 | 81.7 | 22.1 | 17.9 | 80.9 |
| Example Q19 | 21.5 | 16.5 | 76.8 | 17.8 | 15.7 | 87.9 | 25.5 | 18.5 | 72.5 | 23.9 | 17.2 | 72.1 | 16.7 | 11.9 | 71.1 | 28.0 | 24.2 | 86.3 |
| Example Q20 | 22.1 | 18.2 | 82.5 | 12.9 | 11.3 | 87.7 | 25.4 | 18.5 | 72.8 | 26.3 | 21.1 | 80.2 | 19.0 | 15.9 | 84.0 | 222 | 18.1 | 81.7 |
| Example Q21 | 23.2 | 16.3 | 70.4 | 17.2 | 14.2 | 82.2 | 26.0 | 20.8 | 79.8 | 28.9 | 21.5 | 74.4 | 18.3 | 15.8 | 86.1 | 26.5 | 19.8 | 74.8 |
| Example Q22 | 23.8 | 16.7 | 70.2 | 18.2 | 14.8 | 81.2 | 25.9 | 21.9 | 84.7 | 24.8 | 17.7 | 71.2 | 16.2 | 14.1 | 87.0 | 25.1 | 18.0 | 71.7 |
| Example Q23 | 21.8 | 17.8 | 81.7 | 17.2 | 14.8 | 85.8 | 25.2 | 20.6 | 81.6 | 29.7 | 24.5 | 82.6 | 18.4 | 15.1 | 81.9 | 29.8 | 24.3 | 81.7 |
| Example Q24 | 29.3 | 25.1 | 85.6 | 16.7 | 11.9 | 71.5 | 24.1 | 17.7 | 73.2 | 25.8 | 228 | 88.7 | 13.0 | 9.2 | 71.1 | 21.3 | 16.9 | 79.0 |

(continued)

| | Adhesion test 1 | | | | | | | | | Adhesion test 2(with light irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example Q25 | 27.7 | 19.5 | 70.4 | 16.0 | 11.5 | 71.8 | 20.6 | 14.6 | 71.0 | 29.1 | 242 | 83.4 | 16.7 | 14.7 | 88.3 | 24.3 | 17.8 | 73.2 |
| Example Q26 | 21.9 | 21.9 | 100.3 | 16.5 | 15.0 | 91.4 | 28.6 | 29.3 | 102.4 | 27.4 | 27.2 | 99.0 | 13.6 | 14.3 | 105.6 | 28.2 | 30.6 | 108.8 |
| Example Q27 | 29.5 | 32.4 | 109.7 | 16.7 | 16.8 | 100.7 | 20.7 | 19.5 | 94.4 | 22.5 | 21.1 | 93.7 | 15.5 | 14.5 | 94.0 | 23.3 | 25.6 | 109.8 |
| Example Q28 | 27.6 | 22.3 | 80.8 | 17.8 | 14.5 | 81.6 | 24.5 | 21.3 | 87.2 | 28.3 | 25.2 | 88.9 | 12.4 | 10.9 | 88.0 | 23.5 | 19.6 | 83.6 |
| Example Q29 | 25.3 | 19.5 | 76.8 | 13.9 | 11.0 | 78.9 | 25.5 | 20.6 | 80.8 | 22.6 | 18.1 | 80.0 | 12.5 | 9.2 | 73.6 | 26.5 | 22.6 | 85.3 |
| Example Q30 | 21.8 | 16.7 | 76.9 | 16.7 | 12.9 | 77.4 | 28.7 | 22.7 | 79.0 | 24.8 | 17.8 | 71.9 | 17.4 | 12.9 | 74.0 | 28.2 | 22.2 | 78.8 |
| Example Q31 | 26.4 | 24.9 | 94.3 | 18.5 | 18.1 | 98.2 | 26.2 | 26.6 | 101.8 | 27.5 | 25.2 | 91.7 | 16.9 | 16.1 | 95.3 | 25.0 | 25.8 | 103.2 |
| Example Q32 | 26.7 | 25.0 | 93.6 | 16.9 | 18.0 | 106.6 | 21.4 | 21.2 | 99.1 | 26.9 | 25.3 | 94.0 | 17.2 | 16.6 | 96.5 | 20.4 | 19.5 | 95.6 |

[Table 12]

| | Adhesion test 1 | | | | | | | | | Adhesion test 2(with light irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage (MPa) | Maintenance rate (%) | Initial adhesive strength (MPa) | Adhesive strength after storage (MPa) | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Example Q33 | 27.6 | 29.7 | 107.9 | 14.1 | 14.3 | 101.2 | 25.7 | 24.6 | 95.5 | 23.7 | 24.7 | 104.3 | 18.1 | 18.9 | 104.4 | 27.3 | 28.4 | 104.1 |
| Example Q34 | 29.9 | 28.6 | 95.7 | 12.9 | 12.3 | 95.0 | 25.4 | 27.2 | 107.2 | 27.0 | 28.1 | 104.0 | 15.9 | 16.2 | 102.1 | 26.6 | 27.6 | 103.5 |
| Example Q35 | 28.3 | 25.7 | 90.9 | 17.9 | 17.2 | 95.8 | 30.0 | 28.1 | 93.6 | 20.7 | 21.4 | 103.4 | 18.7 | 17.3 | 92.3 | 29.3 | 28.6 | 97.5 |
| Example Q36 | 20.9 | 22.2 | 106.1 | 17.0 | 18.0 | 105.9 | 25.0 | 24.1 | 96.1 | 28.6 | 25.8 | 90.1 | 12.9 | 13.4 | 104.1 | 26.6 | 29.2 | 109.8 |
| Example Q37 | 20.1 | 19.4 | 96.6 | 14.7 | 15.5 | 105.6 | 22.6 | 22.5 | 99.6 | 23.0 | 24.9 | 108.1 | 15.8 | 14.9 | 94.2 | 24.5 | 24.3 | 99.2 |
| Example Q38 | 23.9 | 22.8 | 95.3 | 13.8 | 13.0 | 94.7 | 27.3 | 24.9 | 91.0 | 26.6 | 24.1 | 90.7 | 14.0 | 13.3 | 95.1 | 22.6 | 24.1 | 106.7 |
| Example Q39 | 26.6 | 26.8 | 100.6 | 14.3 | 13.4 | 93.7 | 22.2 | 20.4 | 92.3 | 28.9 | 28.6 | 98.8 | 18.8 | 19.8 | 105.4 | 28.1 | 29.5 | 104.9 |
| Example Q40 | 27.0 | 26.8 | 99.4 | 14.3 | 14.0 | 97.5 | 28.0 | 26.3 | 93.9 | 27.9 | 26.1 | 93.5 | 18.0 | 19.5 | 108.4 | 20.2 | 20.5 | 101.6 |
| Example Q41 | 24.1 | 22.8 | 94.5 | 15.7 | 17.1 | 108.8 | 26.8 | 26.4 | 98.2 | 21.5 | 22.6 | 105.2 | 14.6 | 15.4 | 105.4 | 27.9 | 27.8 | 99.3 |
| Example Q42 | 29.3 | 23.3 | 79.5 | 14.3 | 10.5 | 73.5 | 28.3 | 24.7 | 87.1 | 29.5 | 22.3 | 75.7 | 14.2 | 11.7 | 82.5 | 27.1 | 21.0 | 77.6 |

(continued)

| | Adhesion test 1 | | | | | | | | | Adhesion test 2(with light irradiation) | | | | | | | | |
|---|---|---|---|---|---|---|---|---|---|---|---|---|---|---|---|---|---|---|
| | Enamel | | | Dentin | | | Zirconia | | | Enamel | | | Dentin | | | Zirconia | | |
| | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage | Maintenance rate | Initial adhesive strength | Adhesive strength after storage (MPa) | Maintenance rate (%) | Initial adhesive strength (MPa) | Adhesive strength after storage (MPa) | Maintenance rate |
| | (MPa) | (MPa) | (%) | (MPa) | (MPa) | (%) | (MPa) | (MPa) | (%) | (MPa) | (MPa) | (%) | (MPa) | (MPa) | (%) | (MPa) | (MPa) | (%) |
| Comparative Example CQ1 | 18.6 | 6.1 | 32.9 | 9.8 | 4.7 | 47.9 | 19.7 | 8.8 | 44.8 | 12.3 | 1.9 | 15.4 | 8.4 | 1.9 | 22.8 | 14.7 | 3.2 | 21.5 |
| Example CQ1 Comparative Example CQ2 | 1.4 | 1.2 | 83.4 | 1.9 | 1.4 | 76.8 | 3.6 | 3.2 | 89.2 | 4.6 | 3.5 | 75.1 | 2.3 | 1.7 | 70.8 | 26 | 1.8 | 70.1 |
| Example Comparative Example CQ3 | 16.4 | 6.4 | 39.2 | 10.3 | 3.6 | 35.0 | 16.1 | 4.3 | 27.0 | 16.3 | 4.5 | 27.6 | 10.4 | 4.0 | 38.5 | 13.7 | 1.4 | 10.2 |
| Example CQ3 Comparative Example CQ4 | 18.7 | 3.6 | 19.5 | 9.8 | 4.9 | 49.4 | 14.0 | 3.6 | 25.4 | 15.1 | 25 | 16.8 | 11.1 | 3.8 | 34.3 | 12.2 | 3.3 | 27.5 |

The compositions Q1 to Q40 showed good adhesive property and maintenance rate. Among them, it was confirmed that Examples Q7 to 17, Q26, Q27 and Q31 to 40 had both high adhesive strength and extremely high maintenance rate.

On the other hand, since the composition CQ1 did not contain an amine compound, the adhesive strength after storage was significantly reduced. Since the composition CQ2 did not contain the (A-1) polymerizable monomer having an acidic group, it was confirmed that the adhesive strength was hardly exhibited. The composition of Composition CQ3 showed good adhesive property, but its maintenance rate was low. Although it contains an amine compound, it is considered to be derived from the fact that the amine compound is an aromatic amine. The composition CQ4 showed good adhesive property by containing a photoacid generator, but the adhesive strength after storage was significantly reduced. It was confirmed that good adhesive strength and its maintenance rate were exhibited in the test of composition Q in the same manner as in the adhesion test 1 in which after applying each composition, air drying was performed, and then light irradiation was performed for 5 seconds with the dental polymerization LED light irradiator (PEN Bright, SHOFU INC.).

Table 13 shows the test results of kit R and kit CR, which consist of a combination of a curable composition R1 to R7 and an adhesive compositions P4, P14, P35, CP3, CP5 or CP11.

[Table 13]

| | Dental adhesive composition | Dental curable composition | Adhesion test | | | | | | | | |
|---|---|---|---|---|---|---|---|---|---|---|---|
| | | | Enamel | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example R1 | Composition P4 | Composition R1 | 30.9 | 33.8 | 109.7 | 23.4 | 21.5 | 91.9 | 30.1 | 28.5 | 94.5 |
| Example R2 | | Composition R2 | 33.5 | 36.9 | 110.0 | 24.7 | 24.1 | 97.8 | 33.9 | 30.8 | 90.8 |
| Example R3 | | Composition R3 | 31.5 | 33.9 | 107.7 | 20.4 | 18.6 | 91.1 | 32.7 | 31.9 | 97.7 |
| Example R4 | | Composition R4 | 28.6 | 26.6 | 92.9 | 23.8 | 23.9 | 100.4 | 31.6 | 31.5 | 99.6 |
| Example R5 | | Composition R5 | 33.1 | 30.6 | 92.5 | 19.1 | 18.1 | 94.5 | 28.6 | 28.0 | 97.9 |
| Example R6 | | Composition R6 | 22.0 | 18.8 | 85.5 | 17.8 | 13.0 | 73.0 | 20.4 | 16.6 | 81.4 |
| Example R7 | | Composition R7 | 21.1 | 18.4 | 86.8 | 15.9 | 11.5 | 72.1 | 21.6 | 15.2 | 70.5 |

(continued)

| | Dental adhesive composition | Dental curable composition | Adhesion test | | | | | | | | |
|---|---|---|---|---|---|---|---|---|---|---|---|
| | | | Enamel | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example R8 | Composition P14 | Composition R1 | 20.1 | 18.9 | 94.3 | 12.2 | 12.8 | 104.3 | 24.8 | 24.9 | 100.4 |
| Example R9 | | Composition R2 | 22.9 | 25.1 | 109.6 | 16.5 | 17.6 | 106.4 | 20.2 | 21.7 | 107.4 |
| Example R10 | | Composition R3 | 21.5 | 19.8 | 92.1 | 14.3 | 13.2 | 92.4 | 20.8 | 22.5 | 108.4 |
| Example R11 | | Composition R4 | 22.7 | 23.3 | 102.8 | 16.5 | 15.2 | 92.2 | 22.6 | 21.8 | 96.4 |
| Example R12 | | Composition R5 | 23.9 | 23.9 | 99.7 | 18.4 | 18.2 | 98.9 | 22.4 | 22.8 | 101.8 |
| Example R13 | | Composition R6 | 15.2 | 14.1 | 92.9 | 9.1 | 8.4 | 92.5 | 14.0 | 13.1 | 93.7 |
| Example R14 | | Composition R7 | 16.3 | 15.0 | 92.0 | 11.3 | 12.0 | 105.6 | 18.3 | 19.8 | 108.3 |

(continued)

| | Dental adhesive composition | Dental curable composition | Adhesion test | | | | | | | | |
|---|---|---|---|---|---|---|---|---|---|---|---|
| | | | Enamel | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example R15 | Composition P35 | Composition R1 | 32.4 | 32.4 | 100.0 | 25.0 | 27.1 | 108.3 | 32.0 | 33.8 | 105.5 |
| Example R16 | | Composition R2 | 32.4 | 34.5 | 106.4 | 20.0 | 18.2 | 91.1 | 31.7 | 29.7 | 93.8 |
| Example R17 | | Composition R3 | 31.7 | 32.1 | 101.2 | 21.7 | 22.9 | 105.2 | 30.2 | 30.1 | 99.8 |
| Example R18 | | Composition R4 | 28.4 | 27.5 | 96.8 | 20.4 | 18.9 | 92.7 | 29.4 | 30.6 | 104.0 |
| Example R19 | | Composition R5 | 32.8 | 32.9 | 100.4 | 23.1 | 24.9 | 107.8 | 31.2 | 33.3 | 106.6 |
| Example R20 | | Composition R6 | 23.6 | 24.1 | 102.2 | 16.7 | 16.1 | 96.5 | 24.9 | 22.9 | 91.7 |
| Example R21 | | Composition R7 | 20.6 | 20.5 | 99.5 | 15.6 | 15.0 | 96.0 | 21.8 | 20.6 | 94.6 |

(continued)

| | Dental adhesive composition | Dental curable composition | Adhesion test | | | | | | | | |
|---|---|---|---|---|---|---|---|---|---|---|---|
| | | | Enamel | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Comparative Example CR1 | Composition CP3 | Composition R1 | 4.7 | 4.5 | 95.1 | 2.7 | 2.8 | 102.3 | 1.5 | 1.7 | 109.6 |
| Comparative Example CR2 | | Composition R2 | 3.8 | 4.0 | 106.8 | 2.8 | 2.9 | 102.4 | 2.8 | 2.8 | 101.5 |
| Comparative Example CR3 | | Composition R3 | 2.8 | 3.0 | 109.6 | 2.6 | 2.4 | 90.9 | 3.2 | 3.3 | 103.8 |
| Comparative Example CR4 | | Composition R4 | 2.2 | 2.4 | 106.8 | 2.7 | 2.7 | 102.3 | 4.6 | 4.6 | 99.5 |
| Comparative Example CR5 | | Composition R5 | 2.2 | 2.3 | 105.6 | 1.5 | 1.5 | 97.4 | 4.1 | 3.7 | 90.8 |
| Comparative Example CR6 | | Composition R6 | 1.6 | 1.6 | 101.5 | 1.6 | 1.4 | 90.6 | 3.8 | 4.2 | 108.6 |
| Comparative Example CR7 | | Composition R7 | 1.8 | 1.8 | 99.9 | 1.3 | 1.3 | 99.0 | 2.8 | 2.8 | 99.3 |

(continued)

| | Dental adhesive composition | Dental curable composition | Adhesion test | | | | | | | | |
|---|---|---|---|---|---|---|---|---|---|---|---|
| | | | Enamel | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Comparative Example CR8 | Composition CP5 | Composition R1 | 24.6 | 11.7 | 47.5 | 15.4 | 4.7 | 30.4 | 21.8 | 8.6 | 39.2 |
| Comparative Example CR9 | | Composition R2 | 23.3 | 4.4 | 18.8 | 18.7 | 3.8 | 20.4 | 22.9 | 9.4 | 41.1 |
| Comparative Example CR10 | | Composition R3 | 22.9 | 4.8 | 20.9 | 13.2 | 4.7 | 35.8 | 24.8 | 5.3 | 21.5 |
| Comparative Example CR11 | | Composition R4 | 22.9 | 8.0 | 35.0 | 13.5 | 1.8 | 13.2 | 23.7 | 8.0 | 33.9 |
| Comparative Example CR12 | | Composition R5 | 23.9 | 3.1 | 13.1 | 12.0 | 1.4 | 12.1 | 24.1 | 5.2 | 21.5 |
| Comparative Example CR13 | | Composition R6 | 23.7 | 3.1 | 13.2 | 15.5 | 52 | 332 | 24.3 | 4.1 | 17.0 |
| Comparative Example CR14 | | Composition R7 | 22.0 | 8.7 | 39.7 | 17.0 | 5.8 | 34.2 | 21.0 | 2.8 | 13.2 |

(continued)

| | Dental adhesive composition | Dental curable composition | Adhesion test | | | | | | | | |
|---|---|---|---|---|---|---|---|---|---|---|---|
| | | | Enamel | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Comparative Example CR15 | Composition CP11 | Composition R1 | 22.4 | 2.3 | 10.0 | 14.6 | 4.5 | 30.7 | 24.2 | 2.8 | 11.6 |
| Comparative Example CR16 | | Composition R2 | 20.3 | 5.6 | 27.7 | 15.4 | 2.5 | 16.3 | 23.2 | 3.7 | 16.0 |
| Comparative Example CR17 | | Composition R3 | 24.1 | 10.9 | 45.3 | 16.5 | 2.5 | 14.9 | 24.1 | 4.9 | 20.2 |
| Comparative Example CR18 | | Composition R4 | 22.9 | 6.7 | 29.4 | 12.8 | 22 | 17.4 | 24.3 | 5.8 | 24.0 |
| Comparative Example CR19 | | Composition R5 | 23.6 | 10.0 | 42.5 | 14.6 | 6.0 | 41.4 | 232 | 2.7 | 11.6 |
| Comparative Example CR20 | | Composition R6 | 22.6 | 8.3 | 36.6 | 13.9 | 5.8 | 41.9 | 23.9 | 7.4 | 30.9 |
| Comparative Example CR21 | | Composition R7 | 20.7 | 6.3 | 30.4 | 15.5 | 1.8 | 11.4 | 21.2 | 10.5 | 49.5 |

Examples R1 to R5, R8 to R12 and R15 to R19 showed good adhesive property and maintenance rate. Among them, Examples R1 to R5 and R15 to R19 showed extremely high adhesive property, and a synergistic effect exhibited by containing (A-1) polymerizable monomer having an acidic group and (D-1) aliphatic tertiary amine compound represented by formula (1) in the dental adhesive composition and containing a photoacid generator in the curable composition was confirmed. Although the adhesive strength of Examples R6 and R7 was reduced after storage, it is presumed that it was derived from the fact that it does not contain (C) photoacid generator. In Comparative Examples CR1 to CR7 which were a combination with the composition CP3, since CP3 did not contain (A-1) polymerizable monomer having an acidic group, it was confirmed that the adhesive strength was hardly exhibited. It was confirmed that in Comparative Examples CR8 to CR21 containing the composition CP5 or CP11, adhesive strength after storage was low and maintenance rate was reduced. Since the adhesive composition contained only the (D-1) aliphatic tertiary amine compound represented by formula (1), like other compositions, it is presumed that the adhesive strength decreased after storage.

Table 14 shows the test results of the kits of Examples S1 to S42 consisting of a combination of the two-packs type dental curable composition S1 to S7 and the adhesive composition P1, P15, P36, Q1, Q7 or Q14.

[Table 14]

| | Dental adhesive composition | Dental curable composition | Adhesion test 2 | | | | | | | | |
|---|---|---|---|---|---|---|---|---|---|---|---|
| | | | Ename l | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example S1 | Composition P1 | Composition S1 | 31.8 | 34.0 | 107.0 | 25.4 | 25.0 | 98.5 | 31.0 | 31.3 | 101.2 |
| Example S2 | | Composition S2 | 28.5 | 27.5 | 96.3 | 22.5 | 22.2 | 98.9 | 30.7 | 31.2 | 101.6 |
| Example S3 | | Composition S3 | 29.0 | 272 | 93.8 | 25.5 | 27.7 | 1089 | 31.6 | 31.2 | 98.9 |
| Example S4 | | Composition S4 | 33.9 | 30.8 | 90.8 | 20.7 | 19.1 | 92.2 | 31.0 | 31.5 | 101.8 |
| Example S5 | | Composition S5 | 31.0 | 322 | 104.1 | 24.1 | 21.8 | 90.7 | 30.6 | 27.6 | 90.4 |
| Example S6 | | Composition S6 | 24.6 | 25.4 | 103.3 | 15.8 | 14.3 | 90.5 | 24.1 | 23.0 | 95.4 |
| Example S7 | | Composition S7 | 24.6 | 23.4 | 95.1 | 16.1 | 15.6 | 96.7 | 20.9 | 19.8 | 94.5 |

(continued)

| | Dentaladhesive composition | Dental curable composition | Adhesion test 2 | | | | | | | | |
|---|---|---|---|---|---|---|---|---|---|---|---|
| | | | Ename l | | | Dentin | | | Zirconia | | |
| | | | Initial adhe-sive strength | Adhesive strength af-ter storage | Maintenance rate | Initial adhe-sive strength | Adhesive strength af-ter storage | Maintenance rate | Initial adhe-sive strength | Adhesive strength af-ter storage | Maintenance rate |
| | | | (MPa) | (MPa) | (%) | (MPa) | (MPa) | (%) | (MPa) | (MPa) | (%) |
| Example S8 | Composition P15 | Composition S1 | 30.8 | 30.3 | 98.3 | 21.5 | 19.7 | 91.8 | 33.4 | 32.4 | 96.8 |
| Example S9 | | Composition S2 | 28.4 | 29.4 | 103.8 | 19.9 | 21.4 | 107.6 | 30.3 | 30.9 | 101.9 |
| Example S10 | | Composition S3 | 29.5 | 26.9 | 91.3 | 19.1 | 18.3 | 95.6 | 32.2 | 33.8 | 105.1 |
| Example 811 | | Composition S4 | 28.3 | 28.0 | 99.0 | 19.9 | 19.9 | 99.8 | 28.1 | 27.9 | 99.1 |
| Example S12 | | Composition S5 | 33.5 | 36.3 | 108.4 | 24.4 | 24.8 | 101.6 | 31.9 | 32.3 | 101.3 |
| Example S13 | | Composition S6 | 24.7 | 25.8 | 104.5 | 18.6 | 17.6 | 94.5 | 23.0 | 23.6 | 102.4 |
| Example S14 | | Composition S7 | 24.1 | 22.1 | 91.5 | 14.1 | 13.3 | 94.2 | 20.3 | 20.2 | 99.6 |

(continued)

| | Dental adhesive composition | Dental curable composition | Adhesion test 2 | | | | | | | | |
|---|---|---|---|---|---|---|---|---|---|---|---|
| | | | Ename l | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example S16 | Composition P36 | Composition S1 | 30.4 | 32.5 | 107.0 | 23.7 | 23.8 | 100.3 | 32.1 | 28.9 | 90.1 |
| Example S16 | | Composition S2 | 32.4 | 35.0 | 108.1 | 25.2 | 25.8 | 102.4 | 30.3 | 31.9 | 105.0 |
| Example S17 | | Composition S3 | 33.6 | 30.7 | 91.5 | 20.5 | 22.1 | 107.4 | 29.0 | 30.9 | 106.6 |
| Example S18 | | Composition S4 | 29.8 | 28.6 | 96.1 | 20.3 | 21.3 | 104.6 | 28.7 | 31.3 | 108.8 |
| Example S19 | | Composition S5 | 33.9 | 36.3 | 107.2 | 22.7 | 22.8 | 100.4 | 31.8 | 34.1 | 107.4 |
| Example S20 | | Composition S6 | 20.8 | 19.3 | 92.6 | 14.8 | 14.9 | 100.9 | 23.7 | 24.6 | 103.8 |
| Example S21 | | Composition S7 | 21.0 | 19.1 | 91.0 | 18.4 | 19.6 | 106.8 | 20.1 | 21.0 | 104.5 |

(continued)

| | Dental adhesive composition | Dental curable composition | Adhesion test 2 | | | | | | | | |
|---|---|---|---|---|---|---|---|---|---|---|---|
| | | | Ename l | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example S22 | Composition Q1 | Composition S1 | 30.2 | 29.0 | 96.1 | 19.9 | 21.6 | 108.7 | 30.5 | 30.1 | 98.7 |
| Example S23 | | Composition S2 | 30.8 | 28.9 | 94.0 | 23.0 | 21.5 | 93.2 | 30.6 | 32.8 | 107.2 |
| Example S24 | | Composition S3 | 32.1 | 35.2 | 109.6 | 22.1 | 20.4 | 92.0 | 31.9 | 33.3 | 104.2 |
| Example S25 | | Composition S4 | 32.8 | 32.9 | 100.4 | 24.3 | 22.9 | 94.2 | 28.5 | 30.3 | 106.1 |
| Example S26 | | Composition S5 | 31.5 | 31.8 | 101.0 | 24.9 | 26.5 | 106.4 | 32.6 | 30.2 | 92.8 |
| Example S27 | | Composition S6 | 23.6 | 25.9 | 109.7 | 17.8 | 16.3 | 92.0 | 20.7 | 19.0 | 92.1 |
| Example S28 | | Composition S7 | 21.5 | 22.8 | 106.2 | 15.9 | 16.0 | 100.5 | 20.4 | 21.0 | 102.6 |

(continued)

| | Dental adhesive composition | Dental curable composition | Adhesion test 2 | | | | | | | | |
|---|---|---|---|---|---|---|---|---|---|---|---|
| | | | Ename l | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example S29 | Composition Q7 | Composition S1 | 31.9 | 29.9 | 93.6 | 23.0 | 21.4 | 93.1 | 29.4 | 28.0 | 95.1 |
| Example S30 | | Composition S2 | 33.1 | 35.0 | 105.7 | 19.3 | 21.1 | 109.4 | 32.8 | 30.1 | 91.8 |
| Example S31 | | Composition S3 | 28.9 | 30.4 | 105.1 | 24.4 | 23.7 | 97.3 | 30.2 | 31.9 | 105.5 |
| Example S32 | | Composition S4 | 30.2 | 29.5 | 97.9 | 23.6 | 23.4 | 99.1 | 29.7 | 28.6 | 96.3 |
| Example S33 | | Composition S5 | 33.6 | 34.0 | 101.3 | 24.4 | 22.9 | 93.8 | 28.8 | 31.3 | 108.5 |
| Example S34 | | Composition S6 | 20.9 | 19.6 | 93.6 | 12.6 | 12.0 | 95.5 | 21.7 | 21.8 | 100.5 |
| Example S35 | | Composition S7 | 23.2 | 23.6 | 101.7 | 18.0 | 16.5 | 91.6 | 23.9 | 22.0 | 92.1 |

(continued)

| | Dental adhesive composition | Dental curable composition | Adhesion test 2 | | | | | | | | |
|---|---|---|---|---|---|---|---|---|---|---|---|
| | | | Ename l | | | Dentin | | | Zirconia | | |
| | | | Initial adhesive strength | Adhesive strength after storage | Maintenance rate | Initial adhesive strength | Adhesive strength after storage | Maintenance rate | Initial adhesive strength | Adhesive strength after storage | Maintenance rate |
| | | | (MPa) | (MPa) | (%) | (MPa) | (MPa) | (%) | (MPa) | (MPa) | (%) |
| Example S36 | Composition Q14 | Composition S1 | 32.8 | 35.0 | 106.8 | 21.1 | 22.9 | 108.7 | 32.7 | 30.6 | 93.6 |
| Example S37 | | Composition S2 | 28.7 | 27.8 | 96.6 | 25.9 | 26.0 | 100.4 | 33.9 | 33.6 | 99.4 |
| Example S38 | | Composition S3 | 31.0 | 28.7 | 92.4 | 22.5 | 21.6 | 96.2 | 33.3 | 34.2 | 102.7 |
| Example S39 | | Composition S4 | 29.1 | 27.3 | 93.7 | 25.6 | 25.9 | 101.2 | 29.8 | 30.8 | 103.2 |
| Example S40 | | Composition S5 | 34.0 | 31.9 | 94.0 | 21.8 | 22.5 | 103.4 | 29.9 | 28.6 | 95.8 |
| Example S41 | | Composition S6 | 24.0 | 25.5 | 106.5 | 15.5 | 16.6 | 107.3 | 24.2 | 25.5 | 105.1 |
| Example S42 | | Composition S7 | 21.2 | 22.6 | 106.8 | 18.0 | 16.2 | 90.1 | 20.5 | 21.6 | 105.5 |

Examples S1 to S42 showed good adhesive property and maintenance rate. In particular, kits S1 to S5, S8 to S12, S15 to S19, S22 to S26, S29 to S33 and S36 to S40 showed extremely high adhesive property, and a synergistic effect exhibited by containing (A-1) polymerizable monomer having an acidic group and (D-1) aliphatic tertiary amine compound represented by formula (1) in the dental adhesive composition and containing a photoacid generator in the curable composition was confirmed.

The dental adhesive composition of the present invention evaluated in Examples can be used for any known dental adhesive material, dental composite resin, dental core build-up material, dental resin cement, dental coating material, dental sealant material, dental manicure material, dental splinting material and dental glass ionomer cement and the like.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

[Industrial applicability]

According to the present invention, it is possible to provide a dental adhesive composition having excellent storage stability and adhesive strength.

## Claims

1. A dental adhesive composition, comprising (A) polymerizable monomer, (D) photopolymerization accelerator, and (F) volatile organic solvent, wherein,

   the dental adhesive composition comprises (A-1) polymerizable monomer having an acidic group as the (A) polymerizable monomer, and
   the dental adhesive composition comprises (D-1) aliphatic tertiary amine compound represented by formula (1) as the (D) photopolymerization accelerator.

   [Formula (1)]

   [Chemical formula 1]

   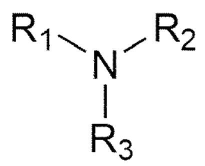

   (wherein $R_1$ represents a substituent consisting of three or more carbons having an electron-attracting group at $\alpha$-carbon and/or $\beta$-carbon of an amine starting from N, $R_2$ represents a substituent consisting of three or more carbons which may have an electron-attracting group, $R_3$ represents a substituent consisting of one or more carbons which may have an electron-attracting group, and $\alpha$-carbon of N in the formula (1) is not an electron-attracting group.)

2. The dental adhesive composition according to claim 1, wherein
   the electron-attracting group in $R_1$ is a substituent selected from a functional group selected from the group consisting of a hydroxyl group, a carboxyl group, a vinyl group, an aryl group and a halogen, and, an organic group which is bonded via an ether bond, an ester bond, a urethane bond or a urea bond and may have -OH group, -O-group, -C(O)-group, -S-group, -NH-C(O)-NH-group, -C(O)-O-group, -O-C(O)-group, -OC (O)-NH-group, -NH-C(O)-O-group, an aromatic hydrocarbon group, or a polymerizable functional group capable of radical polymerization.

3. The dental adhesive composition according to claim 1 or 2, wherein
   the (D-1) aliphatic tertiary amine compound represented by formula (1) is an aliphatic tertiary amine compound in which $R_1$ and $R_2$ have an aliphatic substituent consisting of three or more carbons having an electron-attracting group at $\alpha$-carbon and/or $\beta$-carbon.

**4.** The dental adhesive composition according to claim 1 or 2, wherein
the (D-1) aliphatic tertiary amine compound represented by formula (1) is an aliphatic tertiary amine compound in which $R_1$ and $R_2$ have an aryl group which may have a substituent, at $\alpha$-carbon and/or $\beta$-carbon.

**5.** The dental adhesive composition according to any one of claims 1 to 4, wherein
the dental adhesive composition further comprises (G) water.

**6.** The dental adhesive composition according to any one of claims 1 to 5, wherein
the dental adhesive composition further comprises (B) photosensitizer.

**7.** The dental adhesive composition according to any one of claims 1 to 6, wherein
the dental adhesive composition further comprises (C) photoacid generator.

**8.** The dental adhesive composition according to claim 7, wherein

the dental adhesive composition comprises an aryl iodonium salt as the (C) photoacid generator, and
the aryl iodonium salt is a salt of an anion having an organic group and one or more atoms of P, B, Al, S and Ga, and an aryl iodonium cation.

**9.** The dental adhesive composition according to claim 7, wherein

the dental adhesive composition comprises an aryl iodonium salt as the (C) photoacid generator, and
the aryl iodonium salt is a salt of an anion having an organic group in which at least one H is substituted with F and one or more atoms of P, B, Al, S and Ga, and an aryl iodonium cation.

**10.** The dental adhesive composition according to any one of claims 1 to 9, wherein
the dental adhesive composition is one pack type dental adhesive composition comprising, with respect to 100 parts by mass of total of the (A) polymerizable monomer and the (F) volatile organic solvent or with respect to 100 parts by mass of total of the (A) polymerizable monomer, the (F) volatile organic solvent and (G) water in the case of containing the (G) water,

0.1 to 20 parts by mass of the (A-1) polymerizable monomer having an acidic group,
1 to 99.9 parts by mass of the (F) volatile organic solvent, and
0.01 to 20 parts by mass of the (D-1) aliphatic tertiary amine compound represented by formula (1).

**11.** The dental adhesive composition according to any one of claims 1 to 9, wherein

the dental adhesive composition is two packs type dental adhesive composition consisting of a first pack and a second pack, wherein
a mass ratio of the first pack and the second pack is 0.8:1 to 1.2:1,
the first pack comprises the (A-1) polymerizable monomer having an acidic group, the (F) volatile organic solvent and the (D-1) aliphatic tertiary amine compound represented by formula (1),
the first pack comprises, with respect to 100 parts by mass of total of the (A) polymerizable monomer and the (F) volatile organic solvent contained in the first pack or with respect to 100 parts by mass of total of the (A) polymerizable monomer, the (F) volatile organic solvent and (G) water in the case of containing the (G) water contained in the first pack,

0.2 to 20 parts by mass of the (A-1) polymerizable monomer having an acidic group,
1 to 99.9 parts by mass of the (F) volatile organic solvent, and
0.02 to 20 parts by mass of the (D-1) aliphatic tertiary amine compound represented by formula (1).

**12.** A dental adhesive kit comprising a dental photocurable composition and the dental adhesive composition according to any one of claims 1 to 11, wherein
the dental photocurable composition comprises, with respect to 100 parts by mass of the (A) polymerizable monomer contained in the dental photocurable composition,

0.001 to 2 parts by mass of (B) photosensitizer, and
0.01 to 10 parts by mass of the (C) photoacid generator.

Europäisches Patentamt
European Patent Office
Office européen des brevets

# EUROPEAN SEARCH REPORT

Application Number
EP 21 16 2479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 782 598 A1 (KURARAY NORITAKE DENTAL INC [JP]) 24 February 2021 (2021-02-24)<br>* claim 1 *<br>* table 2 *<br>* paragraphs [0097], [0098], [0131] - [0133] *<br>----- | 1-3,5,6, 10 | INV.<br>A61K6/62<br>A61K6/887<br>C08F2/50<br>A61K6/30 |
| X | JP 5 379563 B2 (TOKUYAMA DENTAL CORP) 25 December 2013 (2013-12-25)<br>* table 1 *<br>* paragraphs [0056], [0064], [0065], [0097], [0098], [0113] *<br>----- | 1-7 | |
| X | EP 2 163 234 A1 (TOKUYAMA DENTAL CORP [JP]) 17 March 2010 (2010-03-17)<br>* tables 7, 8 *<br>* paragraphs [0020], [0063] - [0067], [0079] - [0082], [0109], [0110], [0125], [0162], [0169] *<br>----- | 1-9,11, 12 | |
| X | EP 2 394 628 A1 (TOKUYAMA DENTAL CORP [JP]) 14 December 2011 (2011-12-14)<br>* tables 2, 4, 5 *<br>* paragraphs [0055], [0056], [0068] - [0071], [0101] - [0110] *<br>----- | 1-9,11, 12 | TECHNICAL FIELDS SEARCHED (IPC)<br>A61K<br>C08F |
| A | EP 3 398 975 A1 (SAN APRO LTD [JP]) 7 November 2018 (2018-11-07)<br>* paragraphs [0006], [0009], [0027], [0050] *<br>----- | 7,8 | |
| A | US 2017/355857 A1 (LEE TAI YEON [US] ET AL) 14 December 2017 (2017-12-14)<br>* paragraphs [0076], [0104] - [0113], [0127], [0128], [0146], [0080], [0088], [0133], [0167] *<br>----- | 7,8 | |

The present search report has been drawn up for all claims

2

| Place of search | Date of completion of the search | Examiner |
|---|---|---|
| The Hague | 1 September 2021 | Gomes Pinto F., R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 16 2479

This annex lists the patent family members relating to the patent documents cited in the above-mentioned European search report. The members are as contained in the European Patent Office EDP file on
The European Patent Office is in no way liable for these particulars which are merely given for the purpose of information.

01-09-2021

| Patent document cited in search report | Publication date | Patent family member(s) | Publication date |
|---|---|---|---|
| EP 3782598 A1 | 24-02-2021 | EP 3782598 A1 | 24-02-2021 |
| | | JP WO2019203356 A1 | 22-04-2021 |
| | | US 2021145701 A1 | 20-05-2021 |
| | | WO 2019203356 A1 | 24-10-2019 |
| JP 5379563 B2 | 25-12-2013 | JP 5379563 B2 | 25-12-2013 |
| | | JP 2010275267 A | 09-12-2010 |
| EP 2163234 A1 | 17-03-2010 | CN 101677909 A | 24-03-2010 |
| | | EP 2163234 A1 | 17-03-2010 |
| | | US 2010216096 A1 | 26-08-2010 |
| | | WO 2008149929 A1 | 11-12-2008 |
| EP 2394628 A1 | 14-12-2011 | CN 102300545 A | 28-12-2011 |
| | | EP 2394628 A1 | 14-12-2011 |
| | | JP 5615720 B2 | 29-10-2014 |
| | | JP WO2010090011 A1 | 09-08-2012 |
| | | US 2011288195 A1 | 24-11-2011 |
| | | WO 2010090011 A1 | 12-08-2010 |
| EP 3398975 A1 | 07-11-2018 | EP 3398975 A1 | 07-11-2018 |
| | | JP 6603127 B2 | 06-11-2019 |
| | | JP 2017119803 A | 06-07-2017 |
| | | US 2018373145 A1 | 27-12-2018 |
| | | WO 2017115690 A1 | 06-07-2017 |
| US 2017355857 A1 | 14-12-2017 | US 2017355857 A1 | 14-12-2017 |
| | | US 2020181416 A1 | 11-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006225350 A
- JP 4783151 B
- JP 2006076973 A